# EUROPEAN PATENT SPECIFICATION

(11) **EP 0 587 436 B1**
(45) Date of publication and mention of the grant of the patent: **10.05.2000**
(21) Application number: 93307130.0
(22) Date of filing: 09.09.1993
(51) Int. Cl.: C07D 477/00, C07D 499/88

(54) **Process for preparing carbapenem and penem compounds and new compounds involved in that process**
Verfahren zur Herstellung von Carbapenem- und Penemverbindungen und beteiligte Verbindungen in diesem Verfahren
Procédé de préparation de dérivés de carbapénème et pénème et composés impliqués dans ce procédé

(30) Priority: 09.09.1992 JP 24082592; 04.12.1992 JP 32511492
(43) Date of publication of application: 16.03.1994
(73) Proprietor: SANKYO COMPANY LIMITED, Chuo-ku, Tokyo 103-0023 (JP)
(72) Inventor: Yoshida, Akira, c/o Sankyo Company Limited, Shingawa-ku, Tokyo 140 (JP); Oda, Kozo, c/o Sankyo Company Limited, Shingawa-ku, Tokyo 140 (JP)
(74) Representative: Gibson, Christian John Robert

(56) References cited:
- EP-A- 0 000 828
- EP-A- 0 046 363
- EP-A- 0 060 612
- EP-A- 0 061 231
- EP-A- 0 090 366
- EP-A- 0 105 227
- US-A- 4 820 817

## Description

The present invention relates to a new process for preparing a series of carbapenem and penem compounds which are useful as antibiotics. The invention also provides a series of novel intermediates used in this process.

The penam and cephem antibiotics have been known for many years and have proved of considerable value in the control and prevention of infectious diseases. More recently, penem and carbapenem antibiotics have been developed and have also been found to be of great benefit. The penem and carbapenem compounds have in common a basic structure which may be represented by the formula (A): 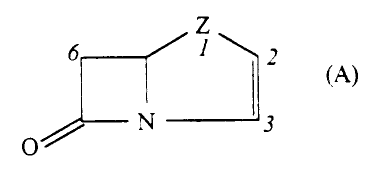 in which Z represents a sulphur atom or a group of formula >CH₂, which may optionally be substituted by an alkyl or alkoxy group. Those compounds in which Z represents a sulphur atom are the penem compounds, whilst those in which Z represents a group of formula >CH₂ or substituted group of formula >CH₂ are the carbapenem compounds. In accordance with the recommendations of the International Union of Pure and Applied Chemistry (IUPAC), the compounds referred to herein are named semi-systematically using penem or carbapenem as the parent compound. For the avoidance of doubt, the above formula (A) also shows the relevant numbers in the peripheral numbering system employed to describe the compounds herein.

Several compounds having a mercapto group attached to the 2-position of a penem or carbapenem compound are known and are thought to be of value as antibiotics. Many such compounds are prepared synthetically. Examples of synthetic methods are provided in EP-A-0060612, EP-A-0061231, EP-A-0105227, EP-A-0090366, EP-A-0000828, EP-A-0046363, EP-A-0480100 and US-A-4820817. In particular, the 1β-methylcarbapenem derivatives, which are currently of considerable interest in this field, must be prepared synthetically, as no microorganism has been found which secretes them. An excellent method of synthesizing such compounds, which overcomes many of the disadvantages of the prior processes, is described in Japanese Patent Kokai Application No. Hei 1-25780 and involves oxidizing a sulphur atom at the carbapenem 2-position to an S-oxide (i.e. a sulphinyl or sulphonyl group) and then replacing the resulting sulphinyl or sulphonyl group by a desired mercapto group, for example as shown in the following reaction:

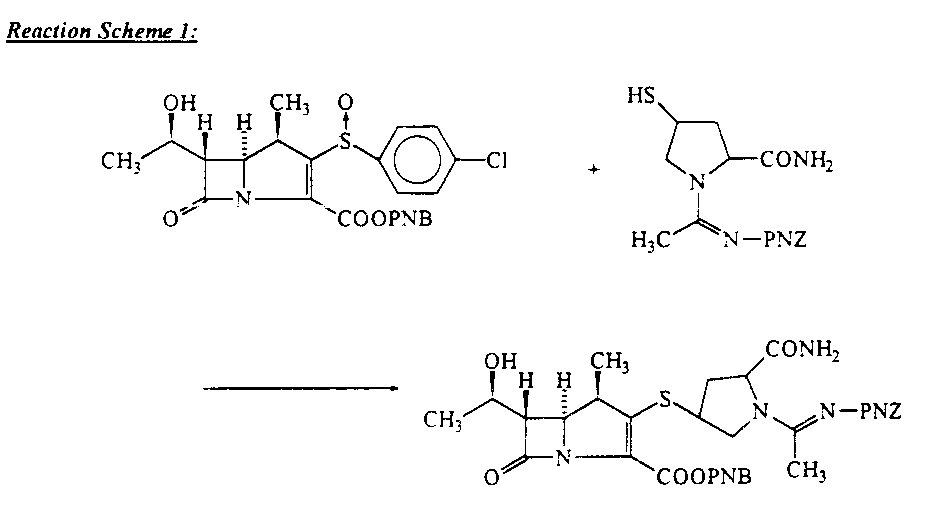

However, when this reaction is carried out by conventional means in which an organic base is employed, in many cases the sulphenic acid formed by the substitution reaction induces the production of various kinds of by-products, which results in a low reaction yield. For example, the yield of the addition-elimination reaction shown in the above reaction scheme is said to be 22% in Japanese Patent Kokai Application No. Hei 1-25780.

We have now surprisingly found that, if the reaction is effected in the presence of certain salts of magnesium or aluminium in place of the organic base, yields can be much improved and yields of 40 to 80% or more (sometimes over 90%) can be achieved, instead of the yields of, at best, around 20% achievable in the prior art.

In general terms, the present invention provides a process for preparing a compound of formula (I): 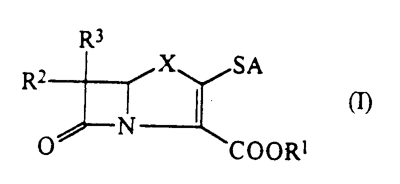 in which:
R¹ represents a hydrogen atom or a carboxy-protecting group;
R² and R³ are the same or different and each represents a hydrogen atom, an alkyl group having from 1 to 6 carbon atoms or a group of formula 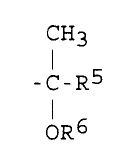
   where R⁵ represents a hydrogen atom or a methyl group; and R⁶ represents a hydrogen atom or a hydroxy-protecting group;
   or
R² and R³ together represent a group of formula =C(CH₃)CH₂OR⁶, in which R⁶ is as defined above;
X represents a sulphur atom or a group of formula >CHR⁷, where R⁷ represents a hydrogen atom, an alkyl group having from 1 to 6 carbon atoms or an alkoxy group having from 1 to 6 carbon atoms; and
A represents
   an alkyl group which has from 1 to 6 carbon atoms and which is unsubstituted or is substituted by 1 or 2 of substituents K, defined below,
   an aryl group, as defined below,
   an aralkyl group in which an alkyl group having from 1 to 6 carbon atoms is substituted by at least one aryl group as defined below,
   an aromatic or non-aromatic heterocylic group which may be monocyclic or may consist of two or more rings attached to each other by fusion or by a spiro attachment and which has from 3 to 10 ring atoms, at least one of said atoms being a nitrogen and/or oxygen and/or sulfur hetero-atom, said group being unsubstituted or being substituted by at least one of substituents B on carbon atoms, at least one of substituents B¹ on nitrogen hetero-atoms, and/or at least one oxygen atom to form a sulphinyl or sulphonyl group on sulphur hetero-atoms, all as defined below;
   a fused heterocyclic group in which a heterocyclic group as defined above is fused to an aryl group as defined below, or
   an alkyl group which has from 1 to 6 carbon atoms and which is substituted by at least one heterocyclic group and/or fused heterocyclic group,
   as defined above;
said aryl groups are aromatic carbocyclic groups having from 6 to 14 ring carbon atoms and are unsubstituted or are substituted by at least one of substituents C, defined below;
said substituents K are selected from hydroxy, protected hydroxy groups, amino, protected amino groups, groups of formula -C(=NR¹⁰)NR¹¹R¹², where
   R¹⁰, R¹¹and R¹² are the same or different and each represents a hydrogen atom, an amino-protecting group or an alkyl group having from 1 to 6 carbon atoms, or R¹¹ and R¹² together represent a group of formula -(CH₂)ₙ-, where n is an integer from 2 to 6, or R¹⁰ and R¹¹ together represent a group of formula -(CH₂)ₚ-, where p is 2 or 3, and
   groups of formula -NR¹³C(=NR¹⁴)R¹⁵, where
   R¹³ and R¹⁴ are the same or different and each represents a hydrogen atom, an amino-protecting group or an alkyl group having from 1 to 6 carbon atoms, and
   R¹⁵ represents a hydrogen atom, an alkyl group having from 1 to 6 carbon atoms, an amino group or a protected amino group,
      or
   any two of R¹³, and R¹⁴ and R¹⁵ together represent a group of formula -(CH₂)ₚ-, where p is 2 or 3;
said substituents B are selected from
   alkyl groups having from 1 to 6 carbon atoms,
   alkoxy groups having from 1 to 6 carbon atoms,
   hydroxy,
   halogen atoms,
   cyano,
   nitro,
   alkoxycarbonyl groups having from 2 to 7 carbon atoms,
   carboxy,
   oxygen atoms, to form with a ring carbon atom a carbonyl group,
   cycloalkyl groups having from 3 to 7 ring carbon atoms,
   alkoxyalkyl groups in which the alkoxy and alkyl parts both have from 1 to 6 carbon atoms,
   alkoxycarbonylalkyl groups in which the alkoxy and alkyl parts both have from 1 to 6 carbon atoms,
   cyanoalkyl groups in which the alkyl part has from 1 to 6 carbon atoms,
   haloalkyl groups having from 1 to 6 carbon atoms,
   alkanoyloxy groups having from 1 to 6 carbon atoms,
   azido,
   alkylthio groups having from 1 to 6 carbon atoms,
   alkylsulphinyl groups having from 1 to 6 carbon atoms,
   alkylsulphonyl groups having from 1 to 6 carbon atoms,
   groups of formula -NR⁸R⁹ and groups of formula -CONR⁸R⁹, where R⁸ and R⁹ are the same or different and each represents a hydrogen atom, an amino-protecting group, an alkyl group having from 1 to 6 carbon atoms or a phenyl group, or R⁸ and R⁹ together represent a group of formula (CH₂)_{q}-Oᵣ-(CH₂)ₛ-, where q and s are the same or different and each represents 0 or an integer of from 1 to 5 and r is 0 or 1, provided that (q+s) is an integer of at least 2; and
   groups of formulae (B-I), (B-II), (B-III), (B-IV), (B-V), (B-VI), (B-VII), (B-VIII) and (B-IX): 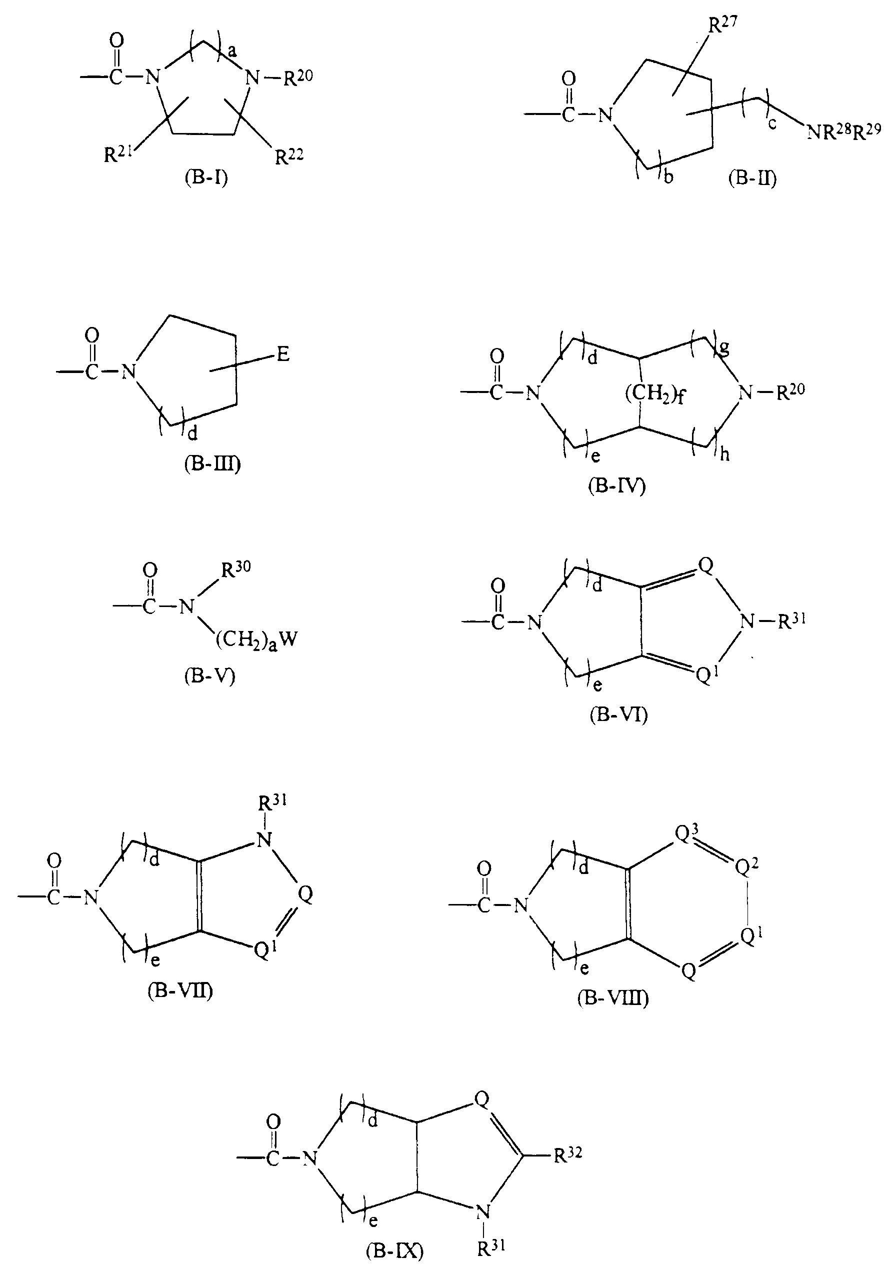 in which:
      a is 1, 2 or 3,
      b is 0, 1 or 2,
      c is 0 or 1,
      d is 0, 1 or 2,
      e is 0, 1 or 2,
      f is 0, 1 or 2,
      g is 0, 1 or 2,
      h is 0, 1 or 2,
      R²⁰ represents a hydrogen atom, an amino-protecting group, a group of formula-COR²⁶,
         where
         R²⁶ represents an alkyl group having from 1 to 6 carbon atoms or a substituted alkyl group which has from 1 to 6 carbon atoms and which is substituted by at least one of substituents K;
         a group of formula -C(=NR¹⁶)R¹⁷, where
         R¹⁶ and R¹⁷ are the same or different and each represents a hydrogen atom, an alkyl group having from 1 to 6 carbon atoms, an amino, a protected amino group or an amino-protecting group;
      an unsubstituted alkyl group having from 1 to 6 carbon atoms, or a substituted alkyl group which has from 1 to 6 carbon atoms and which is substituted by at least one of substituents D, defined below
R²¹ and R²² are the same or different and each represents a hydrogen atom, an alkyl group having from 1 to 6 carbon atoms, a hydroxy or a protected hydroxy group;
R²⁷ represents a hydrogen atom or an alkyl group having from 1 to 6 carbon atoms;
R²⁸ and R²⁹ are the same or different and each represents a hydrogen atom, an alkyl group having from 1 to 6 carbon atoms, an amino-protecting group, an amino, a protected amino group or a group of formula -C(=NR¹⁶)R¹⁷, where R¹⁶ and R¹⁷ are as defined above;
R³⁰ represents a hydrogen atom, an alkyl group having from 1 to 6 carbon atoms or a substituted alkyl group which has from 1 to 6 carbon atoms and which is substituted by at least one hydroxy or protected hydroxy substituent;
R³¹ represents a hydrogen atom, an alkyl group having from 1 to 6 carbon atoms or an amino-protecting group;
R³² represents a hydrogen atom, an alkyl group having from 1 to 6 carbon atoms or a group of formula -NR²⁸R²⁹, in which R²⁸ and R²⁹ are as defined above;
E represents an imidazolyl group or a triazolyl group;
W represents an aromatic heterocyclic group having from 5 to 8 ring atoms, of which from 1 to 4 are nitrogen atoms, said aromatic heterocyclic group being unsubstituted or being substituted by at least one alkyl group having from 1 to 6 carbon atoms;
Q, Q¹, Q² and Q³ are the same or different and each represents a group of formula 〉̶CH or 〉̶N;
said substituents B¹ are selected from alkyl groups having from 1 to 6 carbon atoms, amino-protecting groups, groups of formula -C(=NR¹⁶)R¹⁷, where R¹⁶ and R¹⁷ are as defined above, amino-protecting groups and groups of formula -CONR¹⁸R¹⁹, where
   R¹⁸ and R¹⁹ are the same or different and each represents as alkyl group having from 1 to 6 carbon atoms or a carboxylic acyl group;
said substituents C are selected from alkyl groups having from 1 to 6 carbon atoms, alkoxy groups having from 1 to 6 carbon atoms, halogen atoms, alkoxycarbonyl groups having from 2 to 7 carbon atoms, groups of formula -CONR⁸R⁹, where R⁸ and R⁹ are as defined above, cyano, hydroxy and nitro;
said substituents D are selected from hydroxy, protected hydroxy groups, carboxy, protected carboxy groups, cyano, alkoxy groups having from 1 to 6 carbon atoms, alkylsulphonyl groups having from 1 to 6 carbon atoms, groups of formula -NHCOR²³, -NR²⁴R²⁵, -CONR²⁴R²⁵ or -OCONR²⁴R²⁵, where
   R²³, R²⁴ and R²⁵ are the same or different and each represents a hydrogen atom or an alkyl group having from 1 to 6 carbon atoms,
or a pharmaceutically acceptable salt thereof or a pharmaceutically acceptable ester thereof, which process comprises reacting a compound of formula (II): 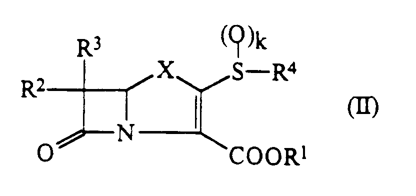
in which R¹, R², R³ and X are as defined above, k is 1 or 2, and R⁴ represents:
   an alkyl group which has from 1 to 6 carbon atoms and which is unsubstituted or is substituted by at least one of substituents E, defined below,
   an alkenyl group which has from 2 to 6 carbon atoms and which is unsubstituted or is substituted by at least one of substituents E, defined below,
   an aryl group, as defined above,
   an aralkyl group in which an alkyl group having from 1 to 6 carbon atoms is substituted by at least one aryl group as defined above,
   a cycloalkyl group which has from 3 to 7 carbon atoms and which is unsubstituted or is substituted by at least one of substituents C, defined above,
   an aromatic heterocylic group which has from 5 to 7 ring atoms in an aromatic ring, at least one of said atoms being a nitrogen and/or oxygen and/or sulfur hetero-atom, said group being unsubstituted or being substituted by at least one of substituents C, defined above;
   a fused heterocyclic group in which an aromatic heterocyclic group as defined above is fused to an aryl group as defined above, or
   an alkyl group which has from 1 to 6 carbon atoms and which is substituted by at least one aromatic heterocylic group and/or fused heterocyclic group, as defined above; and
said substituents E are selected from hydroxy, protected hydroxy groups, amino and protected amino groups;
with a compound of formula (III):

   ASH (III)
in which A is as defined above, and, if desired, removing any protecting groups, characterised in that the reaction of said compound of formula (II) with said compound of formula (III) is carried out in the presence of a salt of magnesium or aluminium sleected from the following group:
a magnesium bromide - diethyl ether complex; a mixture of magnesium bromide - diethyl ether complex with diisopropylethylamine, 1-ethylpiperidine or 1,5-diazabicyclo[5,4,0]-5-undecene; bromomagnesium cyclohexylisopropylamide, bromomagnesium diisopropylamide, bromomagnesium diethylamide or bromomagnesium hexamethyldisilazide; bromomagnesium t-butoxide; a mixture of lithium diisopropylamide or lithium bistrimethylsilylamide with magnesium bromide - diethyl ether complex; or diethylaluminium diisopropylamide;
the compound of formula (II) and the mercaptan of formula (III) being employed in a molar ratio of from 1 : 1 to 1 : 3 and the salt of magnesium or aluminium being present in an amount of from 1 to 20 moles per mole of the compound of formula (II).

Certain of the compounds of formula (II) used as starting materials in the above reaction are novel compounds. These novel compounds are those compounds of formula (IV): 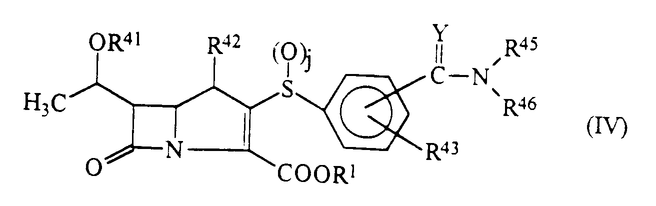 in which:
R¹ is as defined above;
R⁴¹ represents a hydrogen atom or a hydroxy-protecting group;
R⁴² represents an alkyl group having from 1 to 6 carbon atoms, an alkoxy group having from 1 to 6 carbon atoms, a halogen atom, an aryl group as defined above or an aryloxy group in which the aryl part is as defined above;
R⁴³ represents a hydrogen atom, an alkyl group having from 1 to 6 carbon atoms, an alkoxy group having from 1 to 6 carbon atoms, a hydroxy, a halogen atom, a cyano, a nitro or a group of formula -NR⁸R⁹, where R⁸ and R⁹ are as defined above;
R⁴⁵ and R⁴⁶ are the same or different and each represents an alkyl group having from 1 to 6 carbon atoms or an aryl group as defined above, or R⁴⁵ and
R⁴⁶ together represent a group of formula -(CH₂)_{q}-Oᵣ-(CH₂)ₛ-, where
   q and s are the same or different and each represents 0 or an integer of from 1 to 5 and r is 0
   or 1;
Y represents an oxygen atom or a sulphur atom; and
j is 0, 1 or 2;
and pharmaceutically acceptable salts and esters thereof.

In the compounds of formula (I), (II) and (IV), where R¹ represents a carboxy-protecting group, this may be any group commonly employed in the synthesis of β-lactam antibiotics, and, in the case of the compounds of formulae (II) and (IV), which are simply intermediates, there is no particular restriction; in the case of the desired compounds of formula (I), which may be used in therapy, care may need to be taken in selecting the protecting group, if a protecting group is present. More preferably, in the case of the compounds of formula (I), R¹ represents a hydrogen atom or is an ester group, as described in greater detail hereafter. Examples of such protecting groups include:
lower alkyl groups having from 1 to 6, preferably from 1 to 4, carbon atoms, such as the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups; of these, we prefer those alkyl groups having from 1 to 4 carbon atoms, preferably the methyl, ethyl and t-butyl groups;
aralkyl groups, which are preferably alkyl groups which have from 1 to 4, preferably from 1 to 3, more preferably 1 or 2, and most preferably 1, carbon atoms and which are substituted by at least one (more preferably 1, 2 or 3) aryl groups, as defined above, such as the benzyl, diphenylmethyl, triphenylmethyl, 4-nitrobenzyl, 2-nitrobenzyl, 1-naphthylmethyl, α-methylbenzyl, α,α-dimethylbenzyl, 1-phenylethyl, phenethyl (i.e. 2-phenylethyl), 3-phenylpropyl and 4-phenylbutyl groups, of which the benzyl, diphenylmethyl, 4-nitrobenzyl and 2-nitrobenzyl groups are preferred;
alkenyl groups having from 2 to 6, preferably 3 or 4, carbon atoms, and which are optionally substituted by one or more halogen atoms, such as the vinyl, allyl, 2-chloroallyl or 2-methylallyl groups;
haloalkyl groups having from 1 to 6, preferably from 1 to 4, carbon atoms, such as the trifluoromethyl, trichloromethyl, difluoromethyl, dichloromethyl, dibromomethyl, fluoromethyl, chloromethyl, bromomethyl, iodomethyl, 2,2,2-trichloroethyl, 2,2,2-trifluoroethyl, 2-bromoethyl, 2-chloroethyl, 2-fluoroethyl, 2,2-dibromoethyl, 3-chloropropyl, 3,3,3-trichloropropyl, perfluoroethyl, 4-fluorobutyl, 5-bromopentyl, 6-chlorohexyl and 6,6,6-trifluorohexyl groups, preferably the 2,2,2-trichloroethyl or 2,2,2-tribromoethyl group; and
tri-substituted silylethyl groups, in which all three or two or one of the substituents are alkyl groups having from 1 to 5, preferably from 1 to 4, carbon atoms, and correspondingly none, one or two of the substituents are aryl groups, as defined above, but preferably phenyl or substituted phenyl groups, preferably: 1- or 2-[tri(lower alkyl)silyl]ethyl groups, such as the 2-(trimethylsilyl)ethyl, 2-(triethylsilyl)ethyl, 2-(isopropyldimethylsilyl) ethyl, 2-(t-butyldimethylsilyl) ethyl, 2-(methyldiisopropylsilyl)ethyl, 2-(methyldi-t-butylsilyl)ethyl and 2-(triisopropylsilyl)ethyl groups; and 1- or 2- [tri(lower alkyl)silyl]ethyl groups in which one or two of the alkyl groups have been replaced by aryl groups, such as the 2-(diphenylmethylsilyl)ethyl, 2-(diphenylbutylsilyl)ethyl, 2-(diphenyl-t-butylsilyl)ethyl, 2-(diphenyl-isopropylsilyl)ethyl and 2-(phenyldiisopropylsilyl)ethyl groups, especially the 2-(trimethylsilyl)ethyl group;
acyloxyalkyl groups, in which the acyl part is an alkanoyl group having from 1 to 6, preferably from 1 to 5, carbon atoms and the alkyl part has from 1 to 6, preferably 1 or 2, carbon atoms, such as the formyloxymethyl, acetoxymethyl, propionyloxymethyl, butyryloxymethyl, valeryloxymethyl, isovaleryloxymethyl, pivaloyloxymethyl, hexanoyloxymethyl, 1-formyloxyethyl, 1-acetoxyethyl, 1-propionyloxyethyl, 1-butyryloxyethyl, 1-valeryloxyethyl, 1-isovaleryloxyethyl, 1-pivaloyloxyethyl, 1-hexanoyloxyethyl, 1-formyloxypropyl, 1-acetoxypropyl, 1-pivaloyloxypropyl, 1-acetoxybutyl, 1-acetoxypentyl and 1-acetoxyhexyl groups, preferably the acetoxymethyl, pivaloyloxymethyl and 1-acetoxyethyl groups;
alkoxycarbonyloxyalkyl groups in which the alkoxy and alkyl parts each have from 1 to 6, preferably from 1 to 4, carbon atoms, such as the ethoxycarbonyloxymethyl, 1-(ethoxycarbonyloxy)ethyl, 1-(isopropoxycarbonyloxy)ethyl and t-butoxycarbonyloxymethyl groups;
cycloalkoxycarbonyloxyalkyl groups in which the cycloalkyl part has from 3 to 7, preferably 5 or 6, ring carbon atoms and may be unsubstituted or substituted by at least one of substituents C, defined above and exemplified below, but particularly an alkyl group having from 1 to 6, preferably from 1 to 4 and more preferably 1 or 2, carbon atoms, and the alkyl part has from 1 to 6, preferably from 1 to 4 and more preferably 1 or 2, carbon atoms, such the 1-(cyclohexyloxycarbonyloxy)ethyl and (1-methylcyclohexyl)oxycarbonyloxymethyl groups;
(5-alkyl or 5-aryl -2-oxo-1,3-dioxolen-4-yl)methyl groups in which the alkyl part has from 1 to 4, preferably 1 or 2, carbon atoms and the aryl part is as defined above, but is preferably a phenyl group, such as the (5-methyl-2-oxo-1,3-dioxolen-4-yl)methyl and (5-phenyl-2-oxo-1,3-dioxolen-4-yl)methyl groups; and
the 3-phthalidyl group.

The above acyloxyalkyl groups, alkoxycarbonyloxyalkyl groups, cycloalkoxycarbonyloxyalkyl groups, (5-alkyl or 5-aryl -2-oxo-1,3-dioxolen-4-yl)methyl groups and 3-phthalidyl group are capable of hydrolysis in vivo and are therefore particularly preferred, especially for those cases where they are to be retained in the final product for therapeutic use.

Where R², R³ or R⁴ represents an alkyl group, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl, isobutyl, sec-butyl and t-butyl groups, and most preferably the methyl and ethyl groups.

Where R² or R³ represents a group of formula 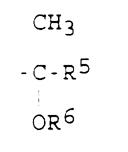 and R⁶ represents a hydroxy-protecting group, the hydroxy-protecting group may be selected from any protecting groups known in the art for use in β-lactam antibiotics. Examples of such groups are given by T. W. Green in "Protective Groups in Organic Synthesis", John Wiley & Sons; and by J. F. W. McOmie in "Protective Groups in Organic Chemistry", Plenum Press. More specifically, preferred such protecting groups include the:
substituted silyl groups, in which the silyl group has up to 3, and preferably 3, substituents selected from alkyl groups having from 1 to 6 carbon atoms and phenyl groups which are unsubstituted or are substituted by at least one of substituents C defined above and exemplified below, for example a trimethylsilyl, triethylsilyl, t-butyldimethylsilyl or t-butyldiphenylsilyl group;
aralkyl groups, which are preferably alkyl groups which have from 1 to 4, preferably from 1 to 3, more preferably 1 or 2, and most preferably 1, carbon atoms and which are substituted by at least one (more preferably 1, 2 or 3) aryl groups, as defined above, such as the benzyl, diphenylmethyl, triphenylmethyl, 4-methoxybenzyl, 4-nitrobenzyl, 2-nitrobenzyl, 1-naphthylmethyl, α-methylbenzyl, α,α-dimethylbenzyl, 1-phenylethyl, phenethyl, 3-phenylpropyl and 4-phenylbutyl groups, of which the benzyl, 4-methoxybenzyl, 4-nitrobenzyl and 2-nitrobenzyl groups are preferred; oxycarbonyl groups, including:
   aralkyloxycarbonyl groups, in which the aralkyl part may be as defined and exemplified above, preferably the benzyloxycarbonyl, 4-nitrobenzyloxycarbonyl, 2-nitrobenzyloxycarbonyl and benzhydryloxycarbonyl groups;
   alkenyloxycarbonyl groups in which the alkenyl part has from 2 to 6, preferably 2 or 3, carbon atoms and may be unsubstituted or may be substituted by at least one (preferably from 1 to 3, more preferably 1) halogen atom, such as the allyloxycarbonyl, 2-chloroallyloxycarbonyl and 2-methylallyloxycarbonyl groups;
   alkoxycarbonyl groups which have in total from 2 to 7 carbon atoms, that is in which the alkyl part has from 1 to 6 carbon atoms and may be any of the alkyl groups exemplified above, and which may be unsubstituted or may be substituted by at least one (preferably from 1 to 3, more preferably 1 or 3) halogen atom, such as the 2,2,2-trichloroethoxycarbonyl, 2,2,2-tribromoethoxycarbonyl and t-butoxycarbonyl groups; and
   substituted silylalkoxycarbonyl groups, in which the alkoxycarbonyl part is as defined and exemplified above and the substituted silyl part is also as defined and exemplified above, such as the 2-(trimethylsilyl)ethoxycarbonyl group;
ether groups, including:
   oxygen-containing heterocyclic groups, for example the substituted and unsubstituted tetrahydropyranyl and tetrahydrofuranyl groups, such as the tetrahydropyranyl group;
   alkoxyalkyl groups in which the alkoxy and alkyl parts each have from 1 to 6, preferably from 1 to 4 and more preferably 1 or 2, carbon atoms, such as the methoxymethyl and 1-ethoxyethyl groups;
   substituted silylalkoxyalkyl groups, in which the alkoxyalkyl part is as defined and exemplified above and the substituted silyl part is also as defined and exemplified above, such as the 2-(trimethylsilyl)ethoxymethyl group; and
and alkanoyl and haloalkanoyl groups which have from 1 to 6 carbon atoms (2 to 6, in the case of the haloalkanoyl groups), such as the acetyl, propionyl, butyryl, isobutyryl, valeryl, isovaleryl, pivaloyl and chloroacetyl groups.

Where R⁷ represents an alkyl group having from 1 to 6 carbon atoms, this may be a straight or branched chain group, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl and sec-butyl groups, and most preferably the methyl group.

Where R⁷ represents an alkoxy group having from 1 to 6 carbon atoms, this may be a straight or branched chain group, and examples include the methoxy, ethoxy, propoxy, isopropoxy, butoxy, isobutoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy and isohexyloxy groups. Of these, we prefer those alkoxy groups having from 1 to 4 carbon atoms, preferably the methoxy, ethoxy, propoxy, isopropoxy, butoxy and sec-butoxy groups, and most preferably the methoxy group.

Where A represents an alkyl group, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl, isobutyl, sec-butyl and t-butyl groups, and most preferably the methyl and ethyl groups.

Where A represents a substituted alkyl group, the alkyl part may be any of the unsubstituted groups defined and exemplified above, and the substituents are selected from substituents K, which include:
hydroxy,
protected hydroxy groups, such as those defined and exemplified above in relation to R⁶,
amino,
protected amino groups, in which the protecting group may be chosen from any amino-protecting group well known in the field of β-lactam chemistry, and there may be one or two such groups, preferably one such group, for example
   acyl groups, including alkanoyl and haloalkanoyl groups as defined above in relation to the hydroxy-protecting groups which may be represented by R⁶, preferably the formyl, acetyl, chloroacetyl and propionyl groups, and arylcarbonyl groups (in which the aryl part may be as defined above and exemplified below), preferably the benzoyl group, and
   oxycarbonyl groups, which may be as defined above in relation to the hydroxy-protecting groups which may be represented by R⁶, preferably the benzyloxycarbonyl, 4-nitrobenzyloxycarbonyl, 2-nitrobenzyloxycarbonyl, allyloxycarbonyl, 2,2,2-trichloroethoxycarbonyl, 2,2,2-tribromoethoxycarbonyl, t-butoxycarbonyl and 2-(trimethylsilyl)ethoxycarbonyl groups;
groups of formula -C(=NR¹⁰)NR¹¹R¹² and -NR¹³C(=NR¹⁴)R¹⁵, where R¹⁰, R¹¹, R¹², R¹³ and R¹⁴ are the same or different and each represents a hydrogen atoms, amino-protecting groups (for example as defined and exemplified above) and alkyl groups having from 1 to 6 carbon atoms (for example as defined and exemplified in relation to the alkyl groups which may be represented by A, preferably a methyl or ethyl group), or R¹¹ and R¹² together represent a group of formula -(CH₂)ₙ-, where n is an integer from 2 to 6, or R¹⁰ and R¹¹ together represent a group of formula -(CH₂)ₚ-, where p is 2 or 3, and R¹⁵ represents a hydrogen atom, an alkyl group having from 1 to 6 carbon atoms (for example as defined and exemplified in relation to the alkyl groups which may be represented by A, preferably a methyl, ethyl or propyl group), an amino group or a protected amino group (for example as defined and exemplified above), or any two of R¹³, R¹⁴ and R¹⁵ together represent a group of formula -(CH₂)ₚ-, where p is 2 or 3.

Where A represents an aryl group, this is a carbocyclic aromatic group having from 6 to 14, preferably from 6 to 10, more preferably 6 or 10, and most preferably 6, ring carbon atoms in one or more aromatic rings. The group may also be unsubstituted or substituted, and, if substituted, the substituents are selected from substituents C, defined above and exemplified below. In the case of the substituted groups, there is no particular limitation on the number of substituents on the aryl group, except such as may be imposed by the number of substitutable positions or possibly by steric constraints, but, in general, from 1 to 5 substituents are preferred, from 1 to 4 being more preferred and 1, 2 or 3 being most preferred. Also, where the group is substituted, it is preferred that it should not be further substituted by a group which is also substituted by another aryl group. Specific examples of the aromatic groups include the phenyl, α-or β- naphthyl, indenyl, phenanthrenyl and anthracenyl groups, of which we prefer those aromatic hydrocarbon groups having from 6 to 10 ring carbon atoms, particularly the phenyl, α-naphthyl and β-naphthyl groups, the phenyl group being most preferred.

Where the aryl group is substituted, the substituents are selected from substituents C, which include:
alkyl groups having from 1 to 6 carbon atoms, which may be a straight or branched chain group, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups; of these, we prefer those alkyl groups having from 1 to 4 carbon atoms, preferably the methyl, ethyl, propyl, isopropyl, butyl and isobutyl groups, more preferably the methyl, ethyl, propyl and isopropyl groups, and most preferably the methyl group;
alkoxy groups having from 1 to 6 carbon atoms, which may be a straight or branched chain group, and examples include the methoxy, ethoxy, propoxy, isopropoxy, butoxy, isobutoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy and isohexyloxy groups; of these, we prefer those alkoxy groups having from 1 to 4 carbon atoms, preferably the methoxy, ethoxy, propoxy, isopropoxy, butoxy and isobutoxy groups, more preferably the methoxy, ethoxy and propoxy groups, and most preferably the methoxy group;
halgen atoms, such as the fluorine, chlorine, bromine and iodine atoms, particularly the fluorine, chlorine and bromine atoms;
alkoxycarbonyl groups having from 2 to 7 carbon atoms, that is the alkoxy part has from 1 to 6 carbon atoms and may be any of the alkoxy groups defined ande exemplifeid above; examples of such alkoxycarbonyl groups include the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl, isopentyloxycarbonyl, neopentyloxycarbonyl, 2-methylbutoxycarbonyl, 1-ethylpropoxycarbonyl, 4-methylpentyloxycarbonyl, 3-methylpentyloxycarbonyl, 2-methylpentyloxycarbonyl, 1-methylpentyloxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl, 2,3-dimethylbutoxycarbonyl, 2-ethylbutoxycarbonyl, hexyloxycarbonyl and isohexyloxycarbonyl groups; of these, we prefer those alkoxycarbonyl groups having from 1 to 4 carbon atoms, preferably the methoxycarbonyl, ethoxycarbonyl, propoxycarbonyl, isopropoxycarbonyl, butoxycarbonyl and isobutoxycarbonyl groups, more preferably the methoxycarbonyl and ethoxycarbonyl groups;
groups of formula -CONR⁸R⁹, where R⁸ and R⁹ are the same or different and each represents:
   a hydrogen atom,
   an amino-protecting group (for example as defined and exemplified above in relation to the groups which may be included in substituents A),
   an alkyl group having from 1 to 6 carbon atoms (such as those exemplified above in relation to substituents C) or
   a phenyl group, or
   R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
cyano, hydroxy and nitro.

Where A represents an aralkyl group, this is as defined above, and examples include any of the alkyl groups having from 1 to 6, preferably from 1 to 4, carbon atoms exemplified above and substituted by at least one aryl group, such as those exemplified above. There is no particular limitation on the number of aryl substituents on the alkyl group, except such as may be imposed by the number of substitutable positions or possibly by steric constraints, but, in general, from 1 to 3 aryl groups are preferred, 2 or 1 being more preferred and 1 being most preferred. The aryl group forming part of the aralkyl group may itself be unsubstituted or it may be substituted by at least one of substituents C, defined above and exemplified below. Again, there is no particular limitation on the number of substituents on the aryl group, except such as may be imposed by the number of substitutable positions or possibly by steric constraints, but, in general, from 1 to 5 substituents are preferred, from 1 to 3 being more preferred and 1 being most preferred. Specific examples of such groups include the benzyl, α-naphthylmethyl, β-naphthylmethyl, 2-methylbenzyl, 3-methylbenzyl, 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 2-nitrobenzyl, 4-nitrobenzyl, 2-chlorobenzyl, 3-chlorobenzyl, 4-chlorobenzyl, 4-bromobenzyl, 4-cyanobenzyl, α or β- naphthylmethyl, benzhydryl (i.e. diphenylmethyl), trityl (i.e. triphenylmethyl), phenethyl, 1-phenylethyl, 1-(α or β- naphthyl)ethyl, 2-(α or β- naphthyl)ethyl, 1-phenylpropyl, 2-phenylpropyl, 3-phenylpropyl, 1-(α or β- naphthyl)propyl, 2-(α or β- naphthyl)propyl, 3-(α or β- naphthyl)propyl, 1-phenylbutyl, 2-phenylbutyl, 3-phenylbutyl, 4-phenylbutyl, 1-(α or β- naphthyl)butyl, 2-(α or β- naphthyl)butyl, 3-(α or β- naphthyl)butyl, 4-(α or β- naphthyl)butyl, 1-phenylpentyl, 2-phenylpentyl, 3-phenylpentyl, 4-phenylpentyl, 5-phenylpentyl, 1-(α or β-naphthyl)pentyl, 2-(α or β- naphthyl)pentyl, 3-(α or β- naphthyl)pentyl, 4-(α or β- naphthyl)pentyl, 5-(α or β- naphthyl)pentyl, 1-phenylhexyl, 2-phenylhexyl, 3-phenylhexyl, 4-phenylhexyl, 5-phenylhexyl, 6-phenylhexyl, 1-(α or β- naphthyl)hexyl, 2-(α or β- naphthyl)hexyl, 3-(α or β- naphthyl)hexyl, 4-(α or β- naphthyl)hexyl, 5-(α or β- naphthyl)hexyl and 6-(α or β- naphthyl)hexyl groups. Of these, we prefer the benzyl, phenethyl, 3-phenylpropyl and 4-phenylpentyl groups, the unsubstituted aralkyl groups being more preferred, and the unsubstituted benzyl and phenethyl groups being most preferred.

Where A represents a heterocylic group, this has from 3 to 10, more preferably from 5 to 7, ring atoms in one or more heterocyclic rings, at least one of said atoms, preferably from 1 to 4 and more preferably from 1 to 3 of said atoms, being a nitrogen and/or oxygen and/or sulfur hetero-atom, said group being unsubstituted or being substituted by at least one of substituents B on carbon atoms, at least one of substituents B¹ on nitrogen hetero-atoms, and/or at least one oxygen atom to form a sulphinyl or sulphonyl group on sulphur hetero-atoms, all as defined above and exemplified below, or it may represent a fused heterocyclic group in which a heterocyclic group as defined above is fused to an aryl group as defined above, preferably a phenyl group. The heterocyclic group may be aromatic or non-aromatic, but is preferably non-aromatic. The group may be a monocyclic group or it may consist of two or more rings attached to each other by fusion or by a spiro attachment. In the case of those groups having 4 hetero-atoms in a ring, we prefer that 3 or 4 of them should be nitrogen atoms and 1 or 0 should be an oxygen or sulphur atom. In the case of those groups having 3 hetero-atoms in a ring, we prefer that 1, 2 or 3 should be nitrogen atoms and, correspondingly, 2, 1 or 0 should be oxygen and/or sulphur atoms. In the case of those groups having 1 or 2 hetero-atoms in a ring, the hetero-atoms may be freely chosen from nitrogen, oxygen and sulphur atoms. Preferably, however, the group contains at least one nitrogen atom. The group may be substituted or unsubstituted and, if substituted, the substituents are selected from substituents B, B¹ and oxygen atoms, as defined above and exemplified below.

The groups may also be saturated, unsaturated or partially saturated and, if fully unsaturated, may be aromatic in character. Examples of such non-aromatic groups include the azetidinyl, pyrrolidinyl, oxopyrrolidinyl, piperidyl, oxopiperidyl, morpholinyl (especially morpholino), thiomorpholinyl, tetrahydropyrimidinyl, thiazolidinyl, oxazolidinyl, hexahydropyrimidinyl, imidazolidinyl, octahydroazocinyl and pyrazolidinyl groups. Of these, we prefer the saturated heterocyclic groups having 5 or 6 ring atoms and containing at least one nitrogen atom, and optionally an oxygen or sulphur atom or atoms.

These heterocyclyl groups may optionally be condensed with one or more other cyclic groups such as, for example, the aryl groups and cycloalkyl groups defined and exemplified herein. Examples of such groups include: the 1,2,3,4-tetrahydroisoquinolyl, pyrazolotriazolyl decahydroisoquinolyl, 1,2,3,4-tetrahydroquinolyl, decahydroquinolyl, isoindolinyl and indolinyl groups.

Where A represents a substituted alkyl group, this has from 1 to 6 carbon atoms and is substituted by at least one substituent selected from the heterocyclic groups and fused heterocyclic groups defined and exemplified above.

Such heterocyclic groups and heterocyclic groups which are substituents on alkyl groups represented by A may be unsubstituted or they may be substituted by one or more of the following groups and atoms. There is no particular restriction upon the number of substituents which may be present, except such as may be imposed by the number of substitutable atoms and possibly by steric constraints. In general, from 1 to 5 substituents are preferred, from 1 to 3 being more preferred.

In the case of carbon atoms or sulphur atoms, these may be substituted by 1 (carbon atoms) or 1 or 2 (sulphur atoms) oxygen atoms, to form a carbonyl group or a sulphinyl or sulphonyl group.

In the case of carbon atoms, these may be substituted by one or more of substituents B:
alkyl groups having from 1 to 6 carbon atoms, such as the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methyl- pentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups, preferably the methyl, ethyl, propyl, isopropyl, butyl, isobutyl, sec-butyl, t-butyl, pentyl and isopentyl groups;
alkoxy groups having from 1 to 6 carbon atoms, such as the methoxy, ethoxy, propoxy, isopropoxy, butoxy, isobutoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy and isohexyloxy groups, preferably the methoxy, ethoxy, propoxy, isopropoxy, butoxy, isobutoxy, sec-butoxy and t-butoxy groups;
hydroxy, cyano, nitro,
halogen atoms, such as the fluorine, chlorine, bromine and iodine atoms;
alkoxycarbonyl groups having from 2 to 7 carbon atoms, such as the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl, isopentyloxycarbonyl, neopentyloxycarbonyl, 2-methylbutoxycarbonyl, 1-ethylpropoxycarbonyl, 4-methylpentyloxycarbonyl, 3-methylpentyloxycarbonyl, 2-methylpentyloxycarbonyl, 1-methylpentyloxycarbonyl, 3,3-dimethylbutoxycarbonyl, 2,2-dimethylbutoxycarbonyl, 1,1-dimethylbutoxycarbonyl, 1,2-dimethylbutoxycarbonyl, 1,3-dimethylbutoxycarbonyl, 2,3-dimethylbutoxycarbonyl, 2-ethylbutoxycarbonyl, hexyloxycarbonyl and isohexyloxycarbonyl groups, preferably the methoxycarbonyl, ethoxycarbonyl, propoxycarbonyl, isopropoxycarbonyl, butoxycarbonyl, isobutoxycarbonyl, sec-butoxycarbonyl and t-butoxycarbonyl groups; carboxy,
cycloalkyl groups having from 3 to 7 ring carbon atoms, preferably 5 or 6 ring carbon atoms, such as the cyclopropyl, cyclobutyl, cyclopentyl and cyclohexyl groups;
alkoxyalkyl groups in which the alkoxy and alkyl parts both have from 1 to 6 carbon atoms, and each may be as defined and exemplified above in relation to those groups included in substituents B, for example the methoxymethyl, ethoxymethyl, propoxymethyl, isopropoxymethyl, butoxymethyl, 2-methoxyethyl, 3-methoxypropyl, 2-methoxypropyl or 4-ethoxybutyl;
alkoxycarbonylalkyl groups in which the alkoxy and alkyl parts both have from 1 to 6 carbon atoms, and each may be as defined and exemplified above in relation to those groups included in substituents B, for example the methoxycarbonylmethyl, ethoxycarbonylmethyl, propoxycarbonylmethyl, isopropoxycarbonylmethyl, butoxycarbonylmethyl, 2-methoxycarbonylethyl, 3-methoxycarbonylpropyl, 2-methoxycarbonylpropyl and 4-ethoxycarbonylbutyl groups;
cyanoalkyl groups in which the alkyl part has from 1 to 6 carbon atoms, such as the cyanomethyl, 2-cyanoethyl, 3-cyanopropyl, 2-cyanopropyl and 4-cyanobutyl groups;
haloalkyl groups having from 1 to 6 carbon atoms, in which the alkyl part may be any of those alkyl groups exemplified above and has at least one halogen substituent; there is no restriction on the number of halogen substituents, and the group may be anything from a monohaloalkyl group to a perhaloalkyl group; preferably there are from 1 to 3 halogen atoms; examples of such groups include the trifluoromethyl, trichloromethyl, fluoromethyl, chloromethyl, bromomethyl, iodomethyl, 2-fluoroethyl, 2,2,2-trifluoroethyl, 2-chloroethyl, 2-bromoethyl, 3-fluoropropyl, 2-fluoropropyl, 4-chlorobutyl and 3-fluorobutyl groups;
alkanoyloxy groups having from 1 to 6 carbon atoms, such as the acetoxy, propionyloxy, butyryloxy, isobutyryloxy, valeryloxy, isovaleryloxy, pivaloyloxy and hexanoyloxy groups, preferably the acetoxy, propionyloxy, butyryloxy or isobutyryloxy;
azido,
alkylthio groups having from 1 to 6 carbon atoms, such as the methylthio, ethylthio, propylthio, isopropylthio, butylthio, isobutylthio, sec-butylthio, t-butylthio, pentylthio, isopentylthio, neopentylthio, 2-methylbutylthio, 1-ethylpropylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 2-ethylbutylthio, hexyl and isohexylthio groups, preferably the methylthio, ethylthio, propylthio, isopropylthio, butylthio and isobutylthio groups;
alkylsulphinyl groups having from 1 to 6 carbon atoms, such as the methylsulphinyl, ethylsulphinyl, propylsulphinyl, isopropylsulphinyl, butylsulphinyl, isobutylsulphinyl, sec-butylsulphinyl, t-butylsulphinyl, pentylsulphinyl, isopentylsulphinyl, neopentylsulphinyl, 2-methylbutylsulphinyl, 1-ethylpropylsulphinyl, 4-methylpentylsulphinyl, 3-methylpentylsulphinyl, 2-methylpentylsulphinyl, 1-methylpentylsulphinyl, 3,3-dimethylbutylsulphinyl, 2,2-dimethylbutylsulphinyl, 1,1-dimethylbutylsulphinyl, 1,2-dimethylbutylsulphinyl, 1,3-dimethylbutylsulphinyl, 2,3-dimethylbutylsulphinyl, 2-ethylbutylsulphinyl, hexylsulphinyl and isohexylsulphinyl groups, preferably the methylsulphinyl, ethylsulphinyl, propylsulphinyl, isopropylsulphinyl, butylsulphinyl and isobutylsulphinyl groups;
alkylsulphonyl groups having from 1 to 6 carbon atoms, such as the methylsulphonyl, ethylsulphonyl, propylsulphonyl, isopropylsulphonyl, butylsulphonyl, isobutylsulphonyl, sec-butylsulphonyl, t-butylsulphonyl, pentylsulphonyl, isopentylsulphonyl, neopentylsulphonyl, 2-methylbutylsulphonyl, 1-ethylpropylsulphonyl, 4-methylpentylsulphonyl, 3-methylpentylsulphonyl, 2-methylpentylsulphonyl, 1-methylpentylsulphonyl, 3,3-dimethylbutylsulphonyl, 2,2-dimethylbutylsulphonyl, 1,1-dimethylbutylsulphonyl, 1,2-dimethylbutylsulphonyl, 1,3-dimethylbutylsulphonyl, 2,3-dimethylbutylsulphonyl, 2-ethylbutylsulphonyl, hexylsulphonyl and isohexylsulphonyl groups, preferably the methylsulphonyl, ethylsulphonyl, propylsulphonyl, isopropylsulphonyl, butylsulphonyl and isobutylsulphonyl groups;
groups of formula -NR⁸R⁹ and groups of formula -CONR⁸R⁹, where R⁸ and R⁹ are the same or different and each represents a hydrogen atoms, amino-protecting groups, alkyl groups having from 1 to 6 carbon atoms and phenyl groups, or R⁸ and R⁹ together represent a group of formula -(CH₂)_{q}-Oᵣ-(CH₂)ₛ-, where q and s are the same or different and each represents a 0 and integers of from 1 to 5 and r is 0 or 1, provided that (q+s) is an integer of at least 2, all as defined and exemplified above; and
groups of formulae (B-I), (B-II), (B-III), (B-IV), (B-V), (B-VI), (B-VII), (B-VIII) and (B-IX), as shown above.

In formulae (B-I) to (B-IX):
R²⁰ represents a hydrogen atom, an amino-protecting group (such as those exemplified above in relation to A), a group of formula-COR²⁶, where
   R²⁶ represents an alkyl group having from 1 to 6 carbon atoms (such as those exemplified above in relation to R¹, preferably a methyl group), or a substituted alkyl group which has from 1 to 6 carbon atoms and which is substituted by at least one of substituents K;
a group of formula -C(=NR¹⁶)R¹⁷, where
   R¹⁶ and R¹⁷ are the same or different and each represents a hydrogen atom, an alkyl group having from 1 to 6 carbon atoms (such as those exemplified above in relation to R¹, preferably a methyl group), an amino group, a protected amino group (such as those exemplified above in relation to A) or an amino-protecting group (such as those exemplified above in relation to A);
an unsubstituted alkyl group having from 1 to 6 carbon atoms (such as those exemplified above in relation to R¹, preferably a methyl group), or a substituted alkyl group which has from 1 to 6 carbon atoms and which is substituted by at least one of substituents D, defined and exemplified below.

R²¹ and R²² are the same or different and each represents a hydrogen atom, an alkyl group having from 1 to 6 carbon atoms (such as those exemplified above in relation to R¹, preferably a methyl group), a hydroxy group or a protected hydroxy group (such as those defined and exemplified above in relation to R⁶);
R²⁸ and R²⁹ are the same or different and each represents a hydrogen atom, an alkyl group having from 1 to 6 carbon atoms (such as those exemplified above in relation to R¹, preferably a methyl group), an amino-protecting group (such as those exemplified above in relation to A), an amino group, a protected amino group (such as those exemplified above in relation to A) or a group of formula -C(=NR¹⁶)R¹⁷, where R¹⁶ and R¹⁷ are as defined above.

R³⁰ represents a hydrogen atom, an alkyl group having from 1 to 6 carbon atoms (such as those exemplified above in relation to R¹, preferably a methyl group) or a substituted alkyl group which has from 1 to 6 carbon atoms and which-is substituted by at least one substituent selected from hydroxy groups (for example a 1-hydroxyethyl group) and protected hydroxy groups (such as those defined and exemplified above in relation to R⁶).

R³¹ represents a hydrogen atom, an alkyl group having from 1 to 6 carbon atoms (such as those exemplified above in relation to R¹, preferably a methyl group) or an amino-protecting group (such as those exemplified above in relation to A).

R³² represents a hydrogen atom, an alkyl group having from 1 to 6 carbon atoms (such as those exemplified above in relation to R¹, preferably a methyl group) or a group of formula -NR²⁸R²⁹, in which R²⁸ and R²⁹ are as defined above.

E represents an imidazolyl group or a triazolyl group.

W represents an aromatic heterocyclic group having from 5 to 8, preferably 5 or 6, ring atoms, of which from 1 to 4 are nitrogen atoms, said aromatic heterocyclic group being unsubstituted or being substituted by at least one alkyl group having from 1 to 6 carbon atoms (such as those exemplified above in relation to R¹, preferably a methyl group); specific examples of such aromatic heterocyclic groups include the pyridyl, pyridazinyl, pyrazinyl, pyrrolyl, imidazolyl, triazolyl and tetrazolyl groups.

Where there is, as is preferred, a nitrogen atom in the heterocyclic group, this may also be substituted, and the substituents are selected from substituents B¹, which include:
alkyl groups having from 1 to 6 carbon atoms, such as those exemplified above in relation to R¹, preferably a methyl group;
groups of formula -C(=NR¹⁶)R¹⁷, where R¹⁶ and R¹⁷ are as defined and exemplified above;
amino-protecting groups, such as those exemplified above in relation to A; and
groups of formula -CONR¹⁸R¹⁹, where
   R¹⁸ and R¹⁹ are the same or different and each represents an alkyl group having from 1 to 6 carbon atoms (such as those exemplified above in relation to R¹, preferably a methyl, ethyl, propyl or isopropyl group) or a carboxylic acyl group (preferably an oxycarbonyl group, as defined and exemplified above in relation to the oxycarbonyl groups which may be represented by R⁶.

Substituents D are selected from hydroxy, protected hydroxy groups (such as those defined and exemplified above in relation to R⁶), carboxy, protected carboxy groups (such as those defined and exemplified above in relation to R¹), cyano, alkoxy groups having from 1 to 6 carbon atoms (such as those defined and exemplified above in relation to R⁷, preferably a methoxy group), alkylsulphonyl groups having from 1 to 6 carbon atoms (such as those exemplified above in relation to substituents B), and groups of formula -NHCOR²³, -NR²⁴R²⁵, -CONR²⁴R²⁵ or -OCONR²⁴R²⁵, where R²³, R²⁴ and R²⁵ are the same or different and each represents a hydrogen atom or an alkyl group having from 1 to 6 carbon atoms (such as those exemplified above in relation to R¹, preferably a methyl, ethyl, propyl or isopropyl group).

Where R⁴ represents an alkyl group, this may be as defined and exemplified above. The group may be unsubstituted or it may be substituted by one or more of

substituents E, which include: hydroxy, protected hydroxy groups (such as those defined and exemplified above in relation to R⁶), amino and protected amino groups (such as those exemplified above in relation to A).

Where R⁴ represents an alkenyl group, this has from 2 to 6, preferably 3 or 4, carbon atoms and is unsubstituted or is substituted by at least one of substituents E, defined and exemplified above. Examples include the vinyl, allyl, methallyl, 1-propenyl, isopropenyl, 1-butenyl, 2-butenyl, 3-butenyl, 1-pentenyl, 2-pentenyl, 3-pentenyl and 4-pentenyl groups, of which the vinyl, allyl, 1-propenyl, and 1-butenyl groups are preferred, the vinyl, allyl and 1-butenyl groups being most preferred.

Where R⁴ represents an aryl or aralkyl group, this may be any one of the aryl or aralkyl groups defined and exemplified above in relation to A, and, as with A, it may be substituted or unsubstituted.

Where R⁴ represents a cycloalkyl group, this has from 3 to 7 carbon atoms and is unsubstituted or is substituted by at least one of substituents C, defined and exemplified above. Preferred cycloalkyl groups have from 4 to 6 carbon atoms, and examples of such groups include the cyclopropyl, cyclobutyl, cyclopentyl, cyclohexyl and cycloheptyl groups, of which the cyclobutyl, cyclopentyl and cyclohexyl groups are preferred.

Where R⁴ represents an aromatic heterocyclic group, this has from 5 to 7, preferably 5 or 6, ring atoms in an aromatic heterocyclic ring, and this may optionally be fused to an aryl group, as defined and exemplified above, preferably a phenyl group. It has at least one nitrogen and/or oxygen and/or sulfur hetero-atom, and preferably has from 1 to 4, more preferably from 1 to 3 and most preferably 1 or 2, such hetero-atoms. In the case of those groups having 4 hetero-atoms in a ring, we prefer that 3 or 4 of them should be nitrogen atoms and 1 or 0 should be an oxygen or sulphur atom. In the case of those groups having 3 hetero-atoms in a ring, we prefer that 1, 2 or 3 should be nitrogen atoms and, correspondingly, 2, 1 or 0 should be oxygen and/or sulphur atoms. In the case of those groups having 1 or 2 hetero-atoms in a ring, the hetero-atoms may be freely chosen from nitrogen, oxygen and sulphur atoms. Preferably, however, the group contains at least one nitrogen atom. Examples of such aromatic heterocyclic groups include the 2-pyridyl, 3-pyridyl, 2-pyrimidinyl, 2-imidazolyl, 3-thiazolyl, 2-thienyl and 2-furyl groups. These groups may be unsubstituted or they may have one or more of substituents C, defined and exemplified above.

Where R⁴ represents an alkyl group which has from 1 to 6 carbon atoms and which is substituted by at least one substituent selected from aromatic heterocylic groups and fused heterocyclic groups, the heterocyclic groups may be as defined and exemplified above; and the alkyl groups may be any of the alkyl groups having from 1 to 6, preferably from 1 to 4 and more preferably 1, 2 or 3, carbon atoms defined and exemplified above in relation to R² etc., the methyl and ethyl groups being most preferred.

In the compounds of formula (IV), R¹ may be the same as in the compounds of formula (I) and (II). In these compounds, particularly preferred groups are the:
alkyl groups, especially the methyl, ethyl and t-butyl groups,
aralkyl groups, especially the benzyl, benzhydryl, 4-nitrobenzyl and 2-nitrobenzyl groups,
alkenyl groups, especially the allyl, 2-chloroallyl and 2-methylallyl groups,
haloalkyl groups, especially the 2,2,2-trichloroethyl and 2,2,2-tribromoethyl groups, and
the 2-trimethylsilylethyl group.

Also, R⁴¹ may be a hydrogen atom or a hydroxy-protecting group, which latter may be any of the groups defined and exemplified above in relation to R⁶. Particularly preferred such groups in the case of R⁴¹ are:
tri-substituted silyl groups, such as those previously exemplified, particularly the t-butyldimethylsilyl, t-butyldiphenylsilyl, trimethylsilyl and triethylsilyl groups;
aralkyloxycarbonyl groups, such as those defined and exemplified above, preferably the benzyloxycarbonyl and 4-nitrobenzyloxycarbonyl groups; and
unsubstituted and substituted acetyl groups, particularly those in which the substituents are selected from substituents C, defined and exemplified above, and especially the acetyl, chloroacetyl and methoxyacetyl groups.

R⁴², R⁴³, R⁴⁵ and R⁴⁶ may represent an alkyl group having from 1 to 6 carbon atoms, which may be as defined and exemplified above in relation to R² and R³. Particularly preferred such groups are the methyl, ethyl, propyl, isobutyl, pentyl and hexyl groups, of which the methyl, ethyl and propyl groups are most preferred.

R⁴² and R⁴³ may represent an alkoxy group having from 1 to 6 carbon atoms, which may be as defined and exemplified above in relation to R⁷. Particularly preferred such groups are the methoxy, ethoxy, propoxy, isobutoxy, pentyloxy and hexyloxy groups, of which the methoxy, ethoxy and propoxy groups are most preferred.

R⁴² and R⁴³ may represent a halogen atom, for example a fluorine, chlorine, bromine or iodine atom, of which the fluorine, chlorine and bromine atoms are preferred and the fluorine and chlorine atoms are more preferred.

R⁴², R⁴⁵ and R⁴⁶ may represent an aryl group, which may be as defined and exemplified above in relation to R⁴. A particularly preferred such group is the phenyl group. Where R⁴² represents an aryloxy group, this may be the aryloxy equivalent of any of the aryl groups defined and exemplified above in relation to R⁴. A particularly preferred such group is the phenoxy group.

Preferred compounds of formula (I) are those in which:
(1) R¹ represents:
   a hydrogen atom,
   an alkyl group having from 1 to 6 carbon atoms,
   an aralkyl group which is an alkyl group having from 1 to 4 carbon atoms and which is substituted by at least one aryl group, as defined above, an alkenyl group having from 2 to 6 carbon atoms, and which is unsubstituted or is substituted by one or more halogen atoms,
   a haloalkyl group having from 1 to 6 carbon atoms,
   a tri-substituted silylethyl group, in which all three or two or one of the substituents are alkyl groups having from 1 to 5 carbon atoms, and none, one or two of the substituents are aryl groups, as defined above,
   an acyloxyalkyl group, in which the acyl part is an alkanoyl group having from 1 to 6 carbon atoms and the alkyl part has from 1 to 6 carbon atoms,
   an alkoxycarbonyloxyalkyl group in which the alkoxy and alkyl parts each have from 1 to 6 carbon atoms,
   a cycloalkoxycarbonyloxyalkyl group in which the cycloalkyl part has from 3 to 7 ring carbon atoms and may be unsubstituted or substituted by at least one of substituents C, defined above and the alkyl part has from 1 to 6 carbon atoms
   a (5-alkyl or 5-aryl -2-oxo-1,3-dioxolen-4-yl)methyl group in which the alkyl part has from 1 to 4 carbon atoms and the aryl part is as defined above; or
   the 3-phthalidyl group;
(2) R² and R³ are the same or different and each represents a hydrogen atom, an alkyl group having from 1 to 4 carbon atoms or a group of formula 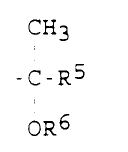
   where R⁵ represents a hydrogen atom or a methyl group; and R⁶ represents:
      a hydrogen atom,
      a substituted silyl group, in which the silyl group has 3 substituents selected from alkyl groups having from 1 to 6 carbon atoms and phenyl groups,
      an aralkyl group, in which an alkyl group having from 1 to 4 carbon atoms is substituted by at least one aryl group, as defined above,
      an aralkyloxycarbonyl group, in which the aralkyl part is as defined above,
      an alkenyloxycarbonyl group in which the alkenyl part has from 2 to 6 carbon atoms and is unsubstituted or is substituted by at least one halogen atom,
      an alkoxycarbonyl group which has from 2 to 7 carbon atoms,
      a substituted silylalkoxycarbonyl group, in which the alkoxycarbonyl part and the substituted silyl part are as defined above,
      an oxygen-containing heterocyclic group,
      an alkoxyalkyl group in which the alkoxy and alkyl parts each have from 1 to 6 carbon atoms, a substituted silylalkoxyalkyl group, in which the alkoxyalkyl part and the substituted silyl part are as defined above, or
      an alkanoyl or haloalkanoyl group which has no more than 6 carbon atoms;
   or
R² and R³ together represent a group of formula =C(CH₃)CH₂OR⁶, in which R⁶ is as defined above;
(3) X represents a sulphur atom or a group of formula >CHR⁷, where R⁷ represents a hydrogen atom, an alkyl group having from 1 to 4 carbon atoms or an alkoxy group having from 1 to 4 carbon atoms; and
(4) A represents
   an alkyl group which has from 1 to 4 carbon atoms and which is unsubstituted or is substituted by 1 or 2 of substituents K^{a}, defined below,
   an aryl group, as defined below,
   an aralkyl group in which an alkyl group having from 1 to 3 carbon atoms is substituted by at least one aryl group as defined below,
   an aromatic or non-aromatic heterocylic group which may be monocyclic or may consist of two or more rings attached to each other by fusion or by a spiro attachment and which has from 4 to 7 ring atoms, at least one of said atoms being a nitrogen and/or oxygen and/or sulfur hetero-atom, said group being unsubstituted or being substituted by at least one of substituents B^{a} on carbon atoms, substituents B^{a1} on nitrogen hetero-atoms, and oxygen atoms to form a sulphinyl or sulphonyl group on sulphur hetero-atoms, all as defined below;
   an alkyl group which has from 1 to 3 carbon atoms and which is substituted by at least one of the heterocyclic groups defined above;
said aryl groups are aromatic carbocyclic groups having from 6 to 10 ring carbon atoms and are unsubstituted or are substituted by at least one of substituents C^{a}, defined below;
said substituents K^{a} are selected from hydroxy, protected hydroxy groups, amino, protected amino groups, groups of formula -C(=NR¹⁰)NR¹¹R¹², where
   R¹⁰, R¹¹and R¹² are the same or different and each represents a hydrogen atom, an amino-protecting group or an alkyl group having from 1 to 4 carbon atoms, or R¹¹ and R¹² together represent a group of formula -(CH₂)ₙ-, where n is an integer from 2 to 6, or R¹⁰ and R¹¹ together represent a group of formula -(CH₂)ₚ-, where p is 2 or 3, and
   groups of formula -NR¹³C(=NR¹⁴)R¹⁵, where
   R¹³ and R¹⁴ are the same or different and each represents a hydrogen atom, an amino-protecting group or an alkyl group having from 1 to 6 carbon atoms, and
   R¹⁵ represents a hydrogen atom, an alkyl group having from 1 to 4 carbon atoms, an amino or a protected amino group,
      or
   any two of R¹³, R¹⁴ and R¹⁵ together represent a group of formula -(CH₂)ₚ-, where p is 2 or 3;
said substituents B^{a} are selected from
   alkyl groups having from 1 to 4 carbon atoms,
   alkoxy groups having from 1 to 6 carbon atoms,
   hydroxy,
   halogen atoms,
   cyano,
   nitro,
   alkoxycarbonyl groups having from 2 to 5 carbon atoms,
   carboxy,
   oxygen atoms, to form with a ring carbon atom a carbonyl group,
   alkoxyalkyl groups in which the alkoxy and alkyl parts both have from 1 to 6 carbon atoms,
   haloalkyl groups having from 1 to 6 carbon atoms,
   alkanoyloxy groups having from 1 to 6 carbon atoms,
   groups of formula -NR⁸R⁹ and groups of formula -CONR⁸R⁹, where R⁸ and R⁹ are the same or different and each represents a hydrogen atom, an amino-protecting group, an alkyl group having from 1 to 4 carbon atoms or a phenyl group, or R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
   groups of formulae (B-I), (B-II), (B-III), (B-IV), (B-V), (B-VI), (B-VII), (B-VIII) and (B-IX) as defined above;
said substituents B^{a1} are selected from alkyl groups having from 1 to 4 carbon atoms, amino-protecting groups, groups of formula -C(=NR¹⁶)R¹⁷, where R¹⁶ and R¹⁷ are as defined above, amino-protecting groups and groups of formula -CONR¹⁸R¹⁹, where
   R¹⁸ and R¹⁹ are the same or different and each represents an alkyl group having from 1 to 4 carbon atoms, an alkanoyl group having from 2 to 5 carbon atoms or a benzoyl group;
said substituents C^{a} are selected from alkyl groups having from 1 to 4 carbon atoms, alkoxy groups having from 1 to 4 carbon atoms, halogen atoms, alkoxycarbonyl groups having from 2 to 5 carbon atoms, groups of formula -CONR⁸R⁹, where R⁸ and R⁹ are as defined above, cyano, hydroxy groups and nitro.
   More preferred compounds of formulae (I) and (II) of the present invention are those compounds in which:
(5) R¹ represents:
   a hydrogen atom,
   an alkyl group having from 1 to 4 carbon atoms,
   an aralkyl group which is an alkyl group having 1 or 2 carbon atoms and which is substituted by at least one phenyl group
   a haloalkyl group having from 1 to 4 carbon atoms,
   a tri-substituted silylethyl group, in which all three or two of the substituents are alkyl groups having from 1 to 4 carbon atoms, and none or one of the substituents are phenyl groups,
   an acyloxyalkyl group, in which the acyl part is an alkanoyl group having from 2 to 5 carbon atoms and the alkyl part has 1 or 2 carbon atoms,
   an alkoxycarbonyloxyalkyl group in which the alkoxy and alkyl parts each have from 1 to 4 carbon atoms,
   a (5-methyl-2-oxo-1,3-dioxolen-4-yl)methyl group, or
   a 3-phthalidyl group;
(6) R² and R³ are the same or different and each represents a hydrogen atom, an alkyl group having from 1 to 4 carbon atoms or a group of formula 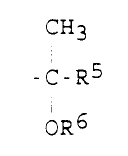
   where R⁵ represents a hydrogen atom or a methyl group; and R⁶ represents:
      a hydrogen atom,
      a substituted silyl group, in which the silyl group has 3 substituents selected from alkyl groups having from 1 to 4 carbon atoms and phenyl groups,
      an aralkyl group, in which an alkyl group having from 1 to 4 carbon atoms is substituted by at least one aryl group, as defined above,
      an alkoxycarbonyl group which has from 2 to 7 carbon atoms,
      a tetrahydropyranyl group,
      an alkoxyalkyl group in which the alkoxy and alkyl parts each have from 1 to 4 carbon atoms,
      an alkanoyl or haloalkanoyl group which has from 2 to 5 carbon atoms;
   or
R² and R³ together represent a group of formula =C(CH₃)CH₂OR⁶, in which R⁶ is as defined above;
(7) X represents a sulphur atom or a group of formula >CHR⁷, where R⁷ represents a hydrogen atom, an alkyl group having 1 or 2 carbon atoms or an alkoxy group having 1 or 2 carbon atoms; and
(8) A represents
   an alkyl group which has 1 or 2 carbon atoms and which is unsubstituted or is substituted by 1 or 2 of substituents K^{b}, defined below,
   a phenyl group, which is unsubstituted or is substituted by 1 or 2 of substituents C^{b}, defined below,
   an aralkyl group in which an alkyl group having from 1 to 3 carbon atoms is substituted by at least one phenyl group, which is unsubstituted or is substituted by 1 or 2 of substituents C^{b}, defined below,
   an aromatic or non-aromatic heterocylic group which may be monocyclic or may consist of two or more rings attached to each other by fusion or by a spiro attachment and which has 5 or 6 ring atoms, from 1 to 3 of said atoms being a nitrogen and/or oxygen and/or sulfur hetero-atom, said group being unsubstituted or being substituted by at least one of substituents B^{b} on carbon atoms, at least one of substituents B^{b1} on nitrogen hetero-atoms, and/or at least one oxygen atom to form a sulphinyl or sulphonyl group on sulphur hetero-atoms, all as defined below;
said substituents K^{b} are selected from hydroxy, protected hydroxy groups, amino and protected amino groups;
said substituents B^{b} are selected from
   alkyl groups having 1 or 2 carbon atoms,
   alkoxy groups having 1 or 2 carbon atoms,
   hydroxy,
   halogen atoms,
   cyano,
   nitro,
   alkoxycarbonyl groups having from 2 to 5 carbon atoms,
   carboxy,
   haloalkyl groups having from 1 to 6 carbon atoms,
   alkanoyloxy groups having from 1 to 6 carbon atoms,
   groups of formula -NR⁸R⁹ and groups of formula -CONR⁸R⁹, where R⁸ and R⁹ are the same or different and each represents a hydrogen atom, an amino-protecting group, an alkyl group having 1 or 2 carbon atoms or a phenyl group, or R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
   groups of formulae (B-I), (B-II), (B-III), (B-IV), (B-V), (B-VI), (B-VII), (B-VIII) and (B-IX) as defined above;
said substituents B^{b1} are selected from alkyl groups having 1 or 2 carbon atoms, amino-protecting groups, groups of formula -C(=NR¹⁶)R¹⁷, where R¹⁶ and R¹⁷ are as defined above, amino-protecting groups and groups of formula -CONR¹⁸R¹⁹, where
   R¹⁸ and R¹⁹ are the same or different and each represents an alkyl group having 1 or 2 carbon atoms, an alkanoyl group having from 2 to 5 carbon atoms or a benzoyl group;
said substituents C^{b} are selected from alkyl groups having 1 or 2 carbon atoms, alkoxy groups having 1 or 2 carbon atoms, halogen atoms, groups of formula -CONR⁸R⁹, where R⁸ and R⁹ are as defined above, cyano, hydroxy and nitro.
   Preferred compounds of formula (IV) are those compounds in which:
(9) R¹ represents:
   a hydrogen atom,
   an alkyl group having from 1 to 6 carbon atoms,
   an aralkyl group which is an alkyl group having from 1 to 4 carbon atoms and which is substituted by at least one aryl group, as defined above,
   an alkenyl group having from 2 to 6 carbon atoms, and which is unsubstituted or is substituted by one or more halogen atoms,
   a haloalkyl group having from 1 to 6 carbon atoms,
   a tri-substituted silylethyl group, in which all three or two or one of the substituents are alkyl groups having from 1 to 5 carbon atoms, and none, one or two of the substituents are aryl groups, as defined above,
   an acyloxyalkyl group, in which the acyl part is an alkanoyl group having from 1 to 6 carbon atoms and the alkyl part has from 1 to 6 carbon atoms,
   an alkoxycarbonyloxyalkyl group in which the alkoxy and alkyl parts each have from 1 to 6 carbon atoms,
   a cycloalkoxycarbonyloxyalkyl groups in which the cycloalkyl part has from 3 to 7 ring carbon atoms and may be unsubstituted or substituted by at least one of substituents C, defined above and the alkyl part has from 1 to 6 carbon atoms
   a (5-alkyl or 5-aryl -2-oxo-1,3-dioxolen-4-yl)methyl group in which the alkyl part has from 1 to 4 carbon atoms and the aryl part is as defined above; or
   the 3-phthalidyl group;
(10) R⁴¹ represents:
   a hydrogen atom,
   a substituted silyl group, in which the silyl group has 3 substituents selected from alkyl groups having from 1 to 6 carbon atoms and phenyl groups,
   an aralkyl group, in which an alkyl group having from 1 to 4 carbon atoms is substituted by at least one aryl group, as defined above, an aralkyloxycarbonyl group, in which the aralkyl part is as defined above,
   an alkenyloxycarbonyl group in which the alkenyl part has from 2 to 6 carbon atoms and is unsubstituted or is substituted by at least one halogen atom,
   an alkoxycarbonyl group which has from 2 to 7 carbon atoms,
   a substituted silylalkoxycarbonyl group, in which the alkoxycarbonyl part and the substituted silyl part are as defined above,
   an oxygen-containing heterocyclic group,
   an alkoxyalkyl group in which the alkoxy and alkyl parts each have from 1 to 6 carbon atoms,
   a substituted silylalkoxyalkyl group, in which the alkoxyalkyl part and the substituted silyl part are as defined above, or
   an alkanoyl or haloalkanoyl group which has no more than 6 carbon atoms;
(11) R⁴² represents an alkyl group having from 1 to 4 carbon atoms, an alkoxy group having from 1 to 4 carbon atoms, a halogen atom, an aryl group as defined below or an aryloxy group in which the aryl part is as defined below;
(12) R⁴³ represents a hydrogen atom, an alkyl group having from 1 to 4 carbon atoms, an alkoxy group having from 1 to 4 carbon atoms, a hydroxy group, a halogen atom, a cyano group, a nitro group or a group of formula -NR⁸R⁹, where R⁸ and R⁹ are the same or different and each represents a hydrogen atom, an amino-protecting group, an alkyl group having from 1 to 4 carbon atoms or a phenyl group, or R⁸ and R⁹ together represent a group of formula (CH₂)_{q}-Oᵣ-(CH₂)ₛ-, where q and s are the same or different and each represents 0 or an integer of from 1 to 5 and r is 0 or 1, provided that (q+s) is an integer of at least 2; and
(13) R⁴⁵ and R⁴⁶ are the same or different and each represents an alkyl group having from 1 to 4 carbon atoms or an aryl group as defined below, or R⁴⁵ and R⁴⁶ together represent a group of formula -(CH₂)_{q}-Oᵣ-(CH₂)ₛ-, where
   q and s are the same or different and each represents 0 or an integer of from 1 to 5 and r is 0 or 1.

   More preferred compounds of formula (IV) are those in which:
(14) R¹ represents:
   a hydrogen atom,
   an alkyl group having from 1 to 4 carbon atoms,
   an aralkyl group which is an alkyl group having 1 or 2 carbon atoms and which is substituted by at least one phenyl group
   a haloalkyl group having from 1 to 4 carbon atoms,
   a tri-substituted silylethyl group, in which all three or two of the substituents are alkyl groups having from 1 to 4 carbon atoms, and none or one of the substituents are phenyl groups,
   an acyloxyalkyl group, in which the acyl part is an alkanoyl group having from 2 to 5 carbon atoms and the alkyl part has 1 or 2 carbon atoms,
   an alkoxycarbonyloxyalkyl group in which the alkoxy and alkyl parts each have from 1 to 4 carbon atoms,
   a (5-methyl-2-oxo-1,3-dioxolen-4-yl)methyl group, or
   a 3-phthalidyl group;
said aryl groups are aromatic carbocyclic groups having from 6 to 10 ring carbon atoms and are unsubstituted or are substituted by at least one of substituents C^{a}, defined above;
(15) R⁴¹ represents:
   a hydrogen atom,
   a substituted silyl group, in which the silyl group has 3 substituents selected from alkyl groups having from 1 to 4 carbon atoms and phenyl groups,
   an aralkyl group, in which an alkyl group having 1 or 2 carbon atoms is substituted by at least one aryl group, as defined above,
   an aralkyloxycarbonyl group, in which the aralkyl part is as defined above,
   an alkoxycarbonyl group which has from 2 to 5 carbon atoms,
   a tetrahydropyranyl group,
   an alkoxyalkyl group in which the alkoxy and alkyl parts each have from 1 to 4 carbon atoms,
   an alkanoyl or haloalkanoyl group which has from 2 to 5 carbon atoms;
(16) R⁴² represents an alkyl group having 1 or 2 carbon atoms, an alkoxy group having 1 or 2 carbon atoms, a halogen atom, a phenyl group or a phenoxy group;
(17) R⁴³ represents a hydrogen atom, an alkyl group having 1 or 2 carbon atoms, an alkoxy group having 1 or 2 carbon atoms, a hydroxy group, a halogen atom, a cyano group, a nitro group or a group of formula -NR⁸R⁹, where R⁸ and R⁹ are the same or different and each represents a hydrogen atom, an amino-protecting group, an alkyl group having 1 or 2 carbon atoms or a phenyl group, or R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
(18) R⁴⁵ and R⁴⁶ are the same or different and each represents an alkyl group having 1 or 2 carbon atoms or a phenyl group, or R⁴⁵ and R⁴⁶ together represent a group of formula -(CH₂)_{q}-Oᵣ-(CH₂)ₛ-, where
   q and s are the same or different and each represents 0 or an integer of from 1 to 5 and r is 0 or 1.

Specific examples of compounds of formulae (I) and (II) are given in the following Tables 1 and 2, and specific examples of compounds of formula (IV) of the present invention are shown in formulae (I-3) and (I-4), in which the substituents are as shown in the respective one of the following Tables 3 and 4, that is Table 3 relates to formula (I-3) and Table 4 relates to formula (I-4). Preferred compounds are those having the configuration shown in formulae (I-3a) and (I-4a). Certain of the groups represented by R⁴ are shown by the following formulae (1-1) to (1-30).

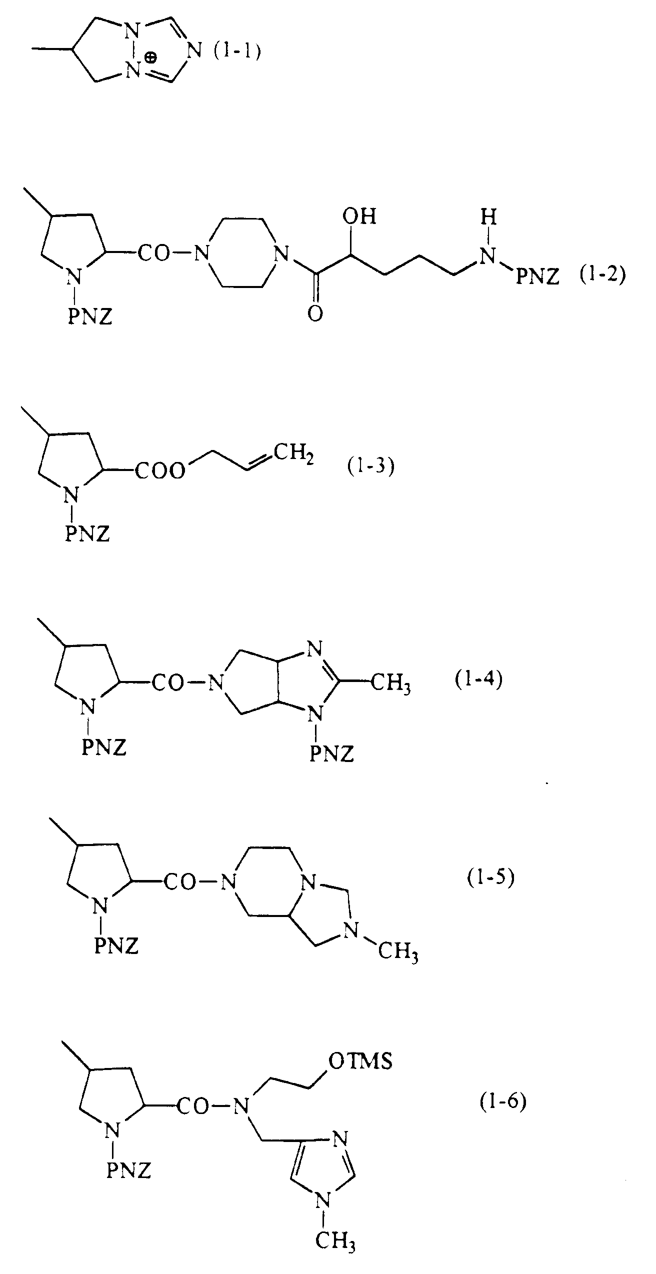

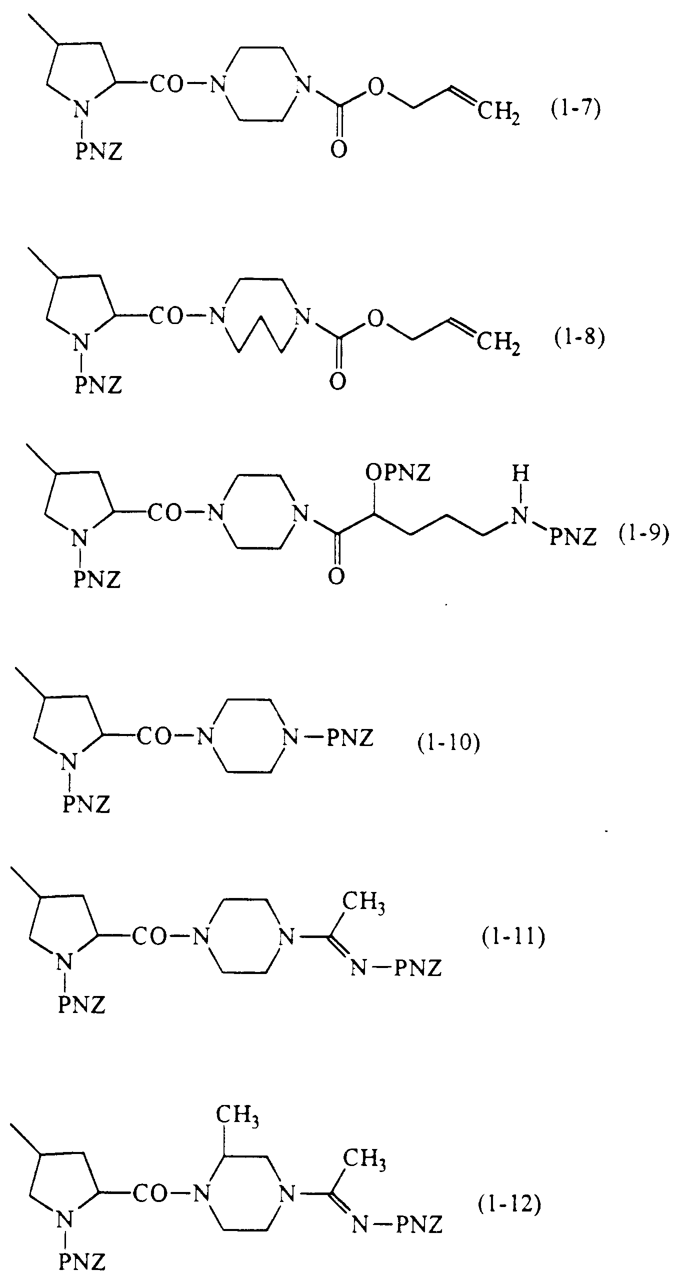

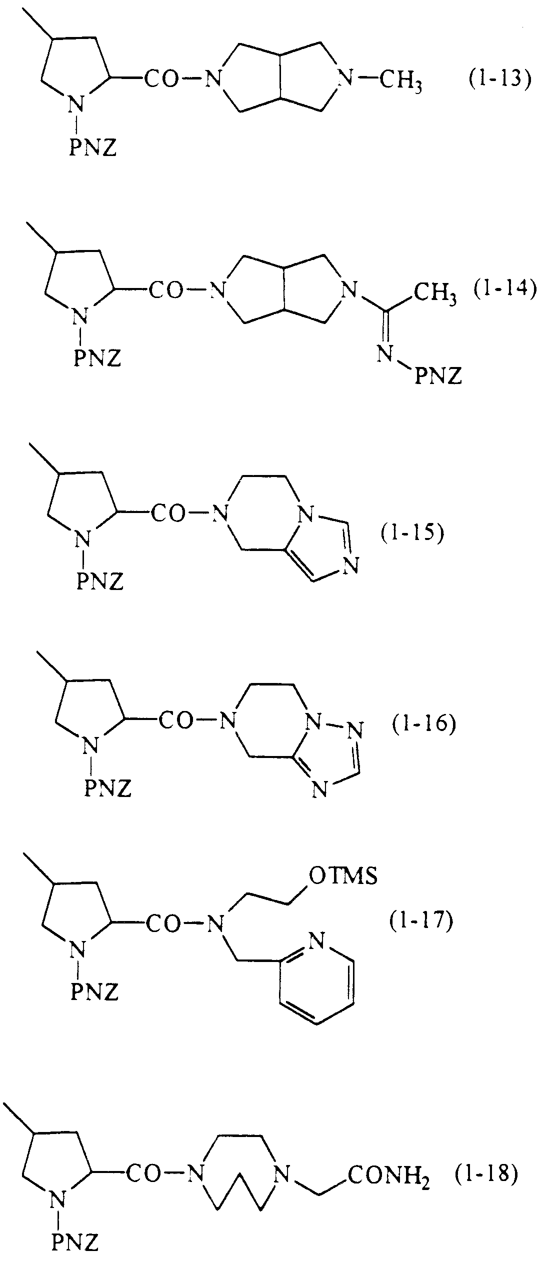

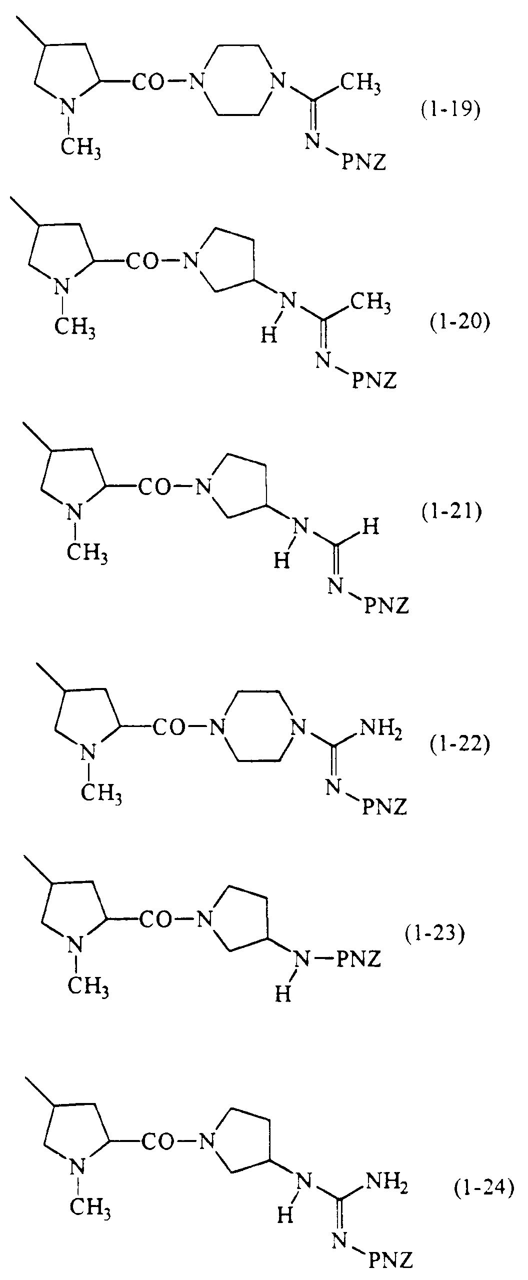

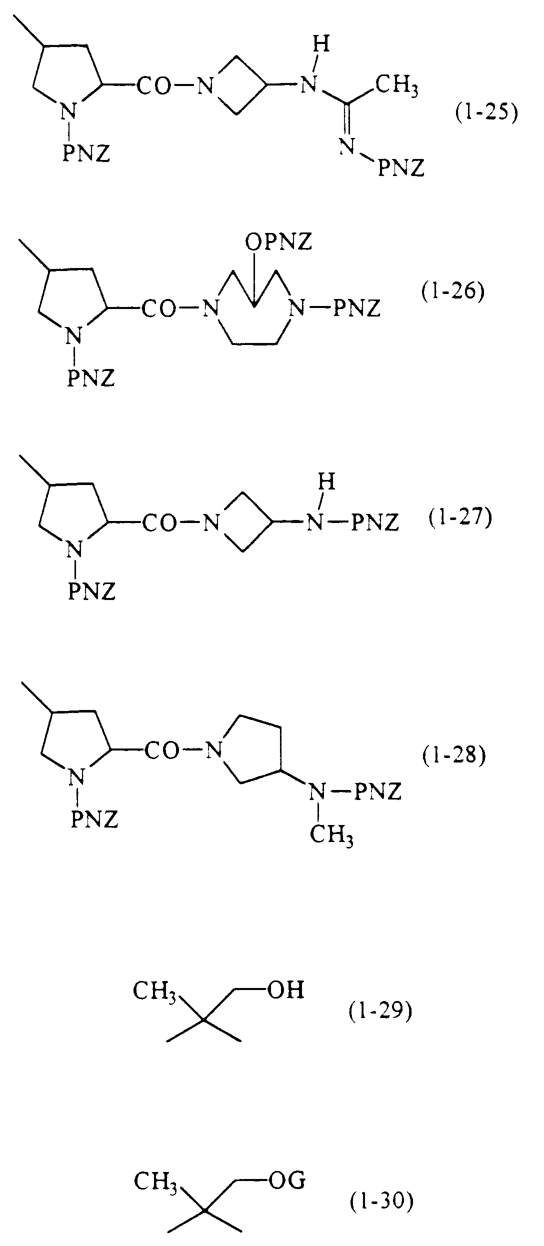

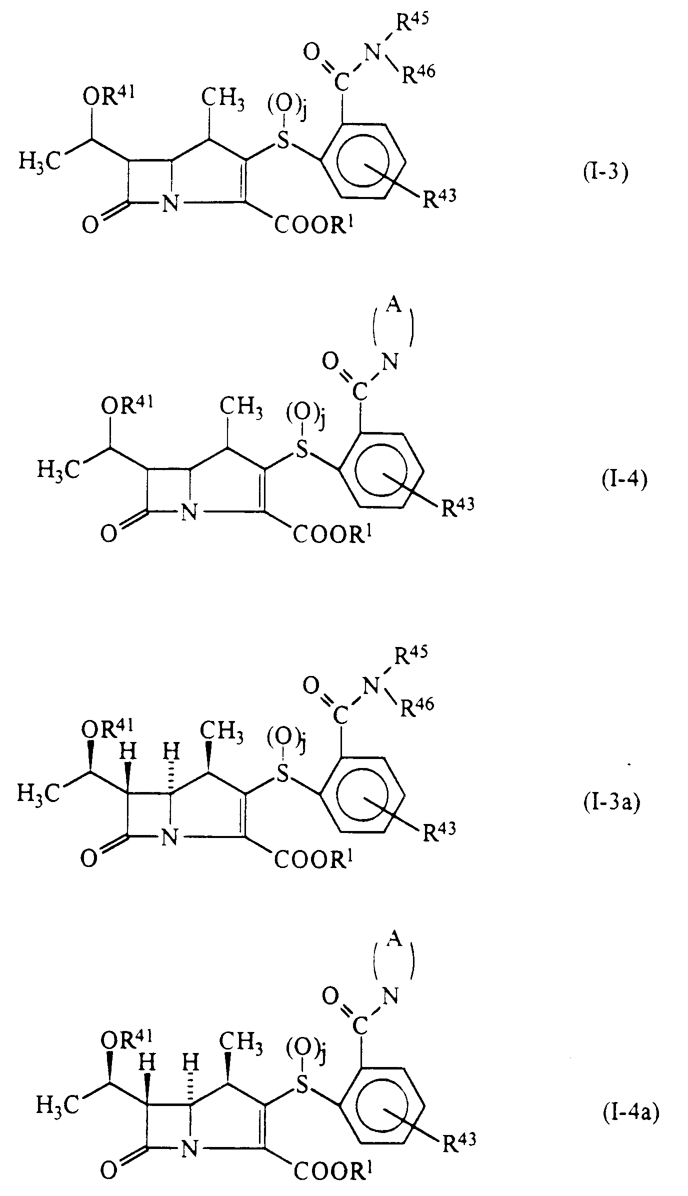

In the Tables, the following abbreviations are used:
- Ac: acetyl
- All: allyl
- Azp: perhydroazepinyl
- Bu: butyl
- iBu: isobutyl
- Bz: benzyl
- Et: ethyl
- Imid: imidazolyl
- Ithiz: isothiazolyl
- Me: methyl
- Ph: phenyl
- Piz: piperazinyl
- PNB: 4-nitrobenzyl
- PNZ: 4-nitrobenzyloxycarbonyl
- Pr: propyl
- iPr: isopropyl
- Pyr: pyridyl
- Pyrd: pyrrolidinyl
- Pyrz: pyrazolidinyl
- TBS: t-butyldimethylsilyl
- Tht: 1-oxotetrahydrothien-3-yl
- TMS: trimethylsilyl
- G: hydrogen, TMS, TBS or PNZ

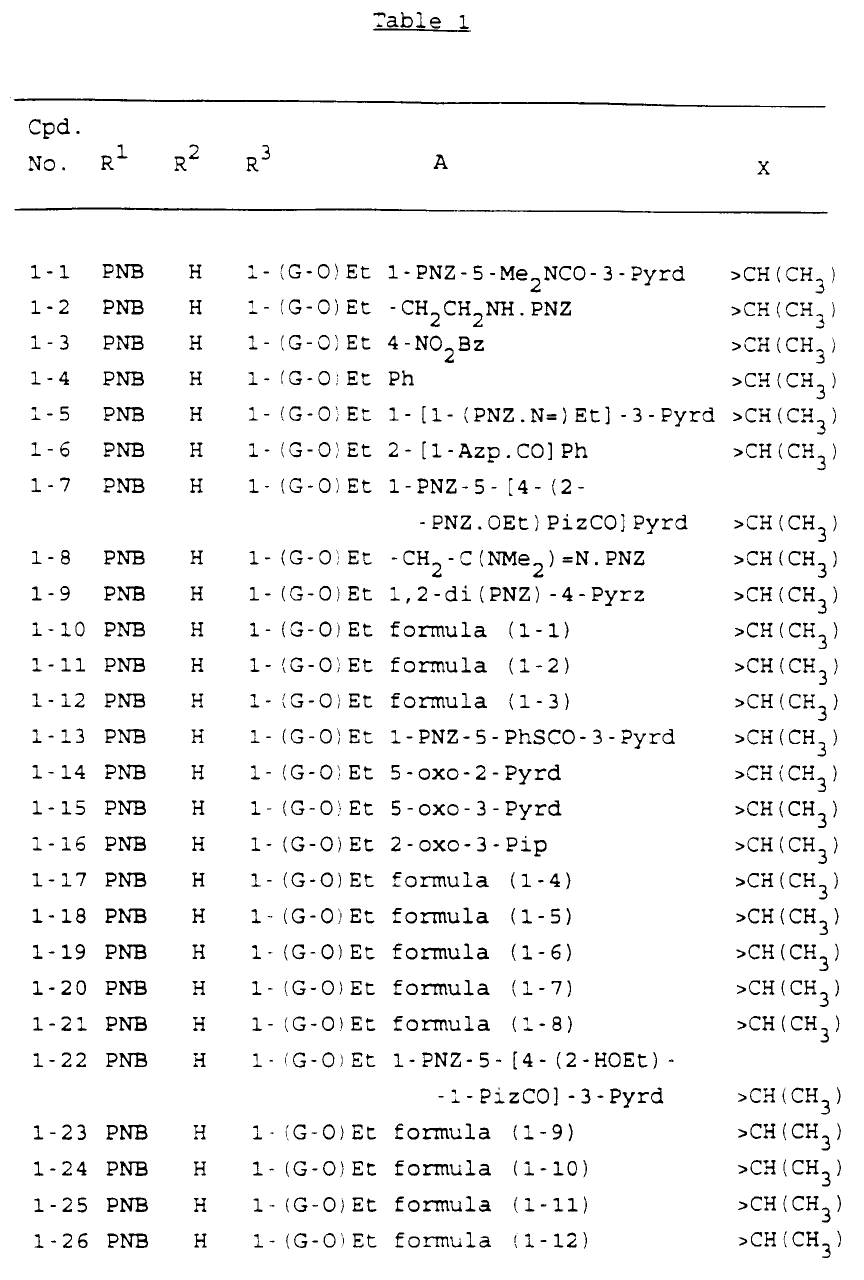

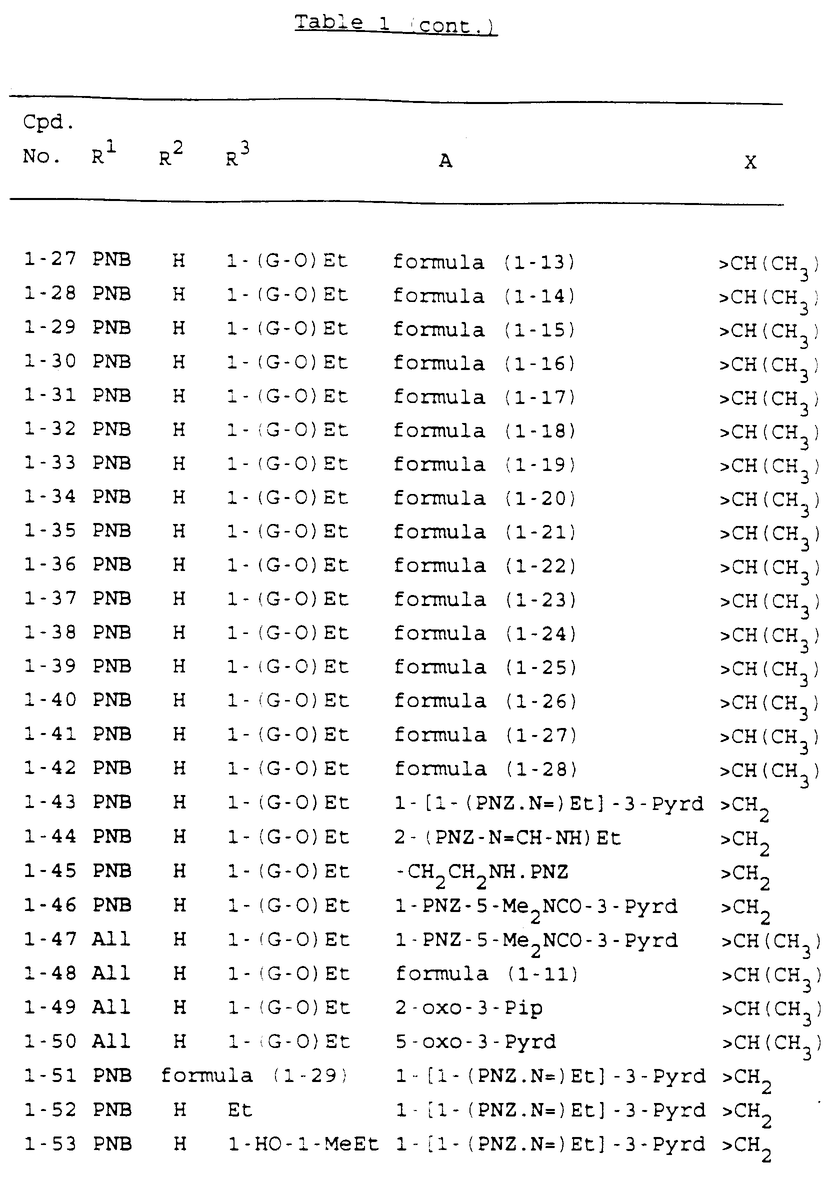

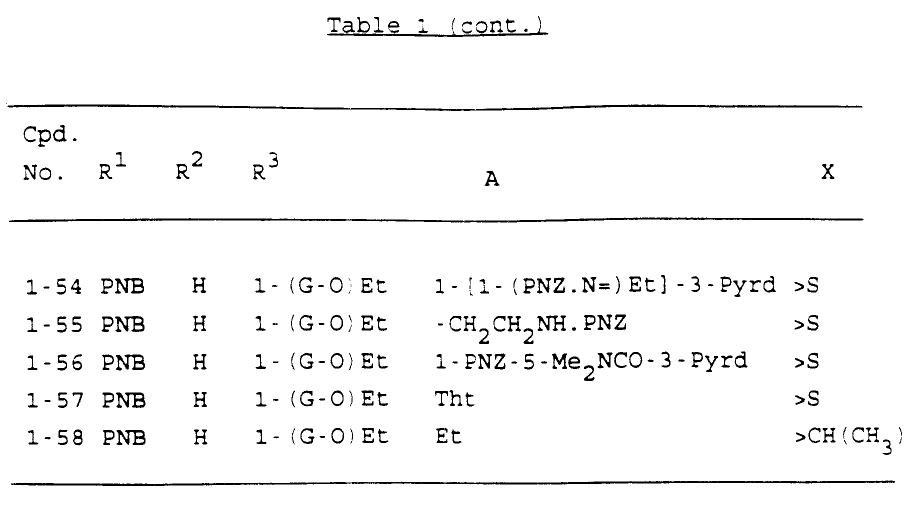

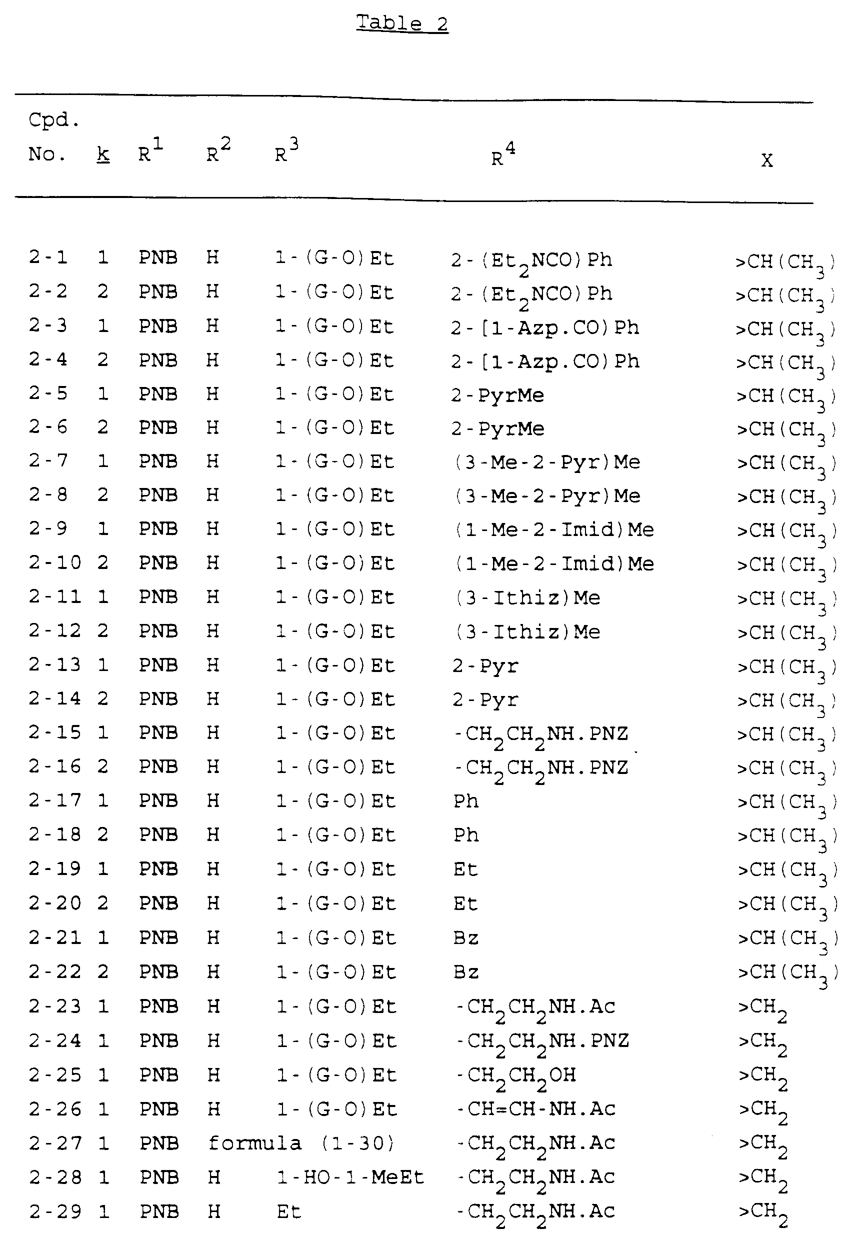

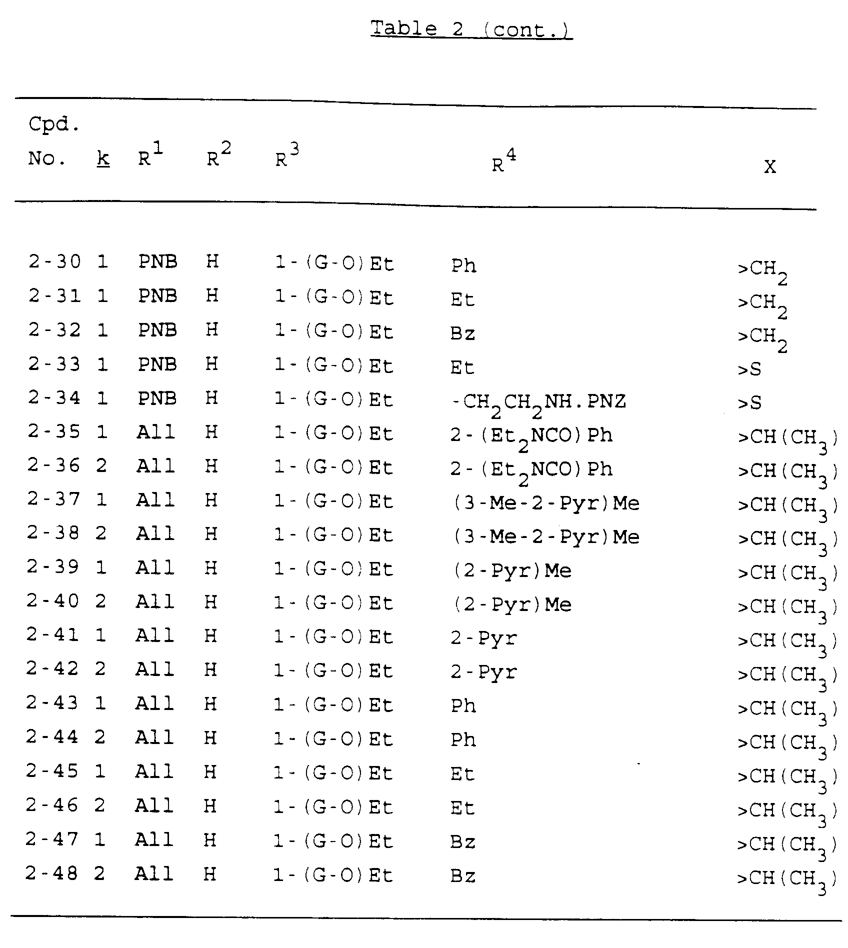

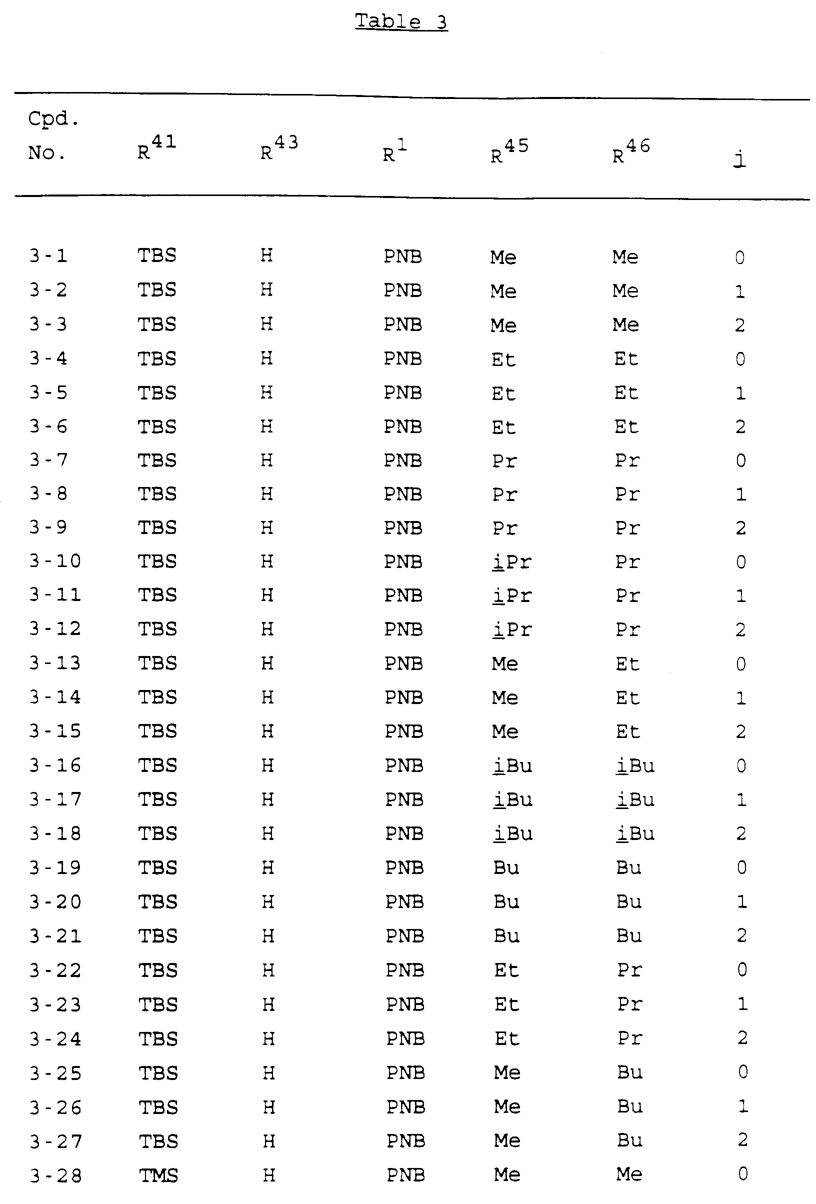

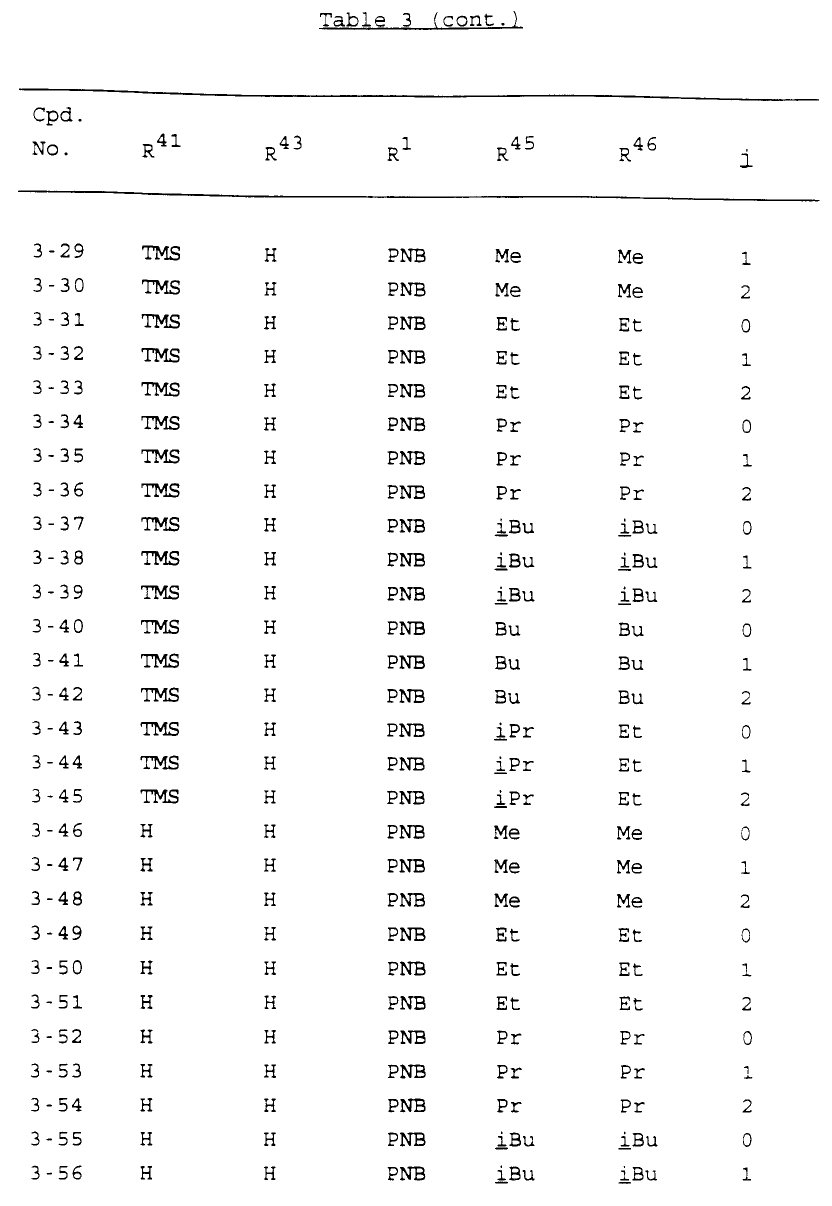

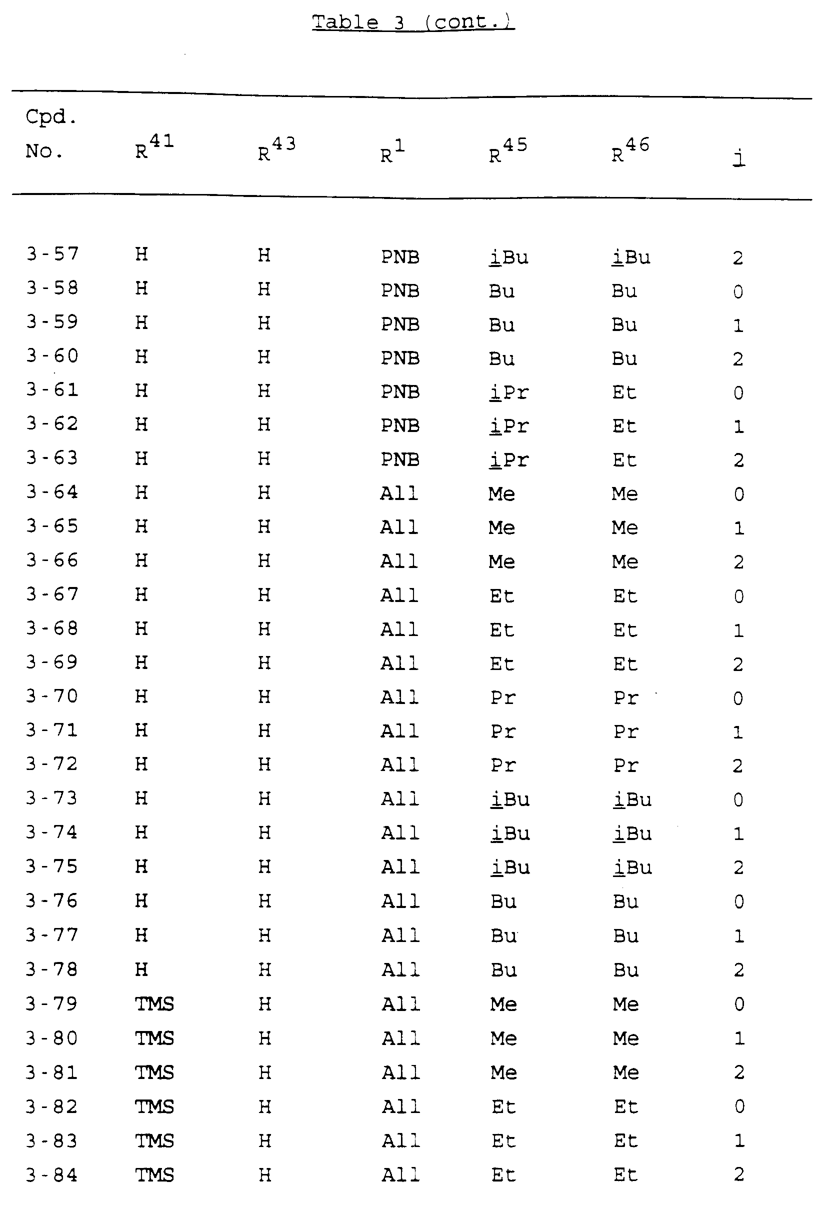

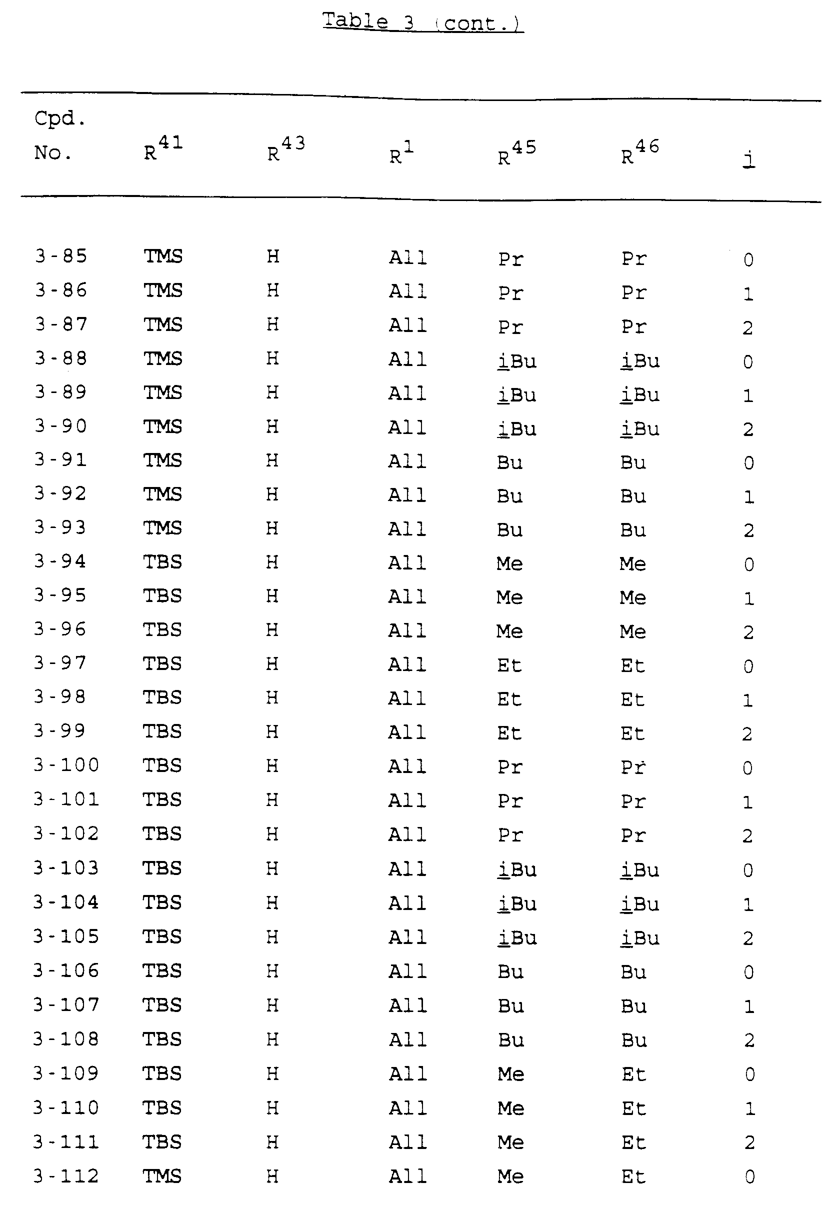

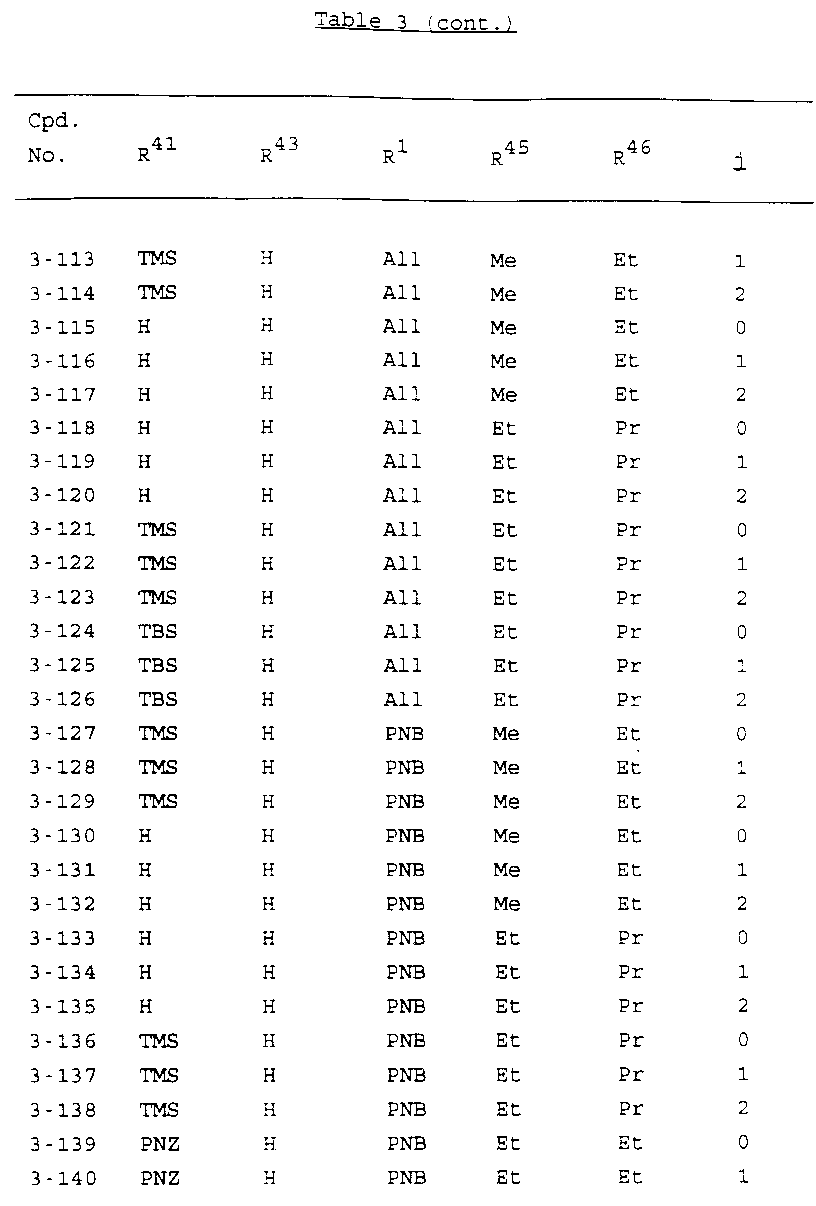

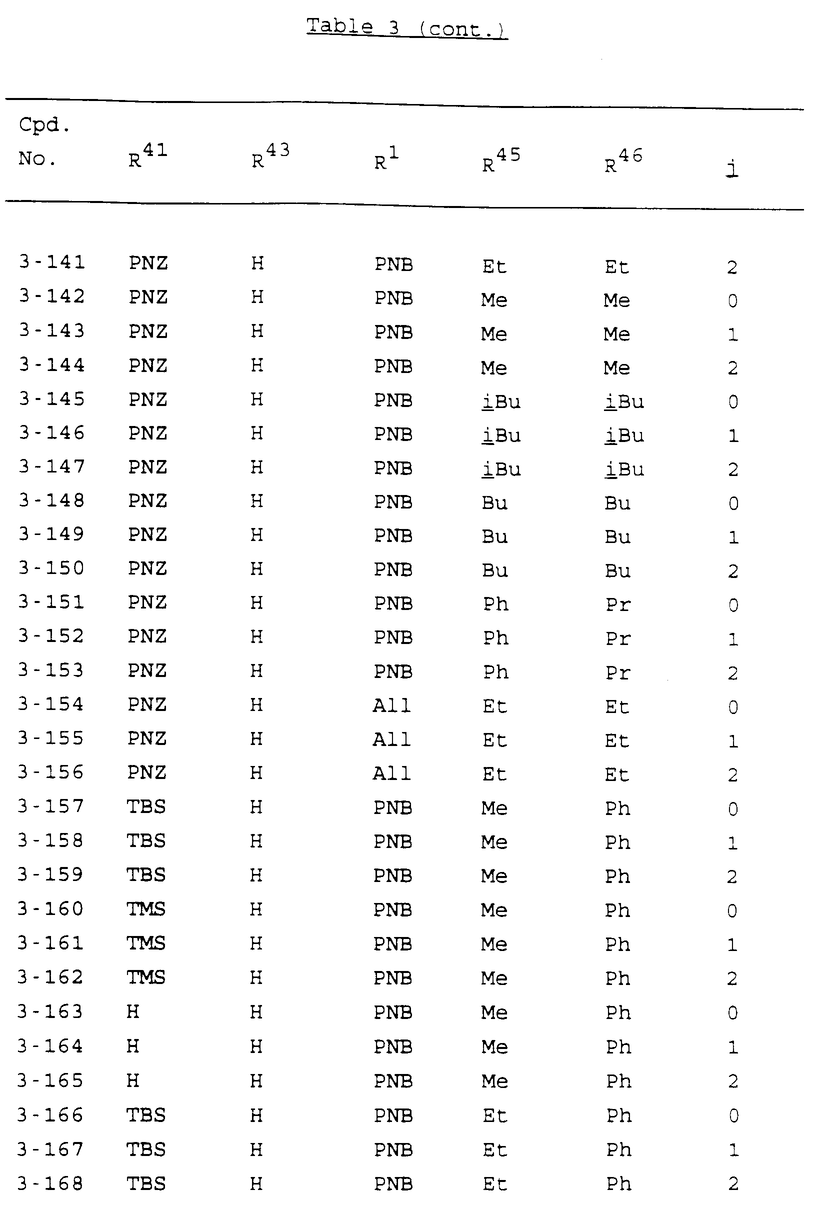

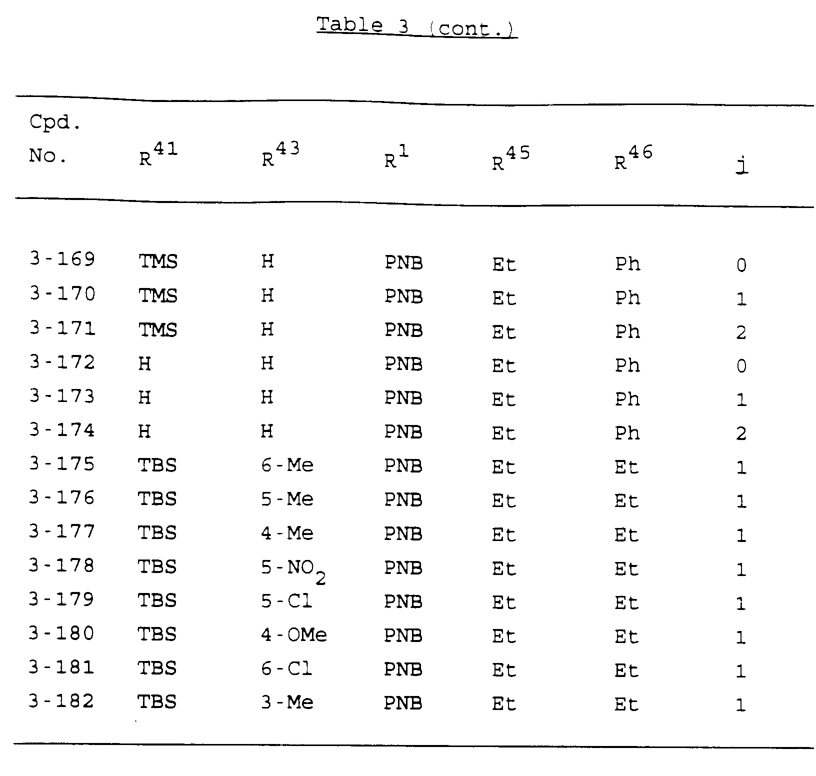

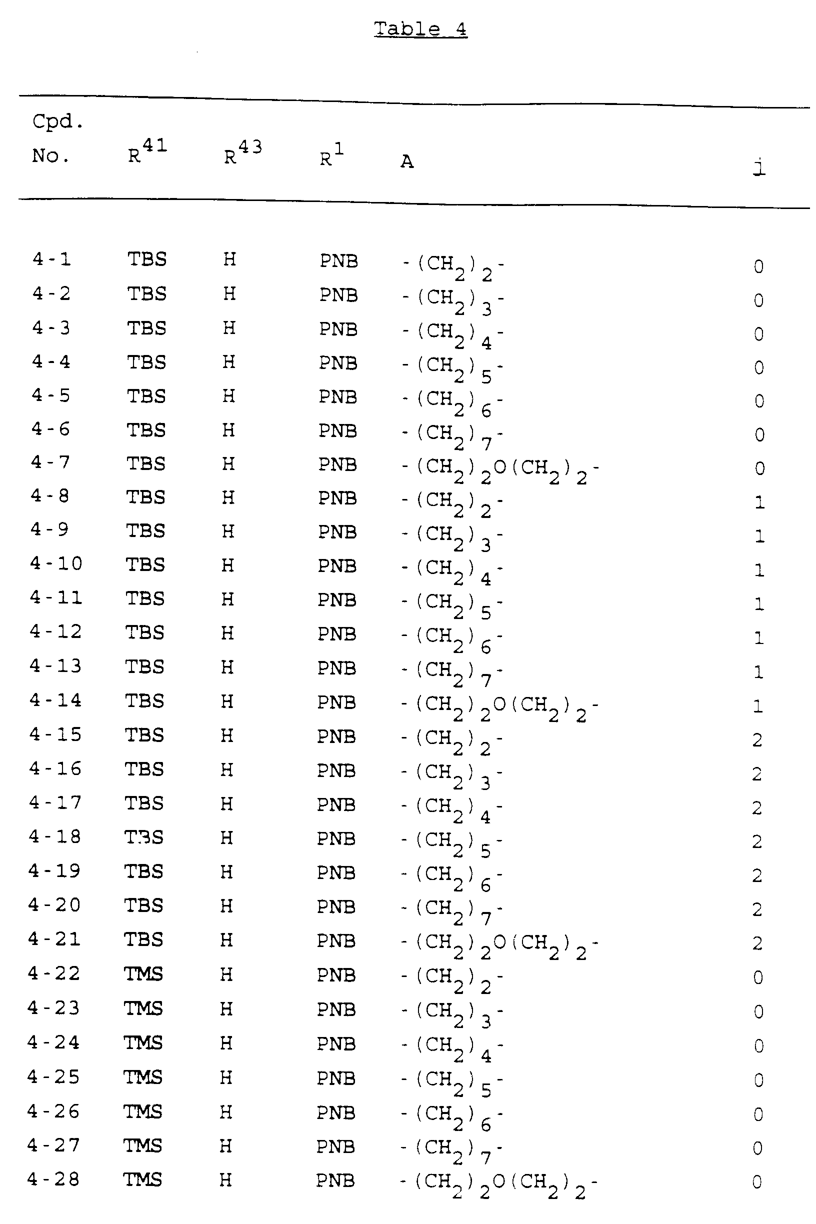

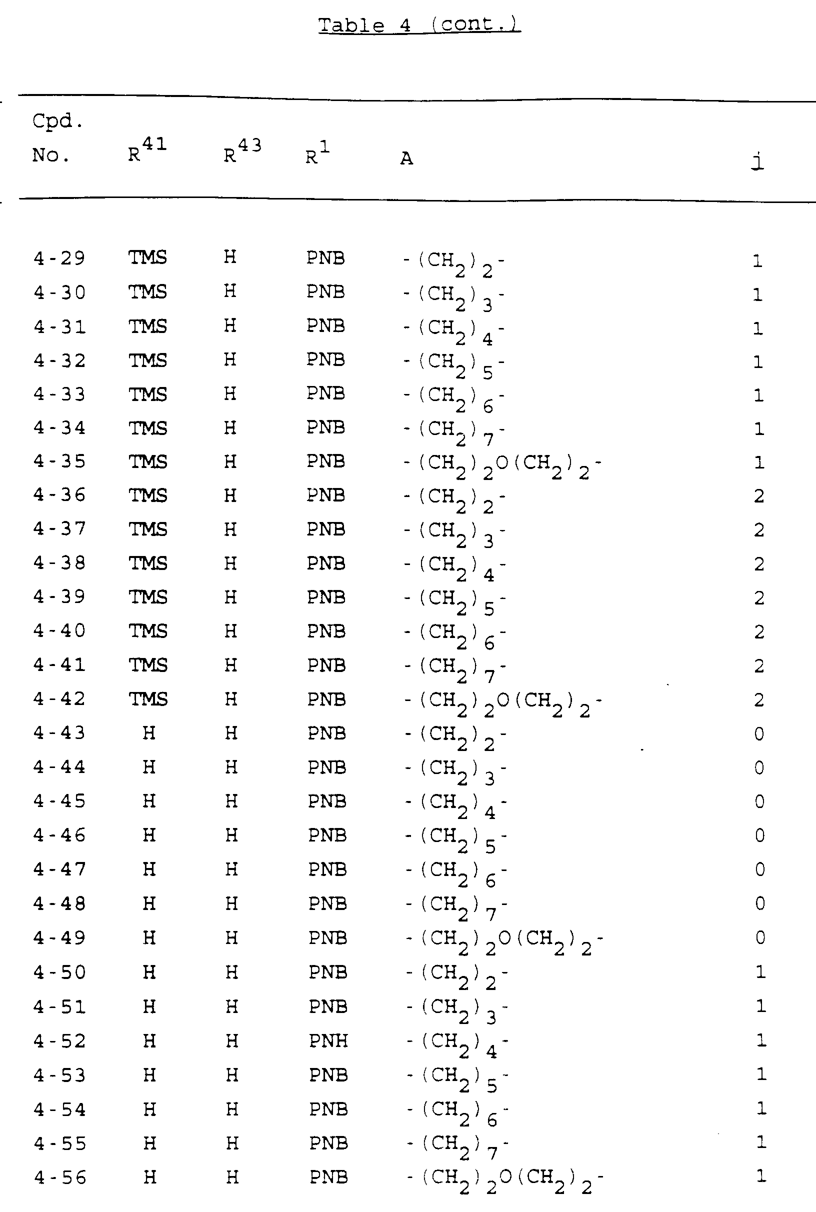

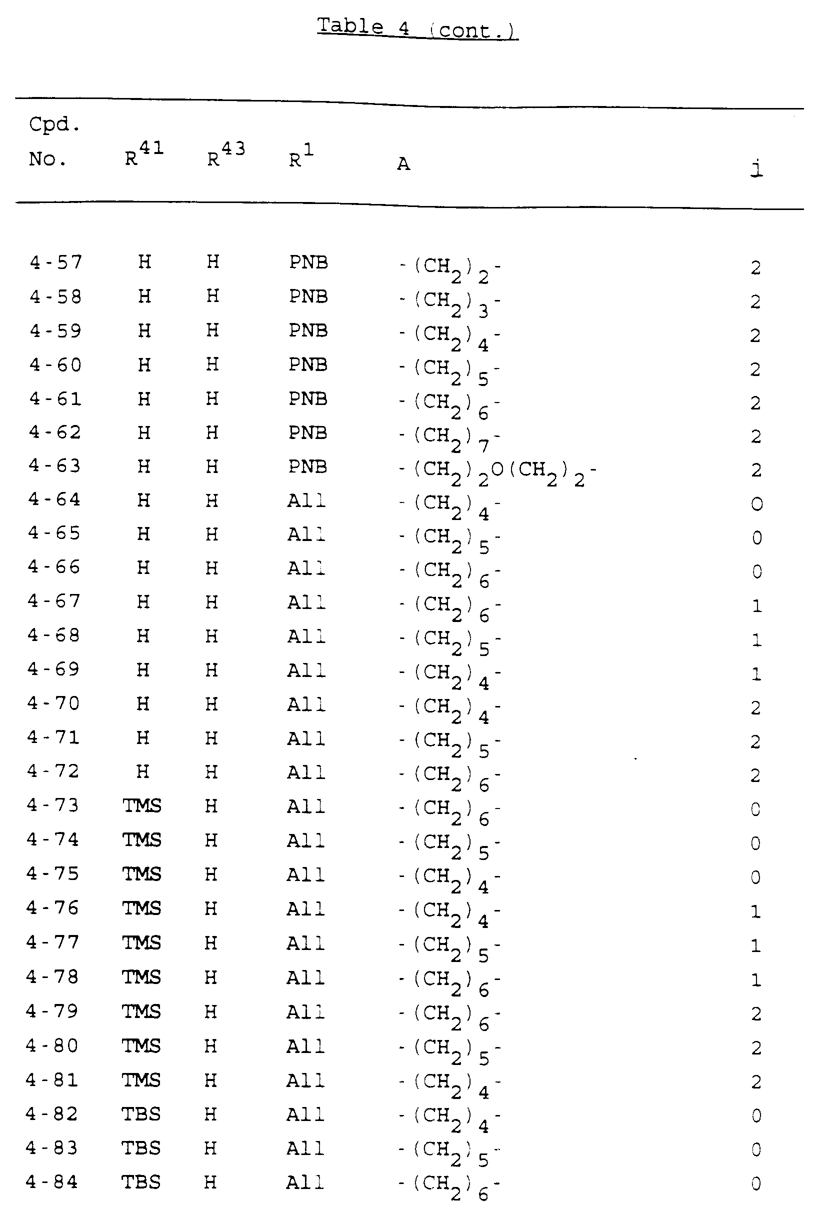

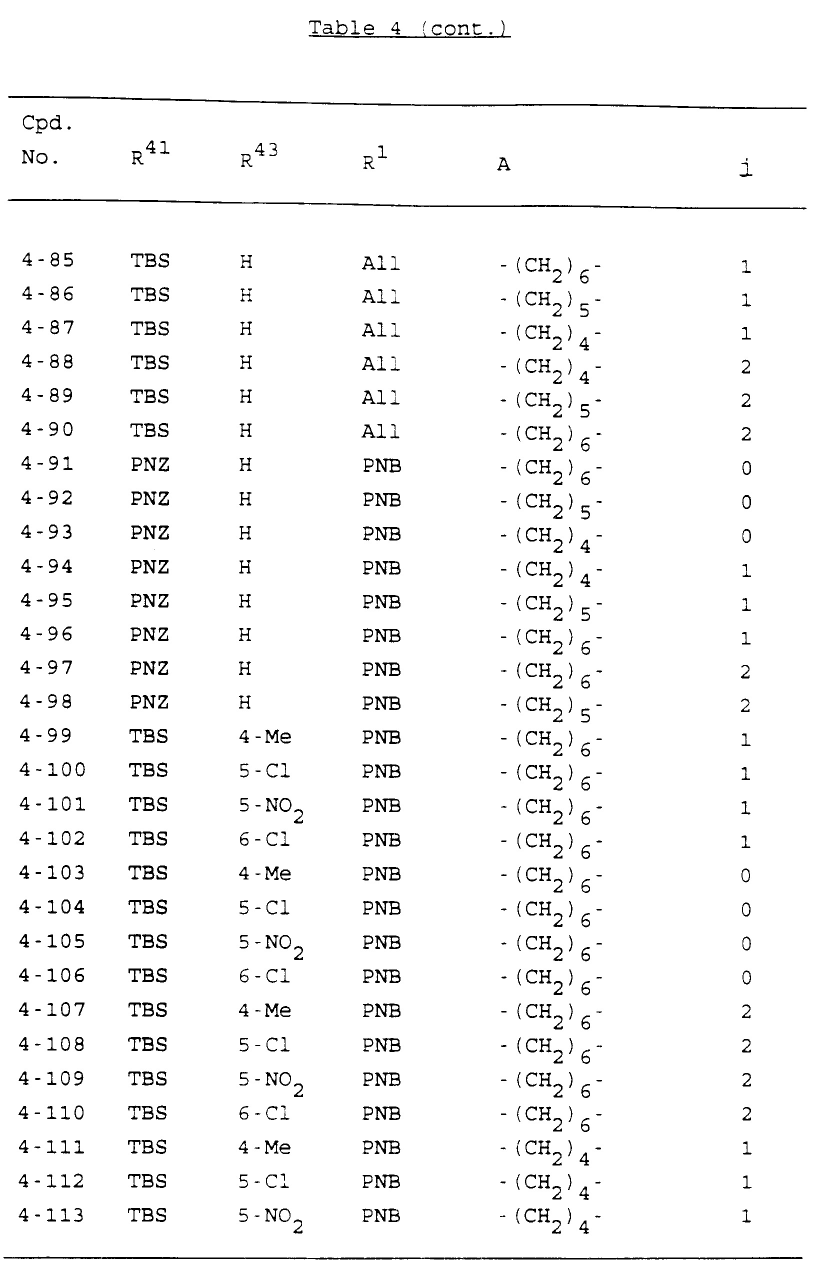

Of the compounds listed in above, preferred compounds are Compounds No. 3-1, 3-2, 3-3, 3-4, 3-5, 3-6, 3-7, 3-8, 3-9, 3-28, 3-29, 3-30, 3-31, 3-32, 3-33, 3-34, 3-35, 3-36, 3-46, 3-47, 3-48, 3-49, 3-50, 3-51, 3-52, 3-53, 3-54, 3-64, 3-65, 3-66, 3-67, 3-68, 3-69, 3-79, 3-80, 3-81, 3-82, 3-83, 3-84, 4-3, 4-4, 4-5, 4-7, 4-10, 4-11, 4-12, 4-14, 4-17, 4-18, 4-19, 4-21, 4-24, 4-26, 4-28, 4-31, 4-33, 4-35, 4-38, 4-40, 4-42, 4-45, 4-47, 4-49, 4-52, 4-54, 4-56, 4-59, 4-61 and 4-63. More preferred compounds are Compounds No. 3-4, 3-5, 3-6, 3-31, 3-32, 3-33, 3-49, 3-50, 3-51, 4-5, 4-12, 4-19, 4-26, 4-33, 4-40, 4-47, 4-54 and 4-61. The most preferred compounds are Compounds No.
3-31. 4-Nitrobenzyl 2-(2-diethylcarbamoylphenylthio)-1-methyl-6-(1-trimethylsilyloxyethyl)-1-carbapen-2-em-3-carboxylate;
3-32. 4-Nitrobenzyl 2-(2-diethylcarbamoylphenylsulphinyl)-1-methyl-6-(1-trimethylsilyloxyethyl)-1-carbapen-2-em-3-carboxylate;
3-33. 4-Nitrobenzyl 2-(2-diethylcarbamoylphenylsulphonyl)-1-methyl-6-(1-trimethylsilyloxyethyl)-1-carbapen-2-em-3-carboxylate;
3-49. 4-Nitrobenzyl 2-(2-diethylcarbamoylphenylthio)-1-methyl-6-(1-hydroxyethyl)-1-carbapen-2-em-3-carboxylate;
3-50. 4-Nitrobenzyl 2-(2-diethylcarbamoylphenylsulphinyl)-1-methyl-6-(1-hydroxyethyl)-1-carbapen-2-em-3-carboxylate;
3-51. 4-Nitrobenzyl 2-(2-diethylcarbamoylphenylsulphonyl)-1-methyl-6-(l-hydroxyethyl)-1-carbapen-2-em-3-carboxylate;
4-26. 4-Nitrobenzyl 2-[2-(1-perhydroazepinylcarbonyl)phenylthio]-1-methyl-6-(1-trimethylsilyloxyethyl)-1-carbapen-2-em-3-carboxylate;
4-33. 4-Nitrobenzyl 2-[2-(1-perhydroazepinylcarbonyl)phenylsulphinyl]-1-methyl-6-(1-trimethylsilyloxyethyl)-1-carbapen-2-em-3-carboxylate;
4-40. 4-Nitrobenzyl 2-[2-(1-perhydroazepinylcarbonyl)phenylsulphonyl]-1-methyl-6-(1-trimethylsilyloxyethyl)-1-carbapen-2-em-3-carboxylate;
4-47. 4-Nitrobenzyl 2-[2-(1-perhydroazepinylcarbonyl)phenylthio]-1-methyl-6-(1-hydroxyethyl)-1-carbapen-2-em-3-carboxylate;
4-54. 4-Nitrobenzyl 2-[2-(1-perhydroazepinylcarbonyl)phenylsulphinyl]-1-methyl-6-(1-hydroxyethyl)-1-carbapen-2-em-3-carboxylate; and
4-61. 4-Nitrobenzyl 2-[2-(1-perhydroazepinylcarbonyl)phenylsulphonyl]-1-methyl-6-(1-hydroxyethyl)-1-carbapen-2-em-3-carboxylate.

In accordance with the present invention, compounds of formula (II), which include the novel compounds of formula (IV), may be converted to the desired compounds of formula (I) by reaction with a compound of formula (III), ASH (in which A is as defined above), in the presence of the salts of magnesium and aluminium noted above.

The reaction is normally and preferably carried out in the presence of a solvent, the nature of which is not critical, provided that it has no adverse effect upon the reaction and that it can dissolve the reagents, at least to some extent. Examples of suitable solvents include: hydrocarbons, especially alicyclic and aromatic hydrocarbons, such as cyclohexane, benzene, toluene or xylene; halogenated hydrocarbons, especially halogenated aliphatic hydrocarbons, such as methylene chloride, 1,2-dichloroethane or carbon tetrachloride; ethers, such as diethyl ether, 1,2-dimethoxyethane, tetrahydrofuran or dioxane; amides, such as dimethylformamide, dimethylacetamide, N-methyl-2-pyrrolidone or hexamethylphosphoric triamide; and sulphoxides, such as dimethyl sulphoxide. Of these, we prefer the hydrocarbons, the halogenated hydrocarbons, the ethers or the amides, the ethers being particularly preferred.

The reaction will take place over a wide range of temperatures, and the precise reaction temperature chosen is not critical to the invention. In general, we find it convenient to carry out the reaction at a temperature in the range of from -70°C to 60°C, more preferably from 0°C to 30°C. The time required for the reaction may likewise vary widely, depending on many factors, notably the reaction temperature and the nature of the reagents. However, in most cases, a period of from 10 minutes to 48 hours will normally suffice if a sulphinyl derivative is used, and from 10 minutes to several days will normally suffice if a sulphonyl derivative is used.

A sulphinyl derivative of formula (IX) or a sulphonyl derivative of formula (X), which may be used as the starting material for this reaction, can be synthesized by oxidation of a compound of formula (XI) according to a known method using a suitable oxidizing agent, for example a peroxide, such as m-chloroperbenzoic acid, peracetic acid or t-butyl peroxide, as shown in the following Reaction Scheme 2:

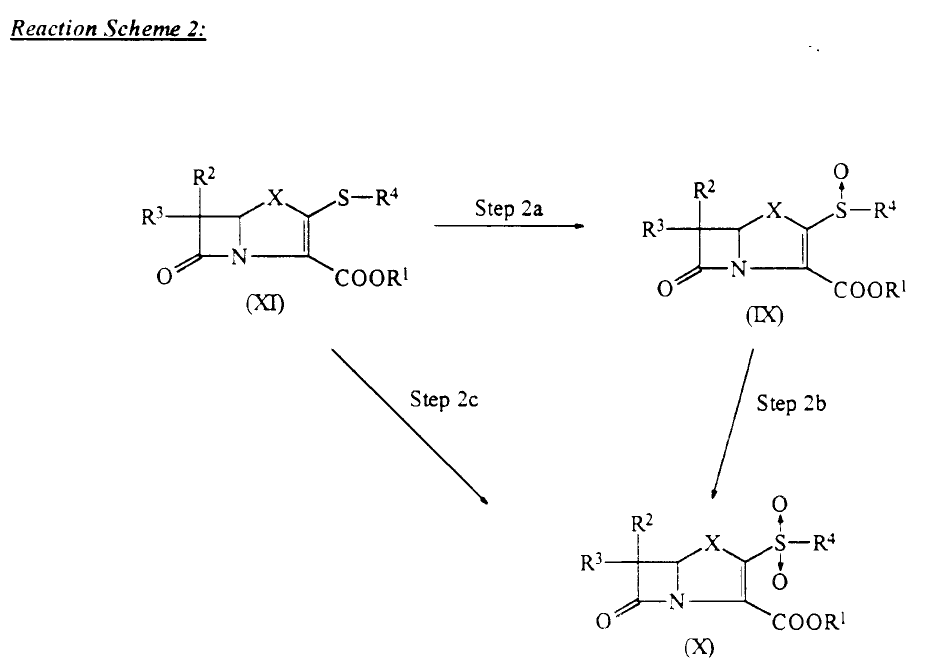

In the above formulae, R¹, R² R³ and R⁴ are as defined above.

When t-butyl peroxide is used, we prefer to employ vanadyl acetylacetonate [VO(acac)₂] as a catalyst. In addition, in order to remove any acidic substances originating from the peroxides which are formed in this reaction, the oxidation reaction is preferably conducted in the presence of a base, for example an alkali metal carbonate or an aqueous solution thereof.

A sulphonyl derivative of formula (X) can be synthesized either by further oxidation of the sulphinyl derivative of formula (IX) according to the method mentioned above or by direct oxidation of the compound of formula (XI).

These oxidation reactions are described in more detail in relation to Step 3d of Reaction Scheme 3.

The compound of formula (XI) employed as the starting material can be synthesized from a natural substance when X represents CH₂ (for example, as described by S. M. Schmitt et al.: J. Org. Chem. 1980, 45, 1142); or according to the reported method when X represents CH₂ or CH(CH₃) [for example, as described by S. Oida et al.: Tetrahedron Letters 25, 2793 (1984); B. G. Christensen et al.: Heterocycles 21, 29 (1984)].

When X represents a sulphur atom in the compound of formula (XI), the compound can be synthesized, for example, according to the following reported methods [S. Oida et al.: Chem. Pharm. Bull. 29, 3158 (1981); A. Yoshida et al.: Chem. Pharm. Bull. 31, 768 (1983)].

Preferred examples of compounds of formula ASH (III) employed in the present invention are those compounds of

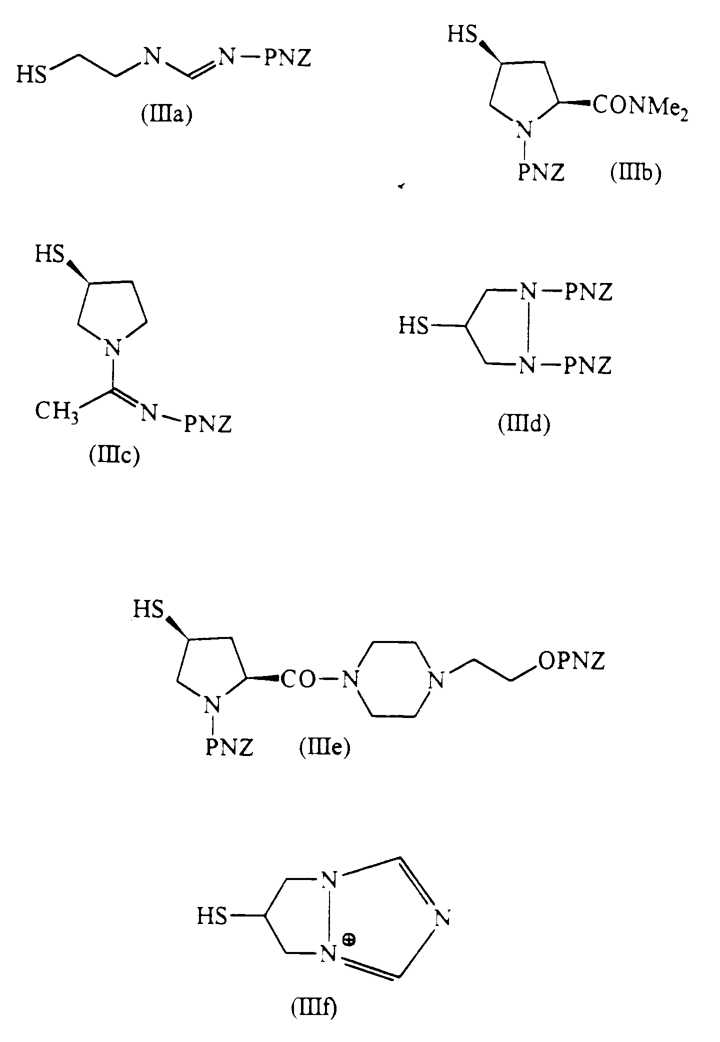

The compound of formula (I) which is obtainable by the method of the present invention can be converted into a corresponding carbapenem or penem derivative having excellent antibacterial activity, by removal of a carboxy-protecting group shown by R¹, and in some cases by removal of hydroxy-protecting groups shown by R² and R³ at position 6 and any protecting group in A by conventional means.

The compounds of formula (IV) of the present invention may be prepared, for example, as illustrated by the following Reaction Scheme 3:

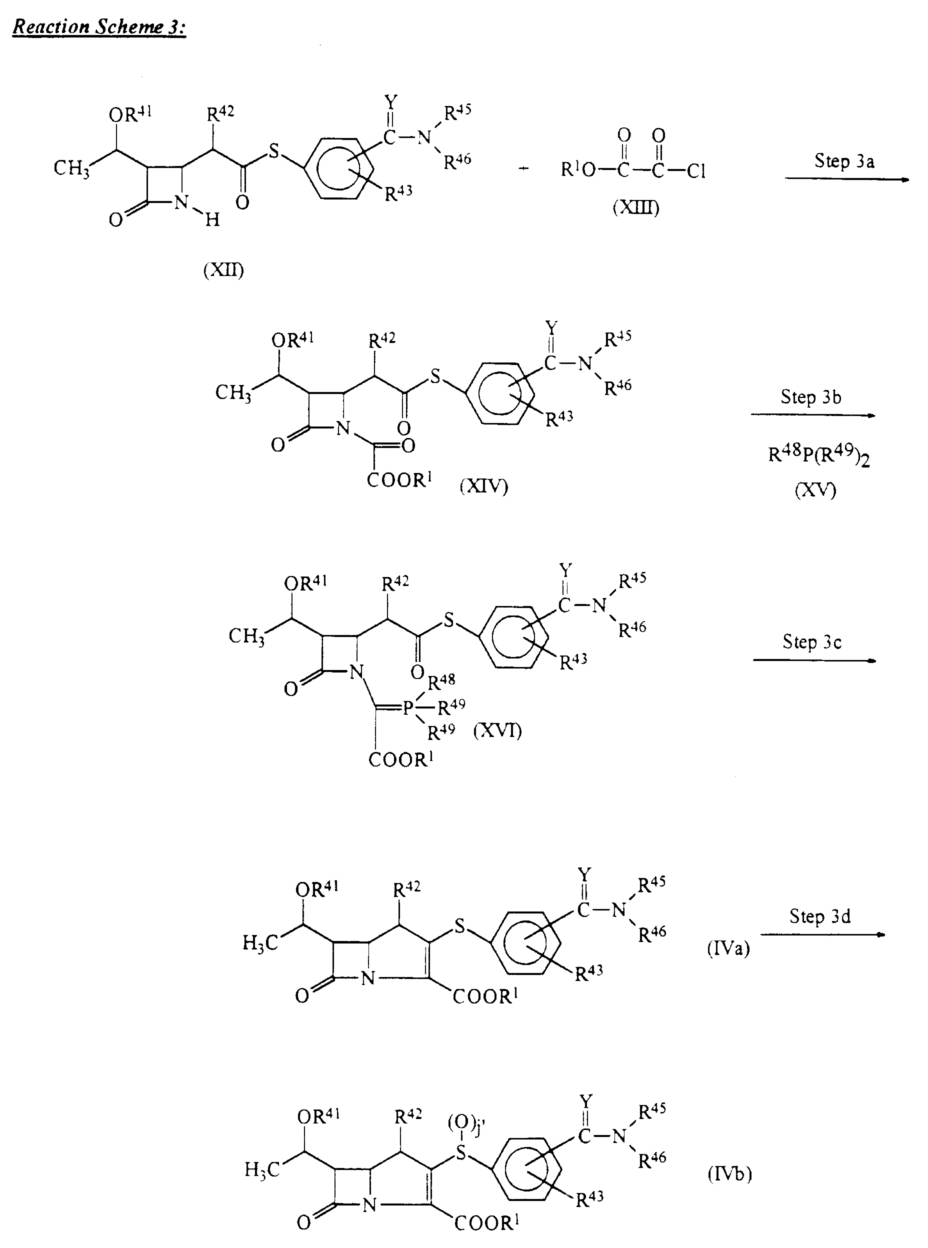

In the above formulae, R¹, R⁴¹, R⁴², R⁴³, R⁴⁵, R⁴⁶ and Y are as defined above.

R⁴⁸ represents an alkyl group having from 1 to 6 carbon atoms or an alkoxy group having from 1 to 6 carbon atoms. Where R⁴⁸ represents an alkyl group having from 1 to 6 carbon atoms, it may be, for example, any of those groups exemplified above in relation to R², particularly a methyl, ethyl, propyl, isopropyl, butyl or isobutyl group.

Where R⁴⁸ represents an alkoxy group having from 1 to 6 carbon atoms, it may be, for example, any of those groups exemplified above in relation to R⁷, particularly a methoxy, ethoxy, propoxy, isopropoxy, butoxy, isobutoxy or t-butoxy group.

R⁴⁹ represents an alkoxy group having from 1 to 6 carbon atoms or an aryloxy group (in which the aryl part may be as generally defined and exemplified above). Where R⁴⁹ represents an alkoxy group, it may be the same alkoxy group as defined and exemplified for R⁴⁸.

Where R⁴⁹ represents an aryloxy group, it may be, for example, a phenoxy, 4-methylphenoxy or 4-methoxyphenoxy group.

j' is 1 or 2.

A compound of formula (XII), wherein R⁴¹ represents a t-butyldimethylsilyl group, R⁴² represents a methyl group, the group of formula -C(=Y)NR⁴⁵R⁴⁶ represents a group of formula -CONEt₂ at the 2-position and R⁴³ represents a hydrogen atom, (disclosed in Japanese Patent Application No. Hei 4-174099) which may be be used as a starting material in Reaction Scheme 3, can be prepared by reacting Z(O)-1-t-butyldimethylsilyloxy-1-(diethylcarbamoylphenylthio)-1-propene with (3R,4R)-3-[1(R)-(t-butyldimethylsilyloxy)ethyl]-4-acetoxyazetidinone, and is obtained in a 77% yield. It may be purified by recrystallization only without carrying out column chromatography. Accordingly this compound is a valuable intermediate in the industrial preparation of 1β-methylcarbapenem derivatives.

Reaction Scheme 3 involves the preparation of a compound of formula (IVa) by reacting a compound of formula (XII) with an acid chloride of formula (XIII) to produce a N-oxalyl derivative of formula (XIV) and then treating the product with a phosphorous acid triester or phosphonous acid diester. The reaction can be accomplished by the well known method described in Tetrahedron Letters, 25, 2793 (1984) or Japanese Patent Application No. Hei 4-180779.

### Step 3a:

A compound of formula (XIV) can be prepared by reacting a compound of formula (XII) with an acid chloride of formula (XIII) in the presence of a base. We prefer to employ from 2 to 5 equivalents of the acid chloride of formula (XIII) per equivalent of the compound of formula (XII).

There is no particular limitation upon the nature of the base used, provided that it has no adverse effect upon the reaction. Examples of suitable bases include: cyclic or linear tertiary amines, such as diisopropylethylamine, diisopropylmethylamine, triethylamine, 1-ethylpiperidine, 1-methylmorpholine, 1-ethylpyrrolidine, 1,4-diazabicyclo[2,2,2]octane, 1,5-diazabicyclo[5,4,0]undec-5-ene or 1,5-diazabicyclo[4,3,0]-non-5-ene.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride, chloroform or dichloroethane; ethers, such as diethyl ether or tetrahydrofuran; hydrocarbons, especially aromatic hydrocarbons, such as benzene or toluene; and nitriles such as acetonitrile or propionitrile.

The reaction can take place over a wide range of temperatures, and the precise reaction temperature is not critical to the invention. In general, we find it convenient to carry out the reaction at a temperature of from -78°C to 50°C, more preferably from -30°C to 30°C. The time required for the reaction may also vary-widely, depending on many factors, notably the reaction temperature and the nature of the reagents and solvent employed. However, provided that the reaction is effected under the preferred conditions outlined above, a period of from 5 minutes to 3 hours will usually suffice.

After completion of the reaction, the desired compound can be recovered from the reaction mixture by conventional means after, if necessary, decomposing any excess of the acid halide with an alcohol. An example of one such technique comprises: adding water to the reaction mixture; extracting the desired compound of formula (XIV) with a water-insoluble organic solvent such as the said reaction solvent or ethyl acetate; and distilling off the solvent from the extract. The compound thus obtained can, if necessary, be purified by conventional means, such as column chromatography.

### Step 3b:

A phosphorane of formula (XVI) can be prepared by reacting a compound of formula (XIV) with a tri-valent phosphorous compound of formula (XV) in the presence or absence of a solvent. There is no particular restriction on the relative proportions of the reagents. However, we generally prefer to employ from 2 to 6 equivalents of the tri-valent phosphorous compound of formula (XV) per equivalent of the compound of formula (XIV)

The reaction may be effected in the absence or the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, especially aromatic hydrocarbons, such as benzene, toluene or xylene; halogenated hydrocarbons, especially halogenated aliphatic hydrocarbons, such as chloroform, methylene chloride or 1,2-dichloroethane; ethers, such as tetrahydrofuran or dioxane; nitriles, such as acetonitrile; esters, such as ethyl acetate; and amides, such as dimethylformamide.

The reaction can take place over a wide range of temperatures, and the precise reaction temperature is not critical to the invention. In general, we find it convenient to carry out the reaction at a temperature of from -10°C to 100°C, more preferably from 0°C to 100°C. The time required for the reaction may also vary widely, depending on many factors, notably the reaction temperature and the nature of the reagents and solvent employed. However, provided that the reaction is effected under the preferred conditions outlined above, a period of from 10 minutes to 30 hours will usually suffice.

After completion of the reaction, the desired phosphorane of formula (XVI) can be recovered from the reaction mixture by distilling off any excess of a phosphorous compound of formula (XV) and its oxygen-adduct under reduced pressure, or by precipitating the product by adding a non-polar solvent, such as hexane, to the reaction mixture.

The crude product of formula (XVI) thus obtained can be used in the subsequent step without any purification. However, the product can, if necessary, be purified by conventional means, such as column chromatography.

### Step 3c:

The compound of formula (IVa) can be prepared by heating the phosphorane of formula (XVI).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: substituted aromatic hydrocarbons, such as toluene, xylene, mesitylene or chlorobenzene.

The reaction can take place over a wide range of temperatures, and the precise reaction temperature is not critical to the invention. In general, we find it convenient to carry out the reaction at a temperature of from 100°C to 200°C, more preferably from 120°C to 170°C. The time required for the reaction may also vary widely, depending on many factors, notably the reaction temperature and the nature of the reagents and solvent employed. However, provided that the reaction is effected under the preferred conditions outlined above, a period of from 1 to 30 hours will usually suffice.

After completion of the reaction, the product of formula (IVa) can be recovered from the reaction mixture by distilling off the solvent and recrystallizing or chromatographing the residue.

### Step 3d:

This optional step for the preparation of a compound of formula (Ib) involves oxidizing a compound of formula (IVa).

An S-oxide compound of formula (IVb) can be prepared by reacting the compound of formula (IVa) with an oxidizing agent. Examples of oxidizing agents which may be used include: peroxides, such as 3-chloroperoxybenzoic acid, peracetic acid, hydrogen peroxide or t-butyl hydroperoxide, preferably 3-chloroperoxybenzoic acid or t-butyl peroxide. When t-butyl peroxide is used as an oxidizing agent in the reaction, the reaction is preferably carried out in the presence of a catalyst, such as vanadyl acetylacetonate or molybdenyl acetylacetonate, preferably vanadyl acetylacetonate. In order to remove any acidic substance arising from such oxidizing agents in the course of the reaction, the oxidation reaction can be carried out in the presence of a base, such as an alkali metal carbonate or an aqueous solution thereof.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol or ethanol; and halogenated hydrocarbons, especially halogenated aliphatic hydrocarbons, such as methylene chloride, chloroform or 1,2-dichloroethane, of which the halogenated hydrocarbons are preferred.

The reaction can take place over a wide range of temperatures, and the precise reaction temperature is not critical to the invention. In general, we find it convenient to carry out the reaction at a temperature of from -10°C to 50°C, more preferably from 0°C to 25°C. The time required for the reaction may also vary widely, depending on many factors, notably the reaction temperature and the nature of the reagents and solvent employed. However, provided that the reaction is effected under the preferred conditions outlined above, a period of from 30 minutes to 6 hours will usually suffice.

After completion of the reaction, the compound of formula (IVb) can be recovered from the reaction mixture by the following means. Any excess of the oxidizing agent is decomposed by adding a 10% aqueous solution of sodium sulphite and then the reaction mixture is diluted with a solvent such as methylene chloride, washed with water and dried, after which the solvent is distilled off.

The resulting residue may be purified by conventional means, such as recrystallization or chromatography to give the compound of formula (IVb).

It is possible to prepare either the sulphinyl compound of formula (IVb) (j' is 1) or the sulphonyl compound of formula (IVb) (j' is 2) by varying the amount of oxidizing agent. An excess of oxidizing agent will favor the production of a compound where j' is 2.

Alternatively, the compound where j' is 2 may be prepared by oxidizing the corresponding compound where j' is 1, using the same conditions as described above.

If desired, after completion of these reactions, the hydroxy-protecting group represented by R⁴¹ in any of the compounds of formula (IVa) or (IVb) may be deprotected. This can be accomplished by the well known methods described in T. W. Green and P. G. M. Wuts, "Protective Groups in Organic Synthesis. 2nd Edition", edited by John Wiley and Sons, Inc., 1991, pp. 10.

The present invention is further illustrated by the following non-limiting Examples. In the formulae in the Examples, the following abbreviations are used for certain groups:
- Et:: ethyl group
- Me:: methyl group
- PNB:: 4-nitrobenzyl group
- PNZ:: 4-nitrobenzyloxycarbonyl group
- TMS:: trimethylsilyl group
- TBS:: t-butyldimethylsilyl group

### EXAMPLE 1

### 4-Nitrobenzyl (1R,5S,6S)-2-[(3S,5S)-5-dimethylcarbamoyl-1-(4-nitrobenzyloxycarbonyl)-3-pyrrolidinylthio]-1-methyl-6-[1(R)-trimethylsilyloxyethyl]-1-carbapen-2-em-3-carboxylate

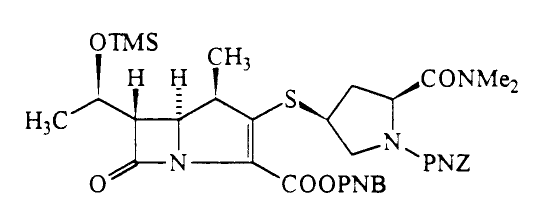

2 ml of a tetrahydrofuran solution containing 68 mg (0.19 mmol) of (2S,4S)-2-dimethylcarbamoyl-1-(4-nitrobenzyloxycarbonyl)-4-mercaptopyrrolidine were cooled in an ice bath, and then 0.19 ml (0.19 mmol) of a 1 M tetrahydrofuran solution of bromomagnesium N-isopropyl-N-cyclohexylamide were added dropwise over a period of 2 minutes. The mixture was stirred for a further 10 minutes at this temperature, and then 1 ml of a tetrahydrofuran solution containing 100 mg of 4-nitrobenzyl (1R,5S,6S)-2-(2-diethylcarbamoylphenylsulphinyl)-1-methyl-6-[1(R)-trimethylsilyloxyethyl]-1-carbapen-2-em-3-carboxylate (prepared as described in Example 21) was added. The mixture was stirred for a further 15 minutes at this temperature, after which ice water was added. The reaction solution was then diluted with ethyl acetate, washed with an aqueous solution of ammonium chloride then with a saturated aqueous solution of sodium chloride. The solvent was then removed by distillation under reduced pressure, and the resulting residue was purified by column chromatography through 10 g of silica gel, eluted with ethyl acetate, to give 94 mg (yield 78%) of the title compound as a foam-like solid.
Infrared Absorption Spectrum (liquid film), νₘₐₓ cm⁻¹:
   1773, 1711, 1655, 1522, 1345, 1142, 845.
Nuclear Magnetic Resonance Spectrum (CDCℓ₃, 270 MHz) δ ppm:
   0.13 (4.5H, singlet);
   0.14 (4.5H, singlet);
   1.25 - 1.29 (6H, multiplet);
   1.89 - 2.00 (1H, multiplet);
   2.67 - 2.80 (1H, multiplet);
   2.94 (1.5H, singlet);
   2.98 (1.5H, singlet);
   3.00 (1.5H, singlet);
   3.11 (1.5H, singlet);
   3.22 - 3.27 (1H, multiplet);
   3.30 - 3.40 (1H, multiplet);
   3.46 - 3.57 (1H, multiplet);
   3.60 - 3.76 (1H, multiplet);
   4.02 - 4.30 (3H, multiplet);
   4.72 (0.5H, triplet, J = 8 Hz);
   4.76 (0.5H, triplet, J = 8 Hz);
   5.07 (0.5H, doublet, J = 14 Hz);
   5.22 (1H, singlet);
   5.25 (1H, doublet, J = 14 Hz);
   5.30 (0.5H, doublet, J = 14 Hz);
   5.46 (1H, doublet, J = 14 Hz);
   7.44 (1H, doublet, J = 9 Hz);
   7.52 (1H, doublet, J = 9 Hz);
   7.65 (2H, doublet, J = 9 Hz);
   8.20 (1H, doublet, J = 9 Hz);
   8.21 (3H, doublet, J = 9 Hz).
Mass spectrum (m/z) : 754 [M⁺ (C₃₅H₄₃N₅O₁₁SSi)-CH₃].

### EXAMPLE 2

### 4-Nitrobenzyl (1R,5S,6S)-2-[(2S,4S)-2-dimethylcarbamoyl-1-(4-nitrobenzyloxycarbonyl)-4-pyrrolidinylthio]-1-methyl-6-[1(R)-t-butyldimethylsilyloxyethyl]-1-carbapen-2-em-3-carboxylate

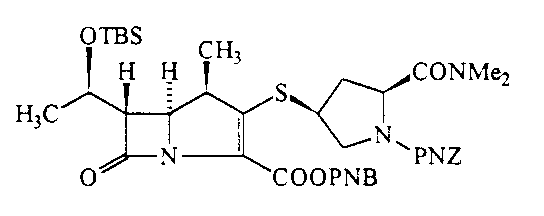

(a) Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22), the title compound was obtained as a foam-like solid in a yield of 86%.
(b) Following a procedure similar to that described in Example 1, but using 4-nitrobenzyl (1R,5S,6S)-2-[2-(2H-1,3,4,5,6,7-hexahydro-1-azepinyl)carbonylphenylsulphinyl]-1-methyl-6-[1(R)-t-butyldimethylsilyloxyethyl]-1-carbapen-2-em-3-carboxylate (prepared as described in Example 24), the title compound was obtained as a foam-like solid in a yield of 83%.
(c) 25 µℓ (0.144 mmol) of diisopropylethylamine were added to 2 ml of a tetrahydrofuran suspension containing 50 mg (0.071 mmol) of 4-nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate (prepared as described in Example 25), 30 mg (0.086 mmol) of (2S, 4S)-dimethylcarbamoyl-1-(4-nitrobenzyloxycarbonyl)-4-mercaptopyrrolidine and 76 mg (0.294 mmol) of a magnesium bromide - diethyl ether complex. The resulting suspension was then stirred for 94 hours. At the end of this time, the reaction solution was diluted with ethyl acetate, washed with water and then with a saturated aqueous solution of sodium chloride. The solvent was then removed by distillation under reduced pressure, and the resulting residue was purified by column chromatography through silica gel, using a 1 : 1 by volume mixture of methylene chloride and ethyl acetate as the eluent, to obtain 40 mg (yield 69%) of the title compound as a foam-like solid.
(d) Following a procedure similar to that described in Example 1, but using 76 mg of 4-nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate (prepared as described in Example 25) and 46 mg of (2S,4S)-dimethylcarbamoyl-1-(4-nitrobenzyloxycarbonyl)-4-mercaptopyrrolidine, the title compound was obtained in a yield of 78%.
(e) Following a procedure similar to that described in Example 1, but using 101 mg of 4-nitrobenzyl (1R,5S,6S)-6-[1(R)-t-butyldimethylsilyloxyethyl]-1-methyl-2-[2-(4-nitrobenzyloxycarbonyl)aminoethylsulphinyl)-1-carbapen-2-em-3-carboxylate (prepared as described in Preparation 5) and 61 mg of (2S,4S)-dimethylcarbamoyl-1-(4-nitrobenzyloxycarbonyl)-4-mercaptopyrrolidine, the title compound was obtained in a yield of 83%.
(f) Following a procedure similar to that described in Example 1, 100 mg of 4-nitrobenzyl (1R,5S,6S)-6-[1(R)-t-butyldimethylsilyloxyethyl] -1-methyl-2-ethylsulphinyl-1-methyl-1-carbapen-2-em-3-carboxylate (prepared as described in Preparation 6) was allowed to react with 79 mg of (2S,4S)-dimethylcarbamoyl-1-(4-nitrobenzyloxycarbonyl)-4-mercaptopyrrolidine, and the product was purified by silica gel column chromatography, using ethyl acetate as the eluent, to give a fraction containing the title compound in a yield of 87%. This fraction, which contained impurities which could not be separated by silica gel column chromatography, showed a purity of 83.5% by as determined the area ratio according to liquid chromatography.
(g) 75 µℓ of a hexane solution containing 1.6 M of butyllithium was added to 1 ml of an ice-cooled toluene solution containing 18 µℓ (0.13 mmol) of diisopropylamine, and the mixture was stirred for 10 minutes. At the end of this time, 125 µℓ (0.12 mmol) of a hexane solution containing 0.97 M of diethylaluminium chloride was added and the mixture was stirred for a further 20 minutes at this temperature. The diethylaluminium diisopropylamide solution thus obtained was added to 2 ml of an ice-cooled tetrahydrofuran solution containing 43 mg (0.12 mmol) of (2S,4S)-dimethylcarbamoyl-1-(4-nitrobenzyloxycarbonyl)-4-mercaptopyrrolidine, and stirred for 15 minutes at this temperature. At the end of this time, 1 ml of a tetrahydrofuran solution containing 68 mg (0.10 mmol) of 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) was added to the reaction mixture, and the mixture was stirred for 105 minutes. A saturated aqueous solution of ammonium chloride and then ethyl acetate were added, and the precipitate which formed was removed. The organic layer was separated, and washed with water and then with a saturated aqueous solution of sodium chloride. The solvent was then removed by distillation under reduced pressure. The resulting residue was purified by column chromatography through 10 g of silica gel, using a 1 : 3 by volume mixture of benzene and ethyl acetate as the eluent, to obtain 60 mg of a fraction containing the title compound in a yield of 74%. This fraction showed a purity of 56% as determined by the area ratio according to liquid chromatography.

The properties of the title compound obtained in (a) to (g) above are shown below.
Infrared Absorption Spectrum (liquid film), νₘₐₓ cm⁻¹:
   1775, 1713, 1657, 1522, 1345, 1142, 837.
Nuclear Magnetic Resonance Spectrum (CDCℓ₃, 270 MHz) δ ppm:
   0.07 - 0.09 (6H, multiplet);
   0.85 (4.5H, singlet);
   0.87 (4.5H, singlet);
   1.23 - 1.29 (6H, multiplet);
   1.88 - 2.00 (1H, multiplet);
   2.67 - 2.81 (1H, multiplet);
   2.94 (1.5H, singlet);
   2.98 (1.5H, singlet);
   2.99 (1.5H, singlet);
   3.11 (1.5H, singlet);
   3.23 - 3.41 (2H, multiplet);
   3.46 - 3.57 (1H, multiplet);
   3.61 - 3.78 (1H, multiplet);
   4.03 - 4.32 (3H, multiplet);
   4.73 (0.5H, triplet, J = 8 Hz);
   4.77 (0.5H, triplet, J = 8 Hz);
   5.07 (0.5H, doublet, J = 13 Hz);
   5.22 (1H, singlet);
   5.25 (1H, doublet, J = 13 Hz);
   5.30 (0.5H, doublet, J = 13 Hz);
   5.45 (1H, doublet, J = 13 Hz);
   7.45 (1H, doublet, J = 9 Hz);
   7.52 (1H, doublet, J = 9 Hz);
   7.65 (2H, doublet, J = 9 Hz);
   8.20 (1H, doublet, J = 9 Hz);
   8.21 (3H, doublet, J = 9 Hz).
Mass spectrum (m/z): 754 (M⁺(C₃₈H₄₉N₅O₁₁SSi)-C₄H₉)

### EXAMPLE 3

### 4-Nitrobenzyl(1R,5S,6S)-6-[1(R)-t-butyldimethylsilyloxyethyl]-1-methyl-2-{(2S,4S)-2-[4-{2-(4-nitrobenzyloxycarbonyloxy)ethyl}piperazin-1-yl-carbonyl]-1-[(4-nitrobenzyloxycarbonyl)pyrrolidin-4-ylthio]}-1-carbapen-2-em-3-carboxylate

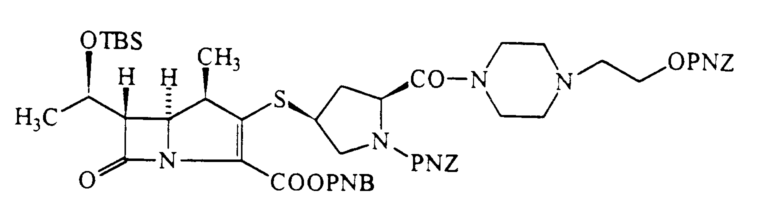

(a) Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and (2S,4S)-4-mercapto-2-{4-[2-(4-nitrobenzyloxycarbonyloxy)ethyl]piperazin-1-ylcarbonyl}-1-(4-nitrobenzyloxycarbonyl)pyrrolidine as starting materials, in relative proportions similar to those used in that Example, the title compound was obtained in a yield of 86%. Recrystallization of this product from toluene afforded it in the form of crystals, melting at 134 - 136°C.
b) Following a procedure similar to that described in of Example 2(c), but using 4-nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate (prepared as described in Example 25) and (2S,4S)-4-mercapto-2-{4-[2-(4-nitrobenzyloxycarbonyloxy)ethyl]piperazin-1-ylcarbonyl}-1-(4-nitrobenzyloxycarbonyl)pyrrolidine as starting materials, in relative proportions similar to those used in that Example, the title compound was obtained in a yield of 60%.

Infrared Absorption Spectrum (KBr), νₘₐₓ cm⁻¹:
   1773, 1750, 1709, 1653, 1522, 1345, 1260, 1146, 837.
Nuclear Magnetic Resonance Spectrum (CDCℓ₃, 270 MHz) δ ppm:
   0.06 - 0.09 (6H, multiplet);
   0.85 (4.5H, singlet);
   0.86 (4.5H, singlet);
   1.22 - 1.29 (6H, multiplet);
   1.84 - 1.99 (1H, multiplet);
   2.38 - 2.81 (7H, multiplet);
   3.23 - 3.77 (8H, multiplet);
   4.04 - 4.32 (5H, multiplet);
   4.73 (1H, doublet of triplets, J = 14 & 8 Hz);
   5.07 (0.5H, doublet, J = 14 Hz);
   5.22 (1H, singlet);
   5.26 (2H, singlet);
   5.25 (1H, doublet, J = 14 Hz);
   5.30 (0.5H, doublet, J = 14 Hz);
   5.45 (1H, doublet, J = 14 Hz);
   7.44 (1H, doublet, J = 9 Hz);
   7.52 (1H, doublet, J = 9 Hz);
   7.56 (2H, doublet, J = 9 Hz);
   7.65 (2H, doublet, J = 9 Hz);
   8.17 - 8.26 (6H, multiplet).

### EXAMPLE 4

### 4-Nitrobenzyl (1R,5S,6S)-6-[1(R)-t-butyldimethylsilyloxyethyl]-1-methyl-2-{1(S)-[N-(4-nitrobenzyloxycarbonyl)acetimidoyl]pyrrolidin-3-ylthio}-1-carbapen-2-em-3-carboxylate

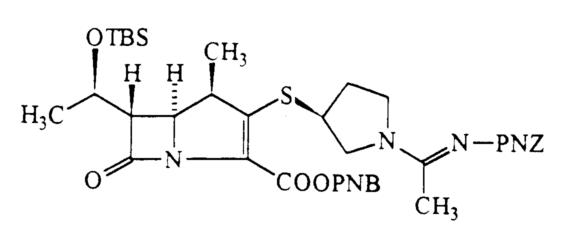

a) Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and (S)-3-mercapto-1-[N-(4-nitrobenzyloxycarbonyl)acetimidoyl]pyrrolidine as starting materials, in relative proportions similar to those used in that Example, the title compound was obtained in a yield of 79%. Recrystallization from a mixture of diisopropyl ether and ethyl acetate afforded it in the form of crystals, melting at 171 - 172.5°C.
(b) 4 ml of a tetrahydrofuran suspension containing 101 mg (0.148 mmol) of 4-nitrobenzyl (1R,5S,6S)-2-(2-diethylcarbamoylphenylsulphinyl)-1-methyl-6-[1(R)-t-butyl-dimethylsilyloxyethyl]-1-carbapen-2-em-3-carboxylate (prepared as described in Example 22), 106 mg (0.328 mmol) of (S)-3-mercapto-1-[N-(4-nitrobenzyloxycarbonyl)acetimidoyl]pyrrolidine, and 155 mg (0.600 mmol) of a magnesium bromide - diethyl ether complex was stirred for 6 hours. At the end of this time, the reaction mixture was diluted with ethyl acetate, and washed with water and then with a saturated aqueous solution of sodium chloride. The solvent was then removed by distillation under reduced pressure. The resulting residue was purified by column chromatography through 5 g of silica gel. The title compound containing a small amount of impurities was obtained by elution with a 1 : 1 by volume mixture of hexane and ethyl acetate. This impure compound was purified by additional chromatography through 5 g of silica gel followed by elution with a 1 : 3 by volume mixture of ethyl acetate and methylene chloride, to afford 74 mg (yield 64%) of the title compound.
(c) Following a procedure similar to that described in Example 2(c), but using 4-nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate (prepared as described in Example 25) and (S)-3-mercapto-1-[N-(4-nitrobenzyloxycarbonyl)acetimidoyl]pyrrolidine as starting materials, in relative proportions similar to those used in that Example, the title compound was obtained in a yield of 39%.

Infrared Absorption Spectrum (KBr), νₘₐₓ cm⁻¹:
   1769, 1694, 1675, 1567, 1520, 1345, 1242, 839.
Nuclear Magnetic Resonance Spectrum (CDCℓ₃, 270 MHz) δ ppm:
   0.08 (3H, singlet);
   0.09 (3H, singlet);
   0.87 (9H, singlet);
   1.22 - 1.31 (6H, multiplet);
   1.96 - 2.16 (1H, multiplet);
   2.29 (0.75H, singlet);
   2.32 (2.25H, singlet);
   2.35 - 2.51 (1H, multiplet);
   3.23 - 3.35 (2H, multiplet);
   3.44 - 4.05 (5H, multiplet);
   4.24 - 4.32 (2H, multiplet);
   5.22 (2H, singlet);
   5.24 (1H, doublet, J = 14 Hz);
   5.45 (1H, doublet, J = 14 Hz);
   7.56 (2H, doublet, J = 9 Hz);
   7.65 (2H, doublet, J = 9 Hz);
   8.19 (2H, doublet, J = 9 Hz);
   8.21 (2H, doublet, J = 9 Hz).
Mass spectrum (m/z): 781 (M⁺, C₃₇H₄₇N₅O₁₀SSi).

### EXAMPLE 5

### 4-Nitrobenzyl (1R,5S,6S)-6-[1(R)-t-butyldimethylsilyloxyethyl]-1-methyl-2-[2-(4-nitrobenzyloxycarbonyl)aminoethylthio]-1-carbapen-2-em-3-carboxylate

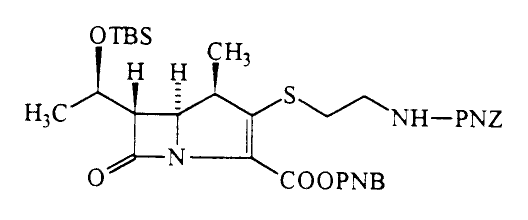

(a) Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and 2-(4-nitrobenzyloxycarbonyl)aminoethylmercaptan as starting materials, in relative proportions similar to those used in that Example, the title compound was obtained as a foam-like solid in a yield of 92%.
(b) Following a procedure similar to that described in Example 4(b), but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and 2-(4-nitrobenzyloxycarbonyl)aminoethylmercaptan as starting materials, in relative proportions similar to those used in that Example, the title compound was obtained in a yield of 83%.
(c) Following a procedure similar to that described in Example 4(b), but using the S-oxide isomer of lower polarity of the 4-nitrobenzyl (1R,5S,6S)-6-[1(R)-t-butyldimethylsilyloxyethyl]-1-methyl-2-phenylsulphinyl-1-carbapen-2-em-3-carboxylate (prepared as described in Preparation 2) and 2-(4-nitrobenzyloxycarbonyl)aminoethylmercaptan as starting materials, in relative proportions similar to those used in that Example, the title compound was obtained in a yield of 81%.
(d) Following the same procedure as that described in (c) above, but using the corresponding S-oxide of higher polarity, the title compound was obtained in a yield of 89%.
(e) Following a procedure similar to that described in Example 2(c), but using 4-nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate (prepared as described in Example 25) and 2-(4-nitrobenzyloxycarbonyl)aminoethylmercaptan as starting materials, in relative proportions similar to those used in that Example, the title compound was obtained in a yield of 87%.
(f) Following a procedure similar to that described in Example 2(c), but using 4-nitrobenzyl (1R,5S,6S)-6-[1(R)-t-butyldimethylsilyloxyethyl]-1-methyl-2-phenylsulphonyl-1-carbapen-2-em-3-carboxylate (prepared as described in Preparation 3) and 2-(4-nitrobenzyloxycarbonyl)aminoethylmercaptan as starting materials, in relative proportions similar to those used in that Example, the title compound was obtained in a yield of 86%.
(g) 0.14 ml (0.21 mmol) of a cyclohexane solution containing 1.5 M of a lithiumdiisopropylamide - tetrahydrofuran complex was added to 2 ml of an ice-cooled tetrahydrofuran suspension containing 91 mg (0.352 mmol) of a magnesium bromide - diethyl ether complex, and the resulting mixture was stirred for 10 minutes at this temperature. At the end of this time, 2 ml of a tetrahydrofuran solution containing 55 mg (0.207 mmol) of 2-(4-nitrobenzyloxycarbonyl)aminoethylmercaptan were added to the mixture, which was then stirred for a further 10 minutes in an ice bath. 102 mg (0.174 mmol) of the S-oxide isomer of lower polarity of the 4-nitrobenzyl (1R,5S,6S)-6-[1(R)-t-butyldimethylsilyloxyethyl]-1-methyl-2-phenylsulphinyl-1-carbapen-2-em-3-carboxylate (prepared as described in Preparation 2) were then added to the mixture, and the mixture was stirred for a further 15 minutes. At the end of this time, a saturated aqueous solution of ammonium chloride was added, and then the mixture was extracted with ethyl acetate. The resulting organic extract was washed first with water and then with a saturated aqueous solution of sodium chloride, after which the solvent was removed by distillation under reduced pressure. The residue was purified by column chromatography through 15 g of silica gel, using a 1 : 19 by volume mixture of ethyl acetate and hexane as the eluent, to give 104 mg (yield 84%) of the title compound as a foam-like solid.

Infrared Absorption Spectrum (liquid film), νₘₐₓ cm⁻¹:
   1771, 1723, 1607, 1522, 1347, 1140.
Nuclear Magnetic Resonance Spectrum (CDCℓ₃, 270 MHz) δ ppm:
   0.07 (3H, singlet);
   0.08 (3H, singlet);
   0.86 (9H, singlet);
   1.22 - 1.28 (6H, multiplet);
   2.93 (1H, doublet of triplets, J = 13 & 7 Hz);
   3.11 (1H, doublet of triplets, J = 13 & 7 Hz);
   3.26 (1H, doublet of doublets, J = 3 & 5 Hz);
   3.30 - 3.60 (3H, multiplet);
   4.22 - 4.31 (2H, multiplet);
   5.20 (2H, singlet);
   5.25 (1H, doublet, J = 14 Hz);
   5.35 (1H, dull triplet, J = 6 Hz);
   5.47 (1H, doublet, J = 14 Hz);
   7.50 (2H, doublet, J = 9 Hz);
   7.66 (2H, doublet, J = 9 Hz);
   8.20 (2H, doublet, J = 9 Hz);
   8.21 (2H, doublet, J = 9 Hz).
Mass spectrum (m/z): 714 (M⁺, C₃₃H₄₂N₄O₁₀SSi).

### EXAMPLE 6

### 4-Nitrobenzyl (1R,5S,6S)-6-[1(R)-t-butyldimethylsilyloxyethyl]-1-methyl-2-(4-nitrobenzylthio)-1-carbapen-2-em-3-carboxylate

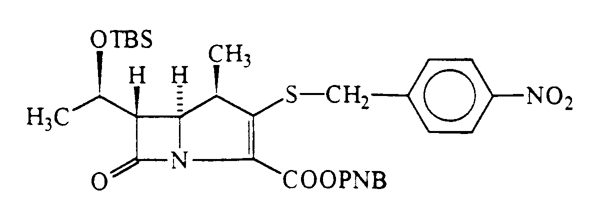

(a) Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and 4-nitrobenzylmercaptan as starting materials, in relative proportions similar to those used in that Example, the title compound was obtained in a yield of 81%. Recrystallization of this product from a mixture of ethyl acetate and diisopropyl ether the title compound in the form of crystals, melting at 150.5 to 151°C.
(b) Following a procedure similar to that described in Example 4(b), but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and 4-nitrobenzylmercaptan as starting materials, in relative proportions similar to those used in that Example, the title compound was obtained in a yield of 88%.

Infrared Absorption Spectrum (KBr), νₘₐₓ cm⁻¹:
   1773, 1711, 1605, 1522, 1345, 1140
Nuclear Magnetic Resonance Spectrum (CDCℓ₃, 270 MHz) δ ppm:
   0.06 (3H, singlet);
   0.08 (3H, singlet);
   0.85 (9H, singlet);
   1.24 (3H, doublet, J = 5 Hz);
   1.26 (3H, doublet, J = 6 Hz);
   3.25 (1H, doublet of doublets, J = 2 & 5 Hz);
   3.30 (1H, doublet of quartets, J = 10 & 6 Hz);
   4.11 - 4.23 (3H, multiplet);
   4.25 (1H, quint, J = 5 Hz);
   5.25 (1H, doublet, J = 14 Hz);
   5.46 (1H, doublet, J = 14 Hz);
   7.52 (2H, doublet, J = 8 Hz);
   7.65 (2H, doublet, J = 8 Hz);
   8.20 (2H, doublet, J = 8 Hz);
   8.21 (2H, doublet, J = 8 Hz).
Mass spectrum (m/z): 627 (M⁺, C₃₀H₃₇N₃O₈SSi).

### EXAMPLE 7

### 4-Nitrobenzyl (1R,5S,6S)-6-[1(R)-t-butyldimethylsilyloxyethyl]-1-methyl-2-phenylthio-1-carbapen-2-em-3-carboxylate

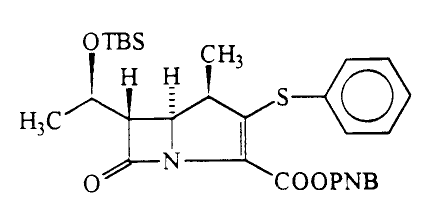

Following a procedure similar to that described in Example 4(b), but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and thiophenol as starting materials, in relative proportions similar to those used in that Example, the title compound was obtained as crystals in a yield of 72%. Recrystallization of this product from a mixture of ethyl acetate and hexane afforded crystals of the title compound melting at 149 to 150°C.
Infrared Absorption Spectrum (KBr), νₘₐₓ cm⁻¹:
   1768, 1694, 1607, 1335, 1136, 839.
Nuclear Magnetic Resonance Spectrum (CDCℓ₃, 270 MHz) δ ppm:
   0.06 (6H, singlet);
   0.84 (9H, singlet);
   0.95 (3H, doublet, J = 7 Hz);
   1.17 (3H, doublet, J = 6 Hz);
   3.06 (1H, doublet of quartets, J = 10 & 7 Hz);
   3.19 (1H, doublet of doublets, J = 2 & 5 Hz);
   4.19 (1H, doublet of doublets, J = 2 & 10 Hz);
   4.25 (1H, doublet of quartets, J = 5 & 6 Hz);
   5.33 (1H, doublet, J = 14 Hz);
   5.50 (1H, doublet, J = 14 Hz);
   7.37 - 7.43 (3H, multiplet);
   7.51 - 7.57 (2H, multiplet);
   7.69 (2H, doublet, J = 8 Hz);
   8.22 (2H, doublet, J = 8 Hz).
Mass spectrum (m/z): 568 (M⁺, C₂₉H₃₆N₂O₆SSi).

### EXAMPLE 8

### 4-Nitrobenzyl (1R,5S,6S)-2-[2-(1-perhydroazepinylcarbonyl)phenylthio]-1-methyl-6-[1(R)-t-butyldimethylsilyoxethyl]-1-carbapen-2-em-3-carboxylate

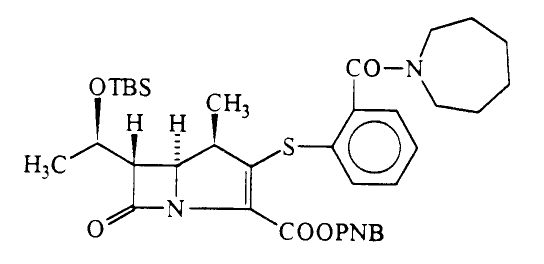

Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and 2-(1-perhydroazepinylcarbonyl)phenylmercaptan as starting materials, in relative proportions similar to those used in that Example, the title compound was obtained in a yield of 95%. This compound was found to be the same as the compound prepared as described in Example 19 and had the same physicochemical properties.

### EXAMPLE 9

### 4-Nitrobenzyl (1R,5S,6S)-6-[1(R)-t-butyldimethylsilyloxyethyl]-2-ethylthio-1-methyl-1-carbapen-2-em-3-carboxylate

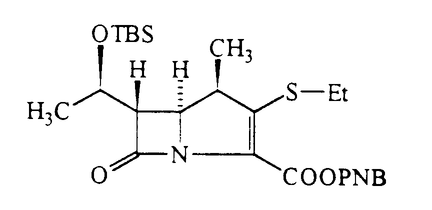

Following a procedure similar to that described in Example 1, but using 4-nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate (prepared as described in Example 22) and ethylmercaptan as starting materials, in relative proportions similar to those used in that Example, the title compound was obtained as a crystal in a yield of 83%. Recrystallization of this product from diisopropyl ether afforded the title compound in the form of crystals melting at 121 - 121.5°C.
Infrared Absorption Spectrum (KBr), νₘₐₓ cm⁻¹:
   1757, 1700, 1522, 1499, 1339, 1215, 1144, 839.
Nuclear Magnetic Resonance Spectrum (CDCℓ₃, 270 MHz) δ ppm:
   0.07 (3H, singlet);
   0.08 (3H, singlet);
   0.86 (9H, singlet);
   1.25 (3H, doublet, J = 7 Hz);
   1.26 (3H, doublet, J = 6 Hz);
   1.34 (3H, triplet, J = 7 Hz);
   2.86 (1H, doublet of quartets, J = 12 & 7 Hz);
   2.90 (1H, doublet of quartets, J = 12 & 7 Hz);
   3.23 (1H, doublet of doublets, J = 3 & 6 Hz);
   3.35 (1H, doublet of quartets, J = 9 & 7 Hz);
   4.20 (1H, doublet of doublets, J = 3 & 9 Hz);
   4.26 (1H, quartet, J = 6 Hz);
   5.25 (1H, doublet, J = 14 Hz);
   5.46 (1H, doublet, J = 14 Hz);
   7.66 (2H, doublet, J = 9 Hz);
   8.21 (2H, doublet, J = 9 Hz).
Mass spectrum (m/z): 520 (M⁺, C₂₅H₃₆N₂O₆SSi).

### EXAMPLE 10

### 4-Nitrobenzyl (1R,5S,6S)-2-[(3S,5S)-5-dimethylcarbamoyl-1-(4-nitrobenzyloxycarbonyl)-3-pyrrolidinylthio]-1-methyl-6-[1(R)-hydroxyethyl]-1-carbapen-2-em-3-carboxylate

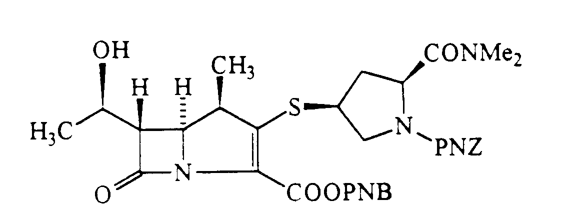

### 10(a) 4-Nitrobenzyl (1R,5S,6S)-2-(2-diethylcarbamoyl-phenylsulphinyl)-1-methyl-6-[1(R)-hydroxyethyl]-1-carbapen-2-em-3-carboxylate

Following a procedure similar to that described in Example 21, but using 467 mg (0.843 mmol) of 4-nitrobenzyl (1R,5S,6S)-2-(2-diethylcarbamoylphenylthio)-1-methyl-6-[1(R)-hydroxyethyl]-1-carbapen-2-em-3-carboxylate (prepared as described in Example 20) and 201 mg (0.932 mmol) of m-chloroperbenzoic acid (purity 80%), 464 mg of the title compound were obtained as a crude product. This product was used for the next reaction without further purification.

### 10(b) 4-Nitrobenzyl (1R,5S,6S)-2-[(3S,5S)-5-dimethylcarbamoyl-1-(4-nitrobenzyloxycarbonyl)-3-pyrrolidinylthio]-1-methyl-6-[1(R)-hydroxyethyl]-1-carbapen-2-em-3-carboxylate

3 ml of a tetrahydrofuran solution containing 347 mg (0.982 mmol) of (2S,4S)-dimethylcarbamoyl-1-(4-nitrobenzyloxycarbonyl)-4-mercaptopyrrolidine were cooled in an ice bath, and then 0.97 ml (0.97 mmol) of a tetrahydrofuran solution containing 1 M of bromomagnesium isopropylcyclohexylamide were added dropwise to the cooled solution over a period of 4 minutes, after which the mixture was stirred for 10 minutes. 2 ml of a tetrahydrofuran solution containing 464 mg of the crude 4-nitrobenzyl (1R,5S,6S)-2-(2-diethylcarbamoylphenylsulphinyl)-1-methyl-6-[1(R)-hydroxyethyl]-1-carbapen-2-em-3-carboxylate prepared as described in step (a) above was then added to the solution, and the resulting solution was stirred for a further 30 minutes. At the end of this time, a saturated aqueous solution of ammonium chloride was added, followed by water, and then the reaction mixture was extracted with ethyl acetate 3 times. The organic layers were collected, combined and washed with water and then with a saturated aqueous solution of sodium chloride. The solvent was then removed by distillation under reduced pressure, and the resulting oily residue was subjected to column chromatography through 50 g of silica gel. Elution with a 1 : 9 by volume mixture of methanol and ethyl acetate afforded 365 mg of the title compound as a foam-like solid (a yield of 62% in terms of the compound of Example 20).
Infrared Absorption Spectrum (liquid film), νₘₐₓ cm⁻¹:
   3500-3300, 1771, 1707, 1649, 1522, 1345, 1140, 855.
Nuclear Magnetic Resonance Spectrum (CDCℓ₃, 270 MHz) δ ppm:
   1.25 (1.5H, doublet, J = 7 Hz);
   1.28 (1.5H, doublet, J = 7 Hz);
   1.34 (3H, doublet, J = 6 Hz);
   1.86 - 1.99 (1H, multiplet);
   2.40 - 3.20 (1H, broad);
   2.67 - 2.81 (1H, multiplet);
   2.94 (1.5H, singlet);
   2.99 (3H, singlet);
   3.11 (1.5H, singlet);
   3.23 - 3.29 (1H, multiplet);
   3.34 - 3.76 (3H, multiplet);
   4.04 - 4.29 (3H, multiplet);
   4.73 (0.5H, triplet, J = 8 Hz);
   4.78 (0.5H, triplet, J = 8 Hz);
   5.07 (0.5H, doublet, J = 14 Hz);
   5.21 (1H, singlet);
   5.22 (0.5H, doublet, J = 14 Hz);
   5.23 (1H, doublet, J = 14 Hz);
   5.48 (1H, doublet, J = 14 Hz);
   7.43 (1H, doublet, J = 8 Hz);
   7.51 (1H, doublet, J = 8 Hz);
   7.64 (2H, doublet, J = 8 Hz);
   8.20 (1H, doublet, J = 9 Hz);
   8.17 - 8.22 (3H, multiplet).

### EXAMPLE 11

### 4-Nitrobenzyl (5S,6S)-6-[1(R)-t-butyldimethylsilyloxyethyl]-2-[(3S,5S)-5-dimethylcarbamoyl-1-(4-nitrobenzyloxycarbonyl)-3-pyrrolidinylthio]-1-carbapen-2-em-3-carboxylate

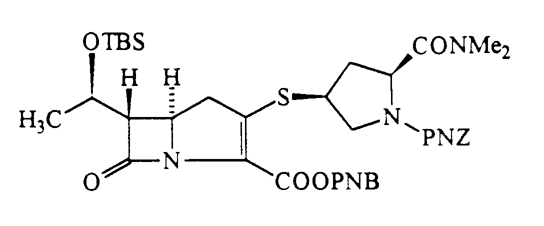

Following a procedure similar to that described in Example 1, but using (2S,4S)-2-dimethylcarbamoyl-1-(4-nitrobenzyloxycarbonyl)-4-mercaptopyrrolidine and the crude 4-nitrobenzyl (5S,6S)-6-[1(R)-t-butyldimethylsilyloxyethyl]-2-phenylsulphinyl-1-carbapen-2-em-3-carboxylate (prepared as described in Preparation 4, but used before purification by column chromatography) as starting materials, in relative proportions similar to those used in that Example, the title compound was obtained as a foam-like solid in a yield of 89%.
Infrared Absorption Spectrum (liquid film), νₘₐₓ cm⁻¹:
   1779, 1709, 1657, 1522, 1345, 1136, 837.
Nuclear Magnetic Resonance Spectrum (CDCℓ₃, 270 MHz) δ ppm:
   0.08 - 0.09 (6H, multiplet);
   0.87 (4.5H, singlet);
   0.88 (4.5H, singlet);
   1.23 - 1.27 (3H, multiplet);
   1.90 - 2.03 (1H, multiplet);
   2.65 - 2.79 (1H, multiplet);
   2.93 (1.5H, singlet);
   2.97 (1.5H, singlet);
   2.99 (1.5H, singlet);
   3.10 (1.5H, singlet);
   3.03 - 3.30 (3H, multiplet);
   3.49 - 3.71 (2H, multiplet);
   4.07 - 4.30 (3H, multiplet);
   4.72 (1H, doublet of triplets, J = 14 & 8 Hz);
   5.07 (0.5H, doublet, J = 14 Hz);
   5.23 (1H, singlet);
   5.25 (1H, doublet, J = 14 Hz);
   5.30 (0.5H, doublet, J = 14 Hz);
   5.44 (1H, doublet, J = 14 Hz);
   7.44 (1H, doublet, J = 9 Hz);
   7.52 (1H, doublet, J = 9 Hz);
   7.64 (2H, doublet, J = 9 Hz);
   8.19 - 8.24 (4H, multiplet).

### EXAMPLE 12

### 4-Nitrobenzyl (5R,6S)-6-[1(R)-butyldimethylsilyloxyethyl]-2-[(3S,5S)-5-dimethylcarbamoyl-1-(4-nitrobenzyloxycarbonyl)-3-pyrrolidinylthio]-penem-3-carboxylate

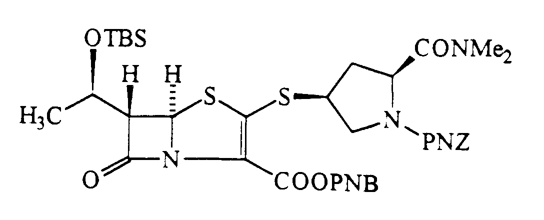

Following a procedure similar to that described in Example 1, but using (2S,4S)-2-dimethylcarbamoyl-1-(4-nitrobenzyloxycarbonyl)-4-mercaptopyrrolidine and 4-nitrobenzyl (5R,6S)-6-[1(R)-t-butyldimethylsilyloxyethyl]-2-ethylsulphinylpenem-3 -carboxylate (prepared as described in Preparation 7) as starting materials, in relative proportions similar to those used in that Example, the title compound was obtained as a foam-like solid in a yield of 81%.
Infrared Absorption Spectrum (liquid film), νₘₐₓ cm⁻¹:
   1788, 1711, 1657, 1522, 1345, 1117, 839.
Nuclear Magnetic Resonance Spectrum (CDCℓ₃, 270 MHz) δ ppm:
   0.04 (3H, singlet);
   0.07 (3H, singlet);
   0.83 (9H, singlet);
   1.25 (3H, doublet, J = 6 Hz);
   1.91 - 2.05 (1H, multiplet);
   2.67 - 2.85 (1H, multiplet);
   2.93 (1.5H, singlet);
   2.97 (1.5H, singlet);
   2.99 (1.5H, singlet);
   3.09 (1.5H, singlet);
   3.46 - 3.60 (1H, multiplet);
   3.69 - 3.80 (2H, multiplet);
   4.22 - 4.40 (2H, multiplet);
   4.68 - 4.79 (1H, multiplet);
   5.07 (0.5H, doublet, J = 14 Hz);
   5.21 (1H, doublet, J = 14 Hz);
   5.23 (1H, singlet);
   5.31 (0.5H, doublet, J = 14 Hz);
   5.41 (1H, doublet, J = 14 Hz);
   5.69 (1H, singlet);
   7.44 (1H, doublet, J = 9 Hz);
   7.52 (1H, doublet, J = 9 Hz);
   7.61 (2H, doublet, J = 9 Hz);
   8.19 - 8.24 (4H, multiplet).

### EXAMPLE 13

### 4-Nitrobenzyl (5R,6S)-6-[1(R)-t-butyldimethylsilyloxyethyl]-2-[2-(4-nitrobenzyloxycarbonyl)aminoethylthio]penem-3-carboxylate

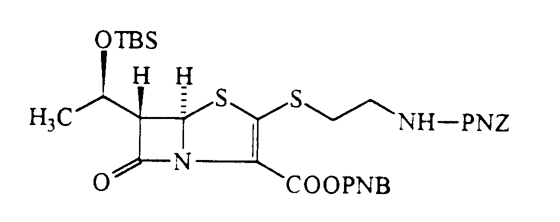

(a) Following a procedure similar to that described in Example 1, but using 4-nitrobenzyl (5R,6S)-6-[1(R)-t-butyldimethylsilyloxyethyl]-2-ethylsulphinylpenem-3-carboxylate (prepared as described in Preparation 7) and 2-(4-nitrobenzyloxycarbonyl)aminoethylmercaptan as starting materials, in relative proportions similar to those used in that Example, the title compound was obtained as a foam-like solid in a yield of 76%.
(b) Following a procedure similar to that described in Example 4(b), but using 4-nitrobenzyl (5R,6S)-6-[1(R)-t-butyldimethylsilyloxyethyl]-2-ethylsulphinyl-penem-3-carboxylate (prepared as described in Preparation 7) and 2-(4-nitrobenzyloxycarbonyl)aminoethylmercaptan as starting materials, in relative proportions similar to those used in that Example, the title compound was obtained in a yield of 86%.

Infrared Absorption Spectrum (liquid film), νₘₐₓ cm⁻¹:
   1788, 1725, 1607, 1522, 1347, 1119.
Nuclear Magnetic Resonance Spectrum (CDCℓ₃, 270 MHz), δ ppm:
   0.04 (3H, singlet);
   0.07 (3H, singlet);
   0.83 (9H, singlet);
   1.24 (3H, doublet, J = 6 Hz);
   3.04 - 3.24 (2H, multiplet);
   3.53 (2H, quartet, J = 6 Hz);
   3.73 (1H, doublet of doublets, J = 1 & 4 Hz);
   4.26 (1H, doublet of quartets, J = 4 & 6 Hz);
   5.18 - 5.27 (1H, multiplet);
   5.20 (2H, singlet);
   5.21 (1H, doublet, J = 14 Hz);
   5.42 (1H, doublet, J = 14 Hz);
   5.67 (1H, doublet, J = 1 Hz);
   7.50 (2H, doublet, J = 9 Hz);
   7.62 (2H, doublet, J = 9 Hz);
   8.20 (2H, doublet, J = 9 Hz);
   8.22 (2H, doublet, J = 9 Hz).
Mass spectrum (m/z): 661 (M⁺ (C₃₁H₃₈N₄O₁₀S₂Si)-t-Bu).

### EXAMPLE 14

### 4-Nitrobenzyl (5S,6S)-6-[1(R)-trimethylsilyloxyethyl]-2-{(S)-1-[N-(4-nitrobenzyloxycarbonyl)-acetimidoyl]pyrrolidin-3 -ylthioy}-1-carbapen-2-em-3-carboxylate

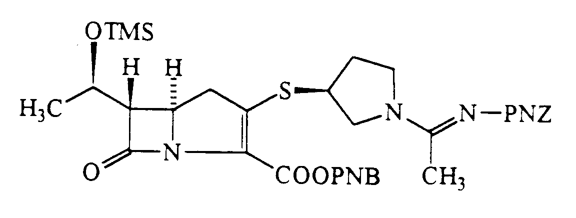

(i) 4-Nitrobenzyl (5S,6S)-6-[1(R)-hydroxyethyl]-2-[2-(4-nitrobenzyloxycarbonyl)aminoethylthio]-1-carbapen-2-em-3-carboxylate was prepared in two steps from thienamycin following the procedure described by S. M. Schmitt et al. [J. Org. Chem. (1980), 45, 1142]. 140 mg (0.239 mmol) of this compound were then dissolved in 2 ml of tetrahydrofuran, and 0.233 ml (1.65 mmol) of triethylamine and 0.147 ml (1.19 mmol) of trimethylsilyl chloride were added to the solution; the resulting mixture was then stirred for 9 hours. Because the starting material did not disappear, a further 0.167 ml (1.20 mmol) of triethylamine and 0.148 ml (1.20 mmol) of trimethylsilyl chloride were added, and the mixture was stirred for a further 1 hour. At the end of this time, the reaction solution was diluted with ethyl acetate, and washed with ice water and then with a saturated aqueous solution of sodium chloride. The solvent was then removed by distillation under reduced pressure. The resulting residue was purified by column chromatography through 10 g of silica gel. Elution with a 15 : 85 by volume mixture of ethyl acetate and methylene chloride yielded 130 mg (83%) of a trimethylsilyl compound in the form of crystals. Recrystallization of these from a mixture of ethyl acetate and hexane afforded crystals of the trimethylsilyl compound melting at 165 to 168°C.
   Infrared Absorption Spectrum (KBr), νₘₐₓ cm⁻¹:
      1773, 1738, 1694, 1607, 1518, 1341, 1140.
   Nuclear Magnetic Resonance Spectrum (CDCℓ₃, 270 MHz) δ ppm:
      0.14 (9H, singlet);
      1.28 (3H, doublet, J = 6 Hz);
      2.91 - 3.18 (3H, multiplet);
      3.16 (1H, doublet of doublets, J = 2 & 6 Hz);
      3.27 - 3.49 (3H, multiplet);
      4.11 - 4.26 (2H, multiplet);
      5.20 (2H, singlet);
      5.25 (1H, doublet, J = 14 Hz);
      5.15 - 5.30 (1H, multiplet);
      5.48 (1H, doublet, J = 14 Hz);
      7.50 (2H, doublet, J = 9 Hz);
      7.66 (2H, doublet, J = 9 Hz);
      8.21 (2H, doublet, J = 9 Hz);
      8.23 (2H, doublet, J = 9 Hz).
(ii) Following a procedure similar to that described in Example 21, but using 130 mg of the trimethylsilyl compound obtained as described in step (i) above, 137 mg of a crude S-oxide were obtained.
   Infrared Absorption Spectrum (liquid film), νₘₐₓ cm⁻¹:
      1786, 1721, 1607, 1522, 1349, 1252, 1053.
   Nuclear Magnetic Resonance Spectrum (CDCℓ₃, 270 MHz) δ ppm:
      0.13 (9H, singlet);
      1.25 (1.5H, doublet, J = 6 Hz);
      1.26 (1.5H, doublet, J = 6 Hz);
      3.03 - 3.38 (4.5H, multiplet);
      3.52 - 3.85 (2.5H, multiplet);
      4.20 - 4.45 (2H, multiplet);
      5.20 (1H, singlet);
      5.22 (1H, singlet);
      5.26 (0.5H, doublet, J = 14 Hz);
      5.30 (0.5H, doublet, J = 14 Hz);
      5.43 (0.5H, doublet, J = 14 Hz);
      5.47 (0.5H, doublet, J = 14 Hz);
      5.62 - 5.70 (1H, multiplet);
      7.51 (2H, doublet, J = 9 Hz);
      7.62 (1H, doublet, J = 9 Hz);
      7.65 (1H, doublet, J = 9 Hz);
      8.22 (2H, doublet, J = 9 Hz);
      8.23 (2H, doublet, J = 9 Hz).
(iii) Following a procedure similar to that described in Example 1, but using 137 mg of the crude S-oxide obtained as described in step (ii) above and 84 mg of (S)-3-mercapto-1-[N-(4-nitrobenzyloxycarbonyl)acetimidoyl]pyrrolidine, 126 mg (a yield of 87% calculated in terms of the trimethylsilyl derivative) of the title compound was obtained as crystals. Recrystallization of these from a mixture of ethyl acetate and hexane afforded crystals of the title compound melting at 95 to 96°C. The physicochemical properties of this compound accorded with those of the compound reported by A. Yoshida et al. [Tetrahedron Letters 25, 2793 (1984)].
   Infrared Absorption Spectrum (KBr), νₘₐₓ cm⁻¹:
      1783, 1700, 1676, 1557, 1520, 1349, 1239, 845.
   Nuclear Magnetic Resonance Spectrum (CDCℓ₃), δ ppm:
      0.14 (2.25H, singlet);
      0.15 (6.75H, singlet);
      1.29 (3H, doublet, J = 6 Hz);
      1.96 - 2.17 (1H, multiplet);
      2.29 (0.75H, singlet);
      2.32 (2.25H, singlet);
      2.30 - 2.51 (1H, multiplet);
      3.06 - 3.31 (3H, multiplet);
      3.45 - 4.07 (5H, multiplet);
      4.15 - 4.27 (2H, multiplet);
      5.23 (2H, singlet);
      5.24 (1H, doublet, J = 14 Hz);
      5.48 (1H, doublet, J = 14 Hz);
      7.56 (2H, doublet, J = 9 Hz);
      7.65 (2H, doublet, J = 9 Hz);
      8.20 (2H, doublet, J = 9 Hz);
      8.22 (2H, doublet, J = 9 Hz).

### EXAMPLE 15

### 4-Nitrobenzyl (1R,5S,6S)-2-{(3S,5S)-5-[3(S)-(4-nitrobenzyloxycarbonyl)aminopyrrolidin-1-ylcarbonyl]-1-methylpyrrolidin-3-ylthio}-6-[1(R)-hydroxyethyl]-1-methyl-1-carbapen-2-em-3-carboxylate

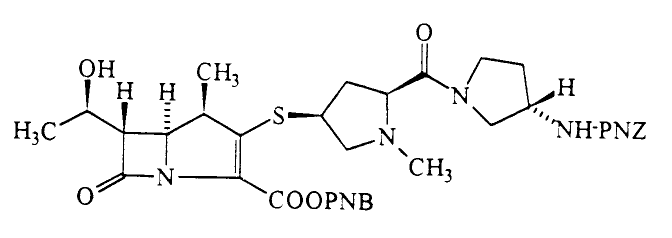

Following a procedure similar to that described in Example 10(b), but using 4-nitrobenzyl (1R,5S,6S)-2-(2-diethylcarbamoylphenylsulphinyl)-1-methyl-6-[1(R)-hydroxyethyl]-1-carbapen-2-em-3-carboxylate [prepared as described in Example 10(a)] and (2S,4S)-4-mercapto-2-[3(S)-(4-nitrobenzyloxycarbonyl)aminopyrrolidin-1-ylcarbonyl]-1-methylpyrrolidine as starting materials, in relative proportions similar to those used in that Example, the title compound was obtained as a powder in a yield of 60%.

The physicochemical properties of this compound were in agreement with those of the intermediate 45(a) described in Example 45 of European Patent Publication No. 518 558A.

### EXAMPLE 16

### 4-Nitrobenzyl (1R,5S,6S)-2-[(RS)-5-oxo-3-pyrrolidinylthio]-6-[1(R)-hydroxyethyl]-1-methyl-1-carbapen-2-em-3-carboxylate

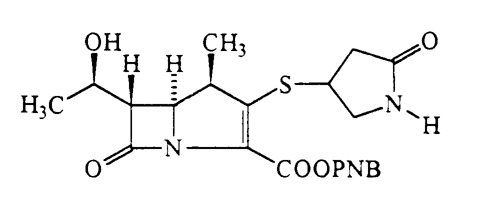

Following a procedure similar to that described in Example 10(b), but using 4-nitrobenzyl (1R,5S,6S)-2-(2-diethylcarbamoylphenylsulphinyl)-1-methyl-6-[1(R)-hydroxyethyl]-1-carbapen-2-em-3-carboxylate [prepared as described in Example 10(a)] and 4-mercapto-2-oxopyrrolidine as starting materials, in relative proportions similar to those used in that Example, the title compound was obtained as a powder in a yield of 59%.

The physicochemical properties of this compound were in agreement with those of the intermediate described in Example 11 of Japanese Patent Publication No. Hei 2-49783.

### EXAMPLE 17

### 4-Nitrobenzyl (1R,5S,6S)-2-(2-diethylcarbamoylphenylthio)-1-methyl-6-[1(R)-trimethylsilyloxyethyl]-1-carbapen-2-em-3-carboxylate

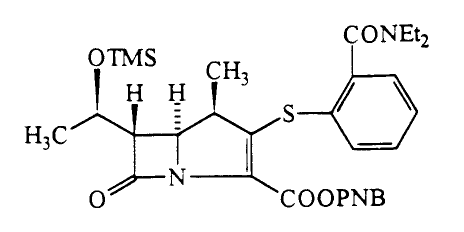

8 ml of a methylene chloride solution containing 492 mg (1.09 mmol) of S-2-diethylcarbamoylphenyl 2(R)-[(2S,3S)-3-[1(R)-(trimethylsilyloxy)ethyl]-4-oxo-2-azetidinyl]thiopropionate (prepared as described in Preparation 1) and 0.46 ml (3.30 mmol) of triethylamine were cooled in a salt-ice bath, and then 797 mg (3.27 mmol) of p-nitrobenzyloxyoxalyl chloride were added to the solution. The resulting mixture was then allowed to react for 15 minutes at this temperature. At the end of this time, 0.25 ml (3.27 mmol) of isopropanol was added, and the reaction mixture was stirred for 10 minutes at this temperature to decompose any excess of the acid chloride. The reaction solution was then diluted with ethyl acetate, and washed twice with water and then twice with a saturated aqueous solution of sodium chloride. The solvent was then removed by distillation under reduced pressure. The resulting residue was suspended in toluene, and insoluble matter was filtered off. Toluene was then removed by distillation under reduced pressure, 784 mg (4.40 mmol) of dipropyl ethylphosphonite were added to the residue, and the mixture was stirred for 140 minutes at room temperature. At the end of this time, excess reagent was removed by distillation under reduced pressure, and the residue was dissolved in 65 ml of xylene and heated under reflux for 3 hours. The solvent was then removed by distillation under reduced pressure, and the resulting residue was purified by column chromatography through 50 g of silica gel. The title compound containing a small amount of impurities was obtained by elution with a 1 : 2 by volume mixture of ethyl acetate and hexane. Further purification by column chromatography under the same conditions yielded 535 mg (78%) of the title compound. Recrystallization of this product from diisopropyl ether afforded the title compound in the form of colourless crystals melting at 202 - 203°C.
Infrared Absorption Spectrum (KBr), νₘₐₓ cm⁻¹:
   1761, 1695, 1630, 1342, 1207.
Nuclear Magnetic Resonance Spectrum (CDCℓ₃, 270 MHz) δ ppm:
   0.10 (9H, singlet);
   1.01 (3H, triplet, J = 7 Hz);
   0.95 - 1.20 (6H, multiplet);
   1.20 (3H, doublet, J = 6 Hz);
   3.06 (2H, quartet, J = 7 Hz);
   3.05 - 3.28 (3H, multiplet);
   3.45 - 3.80 (1H, broad singlet);
   4.18 - 4.25 (2H, multiplet);
   5.27 (1H, doublet, J = 14 Hz);
   5.47 (1H, doublet, J = 14 Hz);
   7.32 - 7.65 (4H, multiplet);
   7.68 (2H, doublet, J = 9 Hz);
   8.21 (2H, doublet, J = 9 Hz).
Mass spectrum (m/z): 625 (M⁺, C₃₁H₃₉N₃O₇SSi).

### EXAMPLE 18

### 4-Nitrobenzyl (1R,5S,6S)-2-(2-diethylcarbamoylphenylthio)-1-methyl-6-[1(R)-t-butyldimethylsilyloxyethyl]-1-carbapen-2-em-3-carboxylate

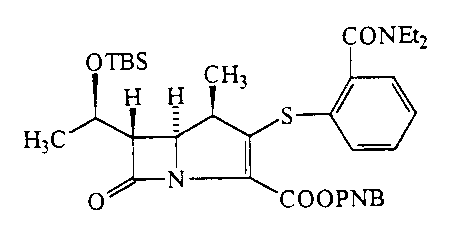

Following a procedure similar to that described in Example 17, but using S-2-diethylcarbamoylphenyl 2(R)-[(2S,3S)-3-[1(R)-t-butyldimethylsilyloxyethyl]-4-oxo-2-azetidinyl]thiopropionate (prepared as described in Preparation 9 of this specification and also in Example 1 of Japanese Patent Application No. Hei 4-174099) as a starting material, in a relative amount similar to that used in that Example, the title compound was obtained in a yield of 80%. Recrystallization of this product from diisopropyl ether afforded the title compound in the form of crystals melting at 166 - 166.5°C.
Infrared Absorption Spectrum (Nujol - trade mark), νₘₐₓ cm⁻¹:
   1762, 1693, 1633, 1340, 1210.
Nuclear Magnetic Resonance Spectrum (CDCℓ₃, 270 MHz) δ ppm:
   0.06 (6H, singlet);
   0.84 (9H, singlet);
   1.01 (3H, triplet, J = 7 Hz);
   0.93 - 1.20 (6H, multiplet);
   1.16 (3H, doublet, J = 6 Hz);
   3.00 - 3.30 (3H, multiplet);
   3.07 (2H, quartet, J = 7 Hz);
   3.40 - 3.90 (1H, multiplet);
   4.21 - 4.33 (2H, multiplet);
   5.28 (1H, doublet, J = 14 Hz);
   5.44 (1H, doublet, J = 14 Hz);
   7.27 - 7.65 (4H, multiplet);
   7.67 (2H, doublet, J = 9 Hz);
   8.21 (2H, doublet, J = 9 Hz).
Mass spectrum (m/z): 667 (M⁺, C₃₄H₄₅N₃O₇SSi).

### EXAMPLE 19

### 4-Nitrobenzyl (1R,5S,6S)-2-[2-(1-perhydroazepinylcarbonyl)phenylthio]-1-methyl-6-[1(R)-t-butyldimethylsilyloxyethyl]-1-carbapen-2-em-3-carboxylate

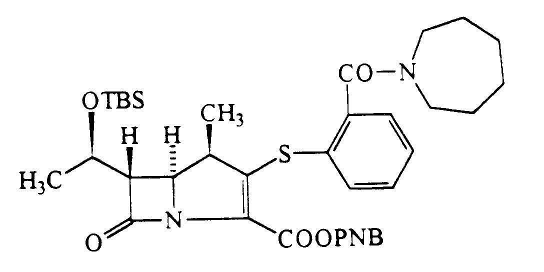

Following a procedure similar to that described in Example 17, but using S-2-(1-perhydroazepinylcarbonyl)phenyl 2(R)-{(2S,3S)-3-[1(R)-t-butyldimethylsilyloxyethyl]-4-oxo-2-azetidinyl}thiopropionate (prepared as described in Example 9 of Japanese Patent Application No. Hei 4-174099) as a starting material, in a relative amount similar to that used in that Example, the title compound was obtained in a yield of 70%. Recrystallization of this product from diisopropyl ether gave the title compound in the form of crystals melting at 210 - 211°C.
Infrared Absorption Spectrum (KBr), νₘₐₓ cm⁻¹:
   1757, 1700, 1632, 1341, 1212, 1138.
Nuclear Magnetic Resonance Spectrum (CDCℓ₃, 270 MHz) δ ppm:
   0.06 (6H, singlet);
   0.85 (9H, singlet);
   0.95 - 1.08 (3H, multiplet);
   1.16 (3H, doublet, J = 6 Hz);
   1.40 - 1.85 (8H, multiplet);
   3.04 - 3.34 (5H, multiplet);
   3.45 - 3.65 (1H, broad singlet);
   4.20 - 4.36 (2H, multiplet);
   5.29 (1H, doublet, J = 14 Hz);
   5.46 (1H, doublet, J = 14 Hz);
   7.30 - 7.65 (4H, multiplet);
   7.68 (2H, doublet, J = 9 Hz);
   8.22 (2H, doublet, J = 9 Hz).
Mass spectrum (m/z): 693 (M⁺, C₃₆H₄₇N₃O₇SSi).

### EXAMPLE 20

### 4-Nitrobenzyl (1R,5S,6S)-2-(2-diethylcarbamoylphenylthio)-1-methyl-6-[1(R)-hydroxyethyl]-1-carbapen-2-em-3-carboxylate

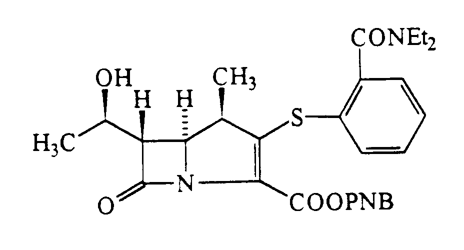

41 mg (0.195 mmol) of citric acid hydrate were added to 30 ml of a solution of 1.072 g (1.71 mmol) of 4-nitrobenzyl (1R,5S,6S)-2-(2-diethylcarbamoylphenylthio)-1-methyl-6-[1(R)-trimethylsilyloxyethyl]-1-carbapen-2-em-3-carboxylate (prepared as described in Example 17) in a 2 : 1 by volume mixture of methanol and methylene chloride, and the mixture was stirred for 20 minutes at room temperature. At the end of this time, the reaction solution was condensed by distillation under reduced pressure. The resulting residue was purified by column chromatography through 25 g of silica gel. Elution with ethyl acetate afforded 970 mg (quantitative yield) of the title compound as a colourless foam-like solid.
Infrared Absorption Spectrum (neat), νₘₐₓ cm⁻¹:
   3600-3200 (broad), 1771, 1707, 1612, 1345, 1207.
Nuclear Magnetic Resonance Spectrum (CDCℓ₃, 270 MHz) δ ppm:
   1.00 (3H, triplet, J = 7 Hz);
   0.94 - 1.15 (6H, multiplet);
   1.30 (3H, doublet, J = 6 Hz);
   3.05 (2H, quartet, J = 7 Hz);
   3.10 - 3.28 (3H, multiplet);
   3.45 - 3.80 (1H, broad singlet);
   4.20 (1H, quintet, J = 7 Hz);
   4.25 (1H, doublet of doublets, J = 2 & 9 Hz);
   5.27 (1H, doublet, J = 14 Hz);
   5.48 (1H, doublet, J = 14 Hz);
   7.31 - 7.65 (4H, multiplet);
   7.67 (2H, doublet, J = 9 Hz);
   8.22 (2H, doublet, J = 9 Hz).
Mass spectrum (m/z): 553 (M⁺, C₂₈H₃₁N₃O₇S).

### EXAMPLE 21

### 4-Nitrobenzyl (1R,5S,6S)-2-(2-diethylcarbamoylphenylsulphinyl)-1-methyl-6-[1(R)-trimethylsilyloxyethyl]-1-carbapen-2-em-3-carboxylate

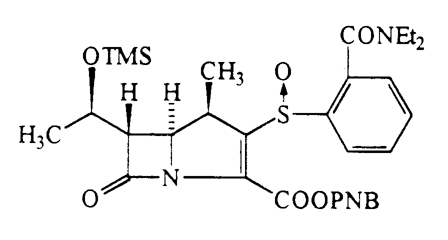

122 mg (1.45 mmol) of sodium hydrogencarbonate were added to 6 ml of a methylene chloride solution containing 304 mg (0.486 mmol) of 4-nitrobenzyl (1R,5S,6S)-2-(2-diethylcarbamoylphenylthio)-1-methyl-6-[1(R)-trimethylsilyloxyethyl]-1-carbapen-2-em-3-carboxylate (prepared as described in Example 17). The mixture was then placed in an ice bath while 105 mg (0.487 mmol) of m-chloroperbenzoic acid (80% purity) were added, with stirring. The reaction mixture was stirred for 1 hour at the same temperature, after which it was diluted with methylene chloride and washed with an ice-cooled dilute aqueous solution of sodium hydrogencarbonate. The solvent was then removed by distillation under reduced pressure. The resulting residue was purified by column chromatography through 25 g of silica gel. Elution with a 2 : 1 by volume mixture of ethyl acetate and hexane afforded 260 mg (yield 83%) of the title compound as a foam-like solid.
Infrared Absorption Spectrum (liquid film), νₘₐₓ cm⁻¹:
   1788, 1734, 1630, 1522, 1061.
Nuclear Magnetic Resonance Spectrum (CDCℓ₃, 270 MHz) δ ppm:
   0.08 (5.4H, singlet);
   0.10 (3.6H, singlet);
   1.04 - 1.27 (12H, multiplet);
   2.98 - 3.53 (5.6H, multiplet);
   3.67 (0.4H, doublet of quartets, J = 11 & 7 Hz);
   4.16 - 4.23 (1H, multiplet);
   4.26 (0.6H, doublet of doublets, J = 10 & 3 Hz);
   4.34 (0.4H, doublet of doublets, J = 10 & 3 Hz);
   5.35 (0.4H, doublet, J = 14 Hz);
   5.38 (0.6H, doublet, J = 14 Hz);
   5.46 (0.4H, doublet, J = 14 Hz);
   5.50 (0.6H, doublet, J = 14 Hz);
   7.27 - 7.36 (1H, multiplet);
   7.50 - 7.73 (4H, multiplet);
   7.84 - 7.90 (0.4H, multiplet);
   8.15 - 8.26 (2.6H, multiplet).
Mass spectrum (m/z): 641(M⁺, C₃₁H₃₉N₃O₈SSi).

### EXAMPLE 22

### 4-Nitrobenzyl (1R,5S,6S)-2-(2-diethylcarbamoylphenylsulphinyl)-1-methyl-6-[1(R)-t-butyldimethylsilyloxyethyl]-1-carbapen-2-em-3-carboxylate

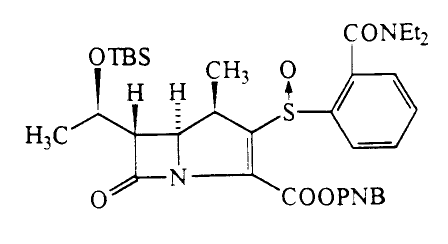

Following a procedure similar to that described in Example 21, but using 4-nitrobenzyl (1R,5S,6S)-2-(2-diethylcarbamoylphenylthio)-1-methyl-6-[1(R)-t-butyldimethylsilyloxyethyl]-1-carbapen-2-em-3-carboxylate (prepared as described in Example 18) as a starting material, in a relative amount similar to that used in that Preparation, the title compound was obtained as a foam-like solid in a yield of 87%.
Infrared Absorption Spectrum (Nujol), νₘₐₓ cm⁻¹:
   1788, 1735, 1630, 1523, 1058.
Nuclear Magnetic Resonance Spectrum (CDCℓ₃, 270 MHz) δ ppm:
   0.03 - 0.06 (6H, multiplet);
   0.83 (4.5H, singlet);
   0.84 (4.5H, singlet);
   1.05 - 1.27 (12H, multiplet);
   2.98-3.56 (5.5H, multiplet);
   3.59 (0.5H, doublet of quartets, J = 10 & 7 Hz);
   4.20 - 4.38 (1H, multiplet);
   4.30 (0.5H, doublet of doublets, J = 11 & 3 Hz);
   4.39 (0.5H, doublet of doublets, J = 11 & 3 Hz);
   5.34 (0.5H, doublet, J = 14 Hz);
   5.38 (0.5H, doublet, J = 14 Hz);
   5.45 (0.5H, doublet, J = 14 Hz);
   5.49 (0.5H, doublet, J = 14 Hz);
   7.29 - 7.36 (1H, multiplet);
   7.50 - 7.71 (4H, multiplet);
   7.85 - 7.89 (0.5H, multiplet);
   8.15 - 8.25 (2.5H, multiplet).
Mass spectrum (m/z): 683 (M⁺, C₃₄H₄₅N₃O₈SSi).

### EXAMPLE 23

### 4-Nitrobenzyl (1R,5S,6S)-2-(2-diethylcarbamoylphenylsulphinyl)-1-methyl-6-[1(R)-hydroxyethyl]-1-carbapen-2-em-3-carboxylate

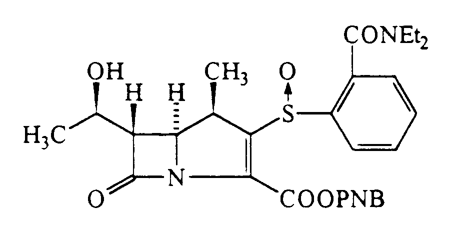

10 ml of a methylene cloride solution containing 554 mg (1.00 mmol) of 4-nitrobenzyl (1R,5S,6S)-2-(2-diethylcarbamoylphenylthio)-1-methyl-6-[1(R)-hydroxyethyl]-1-carbapen-2-em-3-carboxylate (prepared as described in Example 20) were cooled in an ice bath, and then 1.5 ml (1.5 mmol) of a 1 M aqueous solution of sodium hydrogencarbonate and then 215 mg (1.00 mmol) of m-chloroperbenzoic acid (80% purity) were added, and the reaction solution was stirred for 90 minutes at the same temperature. At the end of this time, an aqueous solution of sodium sulphite was added to the reaction solution, and the mixture was stirred for 10 minutes to complete the reaction. The reaction solution was then diluted with methylene chloride, ice-cooled, washed with an aqueous solution of sodium hydrogencarbonate and condensed by evaporation under reduced pressure. The resulting residue was purified by column chromatography through 50 g of silica gel. Elution with ethyl acetate afforded 145 mg (yield 25%) of an isomer of the title compound of lower polarity (the isomer is a result of the configuration of the S-oxide), followed by 96 mg (yield 17%) of a higher polarity isomer of the title compound, both as foam-like solids.

### Lower polarity S-oxide:

Infrared Absorption Spectrum (liquid film), νₘₐₓ cm⁻¹:
   3500-3300, 1786, 1732, 1624, 1522, 1200, 1061.
Nuclear Magnetic Resonance Spectrum (CDCℓ₃, 270 MHz) δ ppm:
   1.02 - 1.11 (6H, multiplet);
   1.24 (6H, triplet, J = 7 Hz);
   3.01 (1H, doublet of quartets, J = 10 & 7 Hz);
   3.12 (2H, quartet, J = 7 Hz);
   3.18 - 3.30 (1H, multiplet);
   3.32 (1H, doublet of doublets, J = 3 & 6 Hz);
   3.39 - 3.52 (1H, multiplet);
   4.19 (1H, quintet, 6 Hz);
   4.28 (1H, doublet of doublets, J = 3 & 10 Hz);
   5.38 (1H, doublet, J = 14 Hz);
   5.52 (1H, doublet, J = 14 Hz);
   7.33 (1H, doublet of doublets, J = 1 & 8 Hz);
   7.58 (1H, doublet of triplets, J = 1 & 8 Hz);
   7.66 (1H, doublet of triplets, J = 1 & 8 Hz);
   7.70 (2H, doublet, J = 9 Hz);
   8.16 (1H, doublet of doublets, J = 1 & 8 Hz);
   8.24 (2H, doublet, J = 9 Hz).

### Higher polarity S-oxide:

Infrared Absorption Spectrum (liquid film), νₘₐₓ cm⁻¹:
   3500-3300, 1786, 1734, 1624, 1522, 1198, 1061.
Nuclear Magnetic Resonance Spectrum (CDCℓ₃, 270 MHz) δ ppm:
   0.83 (3H, doublet, J = 7 Hz);
   1.09 (3H, triplet, J = 7 Hz);
   1.18 (3H, triplet, J = 7 Hz);
   1.28 (3H, doublet, J = 6 Hz);
   3.18 (2H, quartet, J = 7 Hz);
   3.20 - 3.35 (1H, multiplet);
   3.40 - 3.53 (1H, multiplet);
   3.47 (1H, doublet of doublets, J = 3 & 6 Hz);
   3.70 (1H, doublet of quartets, J = 10 & 7 Hz);
   4.25 (1H, quintet, 6 Hz);
   4.38 (1H, doublet of doublets, J = 3 & 10 Hz);
   5.34 (1H, doublet, J = 13 Hz);
   5.48 (1H, doublet, J = 13 Hz);
   7.28 - 7.32 (1H, multiplet);
   7.48 - 7.56 (2H, multiplet);
   7.65 (2H, doublet, J = 9 Hz);
   7.85 - 7.91 (1H, multiplet);
   8.22 (2H, doublet, J = 9 Hz).
Mass spectrum (m/z): 551 [M⁺ (C₂₈H₃₁N₅O₈S)-H₂O].

### EXAMPLE 24

### 4-Nitrobenzyl (1R,5S,6S)-2-[2-(1-perhydroazepinylcarbonyl)phenylsulphinyl]-1-methyl-6-[1(R)-t-butyldimethylsilyloxyethyl]-1-carbapen-2-em-3-carboxylate

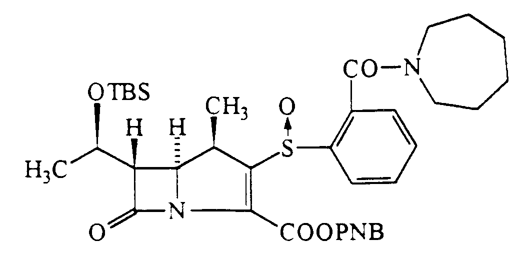

Following a procedure similar to that described in Example 21, but using 4-nitrobenzyl (1R,5S,6S)-2-[2-(1-perhydroazepinylcarbonyl)phenylthio]-1-methyl-6-[1(R)-t-butyldimethylsilyloxyethyl]-1-carbapen-2-em-3-carboxylate (prepared as described in Example 19) as a starting material, in a relative amount similar to that used in that Example, the title compound was obtained as a foam-like solid in a yield of 89%.
Infrared Absorption Spectrum (liquid film), νₘₐₓ cm⁻¹:
   1790, 1734, 1628, 1524, 1347, 1061.
Nuclear Magnetic Resonance Spectrum (CDCℓ₃, 270 MHz) δ ppm:
   0.03, 0.04, 0.05, 0.06 (total 6H, each singlet);
   0.75 (1.5H, doublet, J = 7 Hz);
   0.83 (4.5H, singlet);
   0.84 (4.5H, singlet);
   1.13 (1.5H, doublet, J = 6 Hz);
   1.16 (1.5H, doublet, J = 6 Hz);
   1.28 (1.5H, doublet, J = 7 Hz);
   1.44 - 1.80 (8H, multiplet);
   3.00 (0.5H, doublet of quartets, J = 10 & 7 Hz);
   3.11 - 3.37 (3.5H, multiplet);
   3.41 - 3.50 (1H, multiplet);
   3.60 - 3.77 (1H, multiplet);
   4.19 - 4.31 (1.5H, multiplet);
   4.38 (0.5H, doublet of doublets, J = 10 & 3 Hz);
   5.33 (0.5H, doublet, J = 14 Hz);
   5.40 (0.5H, doublet, J = 14 Hz);
   5.46 (0.5H, doublet, J = 14 Hz);
   5.49 (0.5H, doublet, J = 14 Hz);
   7.27 - 7.32 (0.5H, multiplet);
   7.36 (0.5H, doublet of doublets, J = 7 & 1 Hz);
   7.48 - 7.60 (1.5H, multiplet);
   7.63 - 7.73 (2.5H, multiplet);
   7.93 - 7.96 (0.5H, multiplet);
   8.15 (0.5H, doublet of doublets, J = 8 & 1 Hz);
   8.22 (1H, doublet, J = 9 Hz);
   8.23 (1H, doublet, J = 9 Hz).
Mass spectrum (m/z): 709 (M⁺, C₃₆H₄₇N₃O₈SSi).

### EXAMPLE 25

### 4-Nitrobenzyl (1R,5S,6S)-2-(2-diethylcarbamoylphenylsulphonyl)-1-methyl-6-[1(R)-t-butyldimethylsilyloxyethyl]-1-carbapen-2-em-3-carboxylate

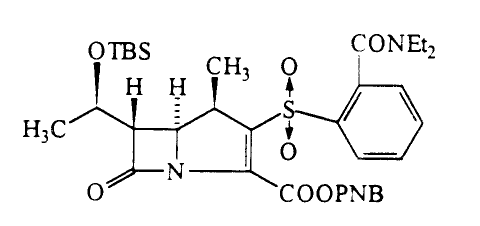

3 ml of a methylene chloride solution containing 157 mg (0.235 mmol) of 4-nitrobenzyl (1R,5S,6S)-2-(2-diethylcarbamoylphenylthio)-1-methyl-6-[1(R)-t-butyldimethylsilyloxyethyl]-1-carbapen-2-em-3-carboxylate (prepared as described in Example 18) was cooled in an ice bath, and 78 mg (0.93 mmol) of sodium hydrogencarbonate and then 78 mg (0.52 mmol) of m-chloroperbenzoic acid (80% purity) were added to the cooled solution. The reaction mixture was then stirred for 20 minutes at the same temperature and for 3.5 hours at room temperature. At the end of this time, an aqueous solution of sodium sulphite was added to the reaction mixture, and the mixture was stirred for 10 minutes to complete the reaction. The reaction solution was then diluted with methylene chloride, washed with an aqueous solution of sodium hydrogencarbonate and condensed by evaporation under reduced pressure. The resulting residue was purified by column chromatography through 15 g of silica gel. Elution with a 4 : 6 by volume mixture of ethyl acetate and hexane afforded 148 mg (yield 90%) of the title compound as a foam-like solid.
Infrared Absorption Spectrum (liquid film), νₘₐₓ cm⁻¹:
   1794, 1748, 1636, 1524, 1320, 1266, 1161.
Nuclear Magnetic Resonance Spectrum (CDCℓ₃, 270 MHz) δ ppm:
   0.03 (3H, singlet);
   0.05 (3H, singlet);
   0.83 (9H, singlet);
   1.06 (3H, triplet, J = 7 Hz);
   1.16 (3H, doublet, J = 6 Hz);
   1.20 - 1.43 (6H, multiplet);
   3.01 - 3.19 (2H, multiplet);
   3.24 - 3.84 (4H, multiplet);
   4.19 - 4.28 (1H, multiplet);
   4.42 - 4.52 (1H, multiplet);
   5.39 (2H, singlet);
   7.25 - 7.34 (1H, multiplet);
   7.48 - 7.65 (2H, multiplet);
   7.62 (2H, doublet, J = 9 Hz);
   8.11 (1H, doublet, J = 8 Hz);
   8.21 (2H, doublet, J = 9 Hz).
Mass spectrum (m/z): 700 [M⁺ (C₃₄H₄₅N₃O₉SSi)+1].

### PREPARATION 1

### S-2-Diethylcarbamoylphenyl 2(R)-[(2S,3S)-3-[1(R)-(trimethylsilyloxy)ethyl]-4-oxo-2-azetidinyl]-thiopropionate

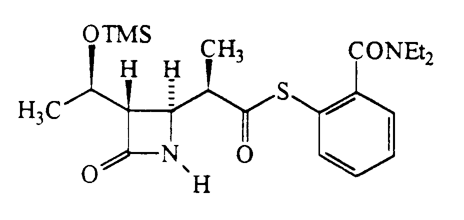

5 ml of 2 N aqueous hydrochloric acid were added to 20 ml of a tetrahydrofuran solution containing 1.002 g (2.03 mmol) of S-2-diethylcarbamoylphenyl 2(R)-[(2S,3S)-3-[1(R)-t-butyldimethylsilyloxyethyl]-4-oxo-2-azetidinyl] thiopropionate (prepared as described in Preparation 9 of this specification and also in Example 1 of Japanese Patent Application No. Hei 4-174099), and the mixture was heated for 3 hours at 50°C. At the end of this time, the reaction solution was cooled and extracted with ethyl acetate. The organic extract was washed with a saturated aqueous solution of sodium hydrogencarbonate, with water and then with a saturated aqueous solution of sodium chloride, in that order, after which the solvent was removed by distillation under reduced pressure. The resulting desilylated compound was dissolved in 10 ml of tetrahydrofuran, and 0.60 ml (4.30 mmol) of triethylamine and 0.53 ml of trimethylsilyl chloride were added to the resulting solution. The reaction mixture was then stirred for 80 minutes at room temperature and then for 100 minutes at 40°C. At the end of this time, the reaction mixture was cooled, diluted with ethyl acetate and washed with twice ice water and then once with a saturated aqueous solution of sodium chloride. The solvent was then removed by distillation under reduced pressure. The resulting residue was purified by column chromatography through 50 g of silica gel, using a gradient elution method, with mixtures of ethyl acetate and hexane in ratios ranging from 3 : 2 to 4 : 1 by volume as the eluent, to give 742 mg (yield 81%) of the title compound as a foam-like solid.
Infrared Absorption Spectrum (liquid film), νₘₐₓ cm⁻¹:
   3250, 1760, 1700, 1625.
Nuclear Magnetic Resonance Spectrum (CDCℓ₃, 270 MHz) δ ppm:
   0.12 (9H, singlet);
   1.03 (3H, triplet, J = 7 Hz);
   1.23 (3H, doublet, J = 6 Hz);
   1.25 (3H, triplet, J = 7 Hz);
   1.22 - 1.31 (3H, multiplet);
   2.95 - 3.12 (4H, multiplet);
   3.25 - 3.85 (2H, multiplet);
   3.90 (1H, doublet of doublets, J = 2 & 4 Hz);
   4.14 (1H, quintet, J = 6 Hz);
   6.07 - 6.18 (1H, broad singlet);
   7.32 - 7.35 (1H, multiplet);
   7.41 - 7.51 (3H, multiplet).
Mass spectrum (m/z): 450 (M⁺, C₂₂H₃₄N₂O₄SSi).

### PREPARATION 2

### 4-Nitrobenzyl (1R,5S,6S)-6-[1(R)-t-butyldimethylsilyl-oxyethyl]-1-methyl-2-phenylsulphinyl-1-carbapen-2-em-3-carboxylate

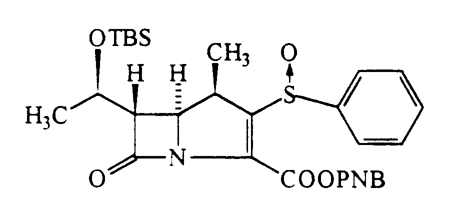

Following a procedure similar to that described in Example 21, but using 4-nitrobenzyl (1R,5S,6S)-6-[1(R)-t-butyldimethylsilyloxyethyl]-1-methyl-2-phenylthio-1-carbapen-2-em-3-carboxylate (prepared as described in Example 7) as a starting material, in a relative amount similar to that used in that Preparation, and then purifying the product by silica gel column chromatography, using a gradient elution method, with mixtures of diethyl ether and methylene chloride in ratios ranging from 5 : 95 to 20 : 80 by volume as the eluent, the lower polarity S-oxide was obtained as crystals in a yield of 62%, and the higher polarity S-oxide was obtained as a foam-like solid in a yield of 30%.

Recrystallization of the lower polarity S-oxide from a mixture of ethyl acetate and hexane afforded crystals of that isomer melting at 150 - 151°C.

### Isomer of lower polarity:

Infrared Absorption Spectrum (KBr), νₘₐₓ cm⁻¹:
   1796, 1715, 1528, 1335, 1044, 839.
Nuclear Magnetic Resonance Spectrum (CDCℓ₃, 270 MHz) δ ppm:
   0.06 (3H, singlet);
   0.07 (3H, singlet);
   0.73 (3H, doublet, J = 7 Hz);
   0.85 (9H, singlet);
   1.18 (3H, doublet, J = 6 Hz);
   3.27 (1H, doublet of doublets, J = 3 & 5 Hz);
   3.90 (1H, doublet of quartets, J = 11 & 7 Hz);
   4.23 (1H, doublet of quartets, J = 5 & 6 Hz);
   4.41 (1H, doublet of doublets, J = 3 & 11 Hz);
   5.36 (1H, doublet, J = 14 Hz);
   5.56 (1H, doublet, J = 14 Hz);
   7.46 - 7.53 (3H, multiplet);
   7.66 (2H, doublet, J = 9 Hz);
   7.72 - 7.78 (2H, multiplet);
   8.24 (2H, doublet, J = 9 Hz).

### Isomer of higher polarity:

Infrared Absorption Spectrum (liquid film), νₘₐₓ cm⁻¹:
   1788, 1730, 1524, 1319, 1273, 1047, 837.
Nuclear Magnetic Resonance Spectrum (CDCℓ₃, 270 MHz) δ ppm:
   0.04 (3H, singlet);
   0.05 (3H, singlet);
   0.83 (9H, singlet);
   1.15 (3H, doublet, J = 6 Hz);
   1.39 (3H, doublet, J = 7 Hz);
   3.16 (1H, doublet of quartets, J = 10 & 7 Hz);
   3.39 (1H, doublet of doublets, J = 3 & 4 Hz);
   4.16 (1H, doublet of doublets, J = 3 & 10 Hz);
   4.26 (1H, doublet of quartets, J = 4 & 6 Hz);
   5.40 (1H, doublet, J = 14 Hz);
   5.49 (1H, doublet, J = 14 Hz);
   7.51 - 7.58 (3H, multiplet);
   7.68 (2H, doublet, J = 9 Hz);
   7.68 - 7.73 (2H, multiplet);
   8.23 (2H, doublet, J = 9 Hz).
Mass spectrum (m/z) (both isomers): 584 (M⁺, C₂₉H₃₆N₂O₇SSi).

### PREPARATION 3

### 4-Nitrobenzyl (1R,5S,6S)-6-[1(R)-t-butyldimethylsilyloxyethyl]-1-methyl-2-phenylsulphonyl-1-carbapen-2-em-3-carboxylate

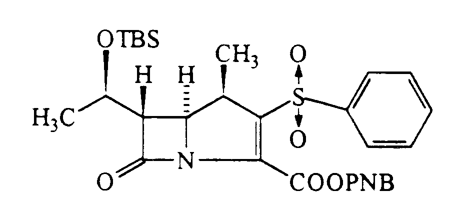

Following a procedure similar to that described in Example 21, but using the isomer of higher polarity of 4-nitrobenzyl (1R,5S,6S)-6-[1(R)-t-butyldimethylsilyloxyethyl]-1-methyl-2-phenylsulphinyl-1-carbapen-2-em-3-carboxylate (prepared as described in Preparation 2) as a starting material, in a relative amount similar to that used in that Preparation, the title compound was obtained as a foam-like solid in a yield of 87%.
Infrared Absorption Spectrum (liquid film), νₘₐₓ cm⁻¹:
   1796, 1750, 1524, 1316, 1264, 1161, 1079, 837.
Nuclear Magnetic Resonance Spectrum (CDCℓ₃, 270 MHz) δ ppm:
   0.03 (3H, singlet);
   0.05 (3H, singlet);
   0.84 (9H, singlet);
   1.16 (3H, doublet, J = 6 Hz);
   1.19 (3H, doublet, J = 7 Hz);
   3.40 (1H, doublet of quartets, J = 11 & 7 Hz);
   3.46 - 3.52 (1H, multiplet);
   4.23 (1H, doublet of quartets, J = 4 & 6 Hz);
   4.44 (1H, doublet of doublets, J = 4 & 11 Hz);
   5.45 (2H, singlet);
   7.51 - 7.58 (2H, multiplet);
   7.60 - 7.68 (3H, multiplet);
   8.00 (2H, doublet, J = 9 Hz);
   8.23 (2H, doublet, J = 9 Hz).
Mass spectrum (m/z): 543 [M⁺ (C₂₉H₃₆N₂O₈SSi)-t-Bu].

### PREPARATION 4

### 4-Nitrobenzyl (5S,6S)-6-[1(R)-t-butyldimethylsilyloxyethyl]-2-phenylsulphinyl-1-carbapen-2-em-3-carboxylate

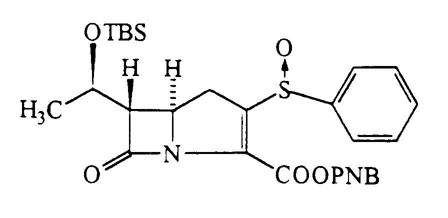

Following a procedure similar to that described in Example 21 but using 4-nitrobenzyl (5S,6S)-6-[1(R)-t-butyldimethylsilyloxyethyl]-3-phenylthio-1-carbapen-2-em-3-carboxylate [Tetrahedron Letters 25, 2793 (1984)] as a starting material, in a relative amount similar to that used in that Example. The product was separated and purified by silica gel column chromatography eluted with a 4 : 6 by volume mixture of ethyl acetate and hexane, to give the lower polarity S-oxide as crystals in a yield of 43%, and the higher polarity S-oxide as a foam-like solid in a yield of 48%. Recrystallization of the lower polarity S-oxide from diisopropyl ether afforded it in the form of crystals melting at 111.5 - 114°C.

### Isomer of lower polarity:

Infrared Absorption Spectrum (KBr), νₘₐₓ cm⁻¹:
   1786, 1723, 1607, 1526, 1323, 1206, 1044, 837.
Nuclear Magnetic Resonance Spectrum (CDCℓ₃, 270 MHz) δ ppm:
   0.05 (3H, singlet);
   0.06 (3H, singlet);
   0.85 (9H, singlet);
   1.17 (3H, doublet, J = 6 Hz);
   2.55 (1H, doublet of doublets, J = 9 & 18 Hz);
   3.08 (1H, doublet of doublets, J = 3 & 5 Hz);
   3.51 (1H, doublet of doublets, J = 11 & 18 Hz);
   4.18 (1H, doublet of quartets, J = 5 & 6 Hz);
   4.37 (1H, doubled doublet of doublets, J = 3, 9 & 11 Hz);
   5.37 (1H, doublet, J = 14 Hz);
   5.54 (1H, doublet, J = 14 Hz);
   7.48 - 7.54 (3H, multiplet);
   7.65 (2H, doublet, J = 9 Hz);
   7.70 - 7.77 (2H, multiplet);
   8.24 (2H, doublet, J = 9 Hz).
Mass spectrum (m/z): 570 (M⁺, C₂₈H₃₄N₂O₇SSi).

### Isomer of higher polarity:

Infrared Absorption Spectrum (liquid film), νₘₐₓ cm⁻¹:
   1790, 1723, 1607, 1524, 1322, 1269, 1044, 837.
Nuclear Magnetic Resonance Spectrum (CDCℓ₃, 270 MHz) δ ppm:
   0.05 (3H, singlet);
   0.06 (3H, singlet);
   0.84 (9H, singlet);
   1.18 (3H, doublet, J = 6 Hz);
   2.80 (1H, doublet of doublets, J = 11 & 19 Hz);
   3.29 (1H, doublet of doublets, J = 8 & 19 Hz);
   3.34 (1H, triplet, J = 3 Hz);
   4.17 - 4.30 (2H, multiplet);
   5.40 (1H, doublet, J = 14 Hz);
   5.49 (1H, doublet, J = 14 Hz);
   7.49 - 7.55 (3H, multiplet);
   7.68 (2H, doublet, J = 9 Hz);
   7.70 - 7.76 (2H, multiplet);
   8.23 (2H, doublet, J = 9 Hz).

### PREPARATION 5

### 4-Nitrobenzyl (1R,5S,6S)-6-[1(R)-t-butyldimethylsilyloxyethyl]-1-methyl-2-[2-(4-nitrobenzyloxycarbonyl)-aminoethylsulphinyl)-1-carbapen-2-em-3-carboxylate

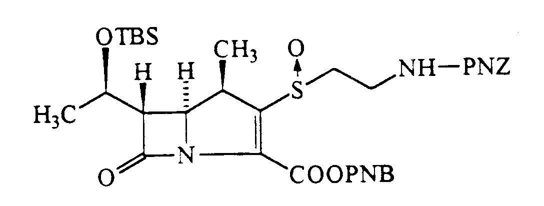

Following a procedure similar to that described in Example 21, but using 4-nitrobenzyl (1R,5S,6S)-6-[1(R)-t-butyldimethylsilyloxyethyl]-1-methyl-2-[2-(4-nitrobenzyloxycarbonyl)aminoethylthio]-1-carbapen-2-em-3-carboxylate (prepared as described in Example 5) as a starting material, in a relative amount similar to that used in that Example, the title compound was obtained as a foam-like solid in a yield of 92%.
Infrared Absorption Spectrum (liquid film), νₘₐₓ cm⁻¹:
   1786, 1721, 1607, 1522, 1347, 1254, 1048.
Nuclear Magnetic Resonance Spectrum (CDCℓ₃, 270 MHz) δ ppm:
   0.07 (3H, singlet);
   0.09 (3H, singlet);
   0.86 (9H, singlet);
   1.23 (1.5H, doublet, J = 6 Hz);
   1.24 (1.5H, doublet, J = 6 Hz);
   1.39 (1.5H, doublet, J = 7 Hz);
   1.40 (1.5H, doublet, J = 7 Hz);
   3.12 - 3.45 (3H, multiplet);
   3.60 - 3.92 (3H, multiplet);
   4.29 (1H, quartet, J = 6 Hz);
   4.34 (0.5H, doublet of doublets, J = 3 & 10 Hz);
   4.46 (0.5H, doublet of doublets, J = 3 & 10 Hz);
   5.14 - 5.46 (2H, multiplet);
   5.23 (2H, singlet);
   5.68 - 5.77 (1H, multiplet);
   7.51 (1H, doublet, J = 9 Hz);
   7.52 (1H, doublet, J = 9 Hz);
   7.61 (1H, doublet, J = 9 Hz);
   7.66 (1H, doublet, J = 9 Hz);
   8.20 - 8.24 (4H, multiplet).
Mass spectrum (m/z): 705 [M⁺ (C₃₃H₄₂N₄O₁₁SSi)-CH₃].

### PREPARATION 6

### 4-Nitrobenzyl (1R,5S,6S)-6-[1(R)-t-butyldimethylsilyloxyethyl]-1-methyl-2-ethylsulphinyl-1-methyl-1-carbapen-2-em-3-carboxylate

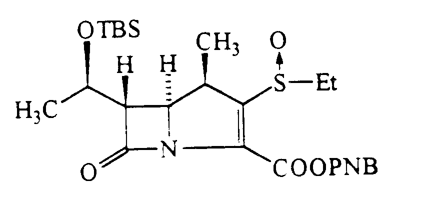

Following a procedure similar to that described in Example 21, but using 4-nitrobenzyl (1R,5S,6S)-6-[1(R)-t-butyldimethylsilyloxyethyl]-2-ethylthio-1-methyl-1-carbapen-2-em-3-carboxylate (prepared as described in Example 9) as a starting material, in a relative amount similar to that used in that Example, the title compound was obtained as a foam-like solid in a yield of 92%.
Infrared Absorption Spectrum (liquid film), νₘₐₓ cm⁻¹:
   1784, 1715, 1524, 1333, 1055, 837.
Nuclear Magnetic Resonance Spectrum (CDCℓ₃, 270 MHz) δ ppm:
   0.08 (3.6H, singlet);
   0.09 (2.4H, singlet);
   0.86 (9H, singlet);
   1.24 (3H, doublet, J = 6 Hz);
   1.39 (3H, doublet, J = 7 Hz);
   1.41 (1.8H, triplet, J = 7 Hz);
   1.44 (1.2H, triplet, J = 7 Hz);
   2.93 - 3.10 (2H, multiplet);
   3.38 (0.6H, doublet of doublets, J = 3 & 5 Hz);
   3.41 (0.4H, doublet of doublets, J = 3 & 5 Hz);
   3.61 (0.6H, doublet of quartets, J = 10 & 7 Hz);
   3.85 (0.4H, doublet of quartets, J = 10 & 7 Hz);
   4.23 - 4.34 (1.6H, multiplet);
   4.42 (0.4H, doublet of doublets, J = 3 & 10 Hz);
   5.27 (0.4H, doublet, J = 14 Hz);
   5.32 (0.6H, doublet, J = 14 Hz);
   5.41 (0.6H, doublet, J = 14 Hz);
   5.44 (0.4H, doublet, J = 14 Hz);
   7.63 (1.2H, doublet, J = 9 Hz);
   7.66 (0.8H, doublet, J = 9 Hz);
   8.23 (2H, doublet, J = 9 Hz).
Mass spectrum (m/z): 479 (M⁺ (C₂₅H₃₆N₂O₇SSi)-t-Bu).

### PREPARATION 7

### 4-Nitrobenzyl (5R,6S)-6-[1(R)-t-butyldimethylsilyloxyethyl]-2-ethylsulphinylpenem-3-carboxylate

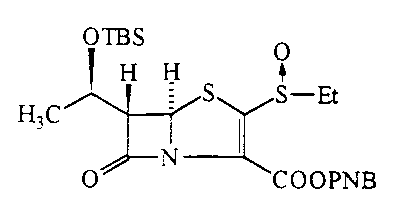

20 ml of a methylene chloride solution containing 1.039 g (1.98 mmol) of 4-nitrobenzyl (5R,6S)-6-[1(R)-t-butyldimethylsilyloxyethyl]-2-ethylthiopenem-3-carboxylate was cooled in an ice bath and 469 mg of m-chloroperbenzoic acid (purity 80%) was added carefully over a period of 5 minutes to the cooled solution. The reaction mixture was stirred for 30 minutes at the same temperature, and then a further 88 mg of m-chloroperbenzoic acid (purity 80%) (making a total of 557 mg, 2.58 mmol) were added, and the mixture was stirred for a further 15 minutes. At the end of this time, the reaction mixture was diluted with methylene chloride and washed with a dilute aqueous solution of sodium hydrogencarbonate. The organic layer was condensed by evaporation under reduced pressure, and the resulting residue was purified by column chromatography through 50 g of silica gel. Elution with a 4 : 6 by volume mixture of ethyl acetate and hexane afforded, in order, the following:
202 mg (yield 19%) of an S-oxide of lower polarity as a foam-like solid;
336 mg (31% yield) of a mixture of the S-oxides of lower and higher polarity in a ratio of 1 : 3; and
72 mg (7% yield) of an S-oxide of higher polarity as a foam-like solid.

In Examples 12 and 13, all three of the fractions separated by column chromatography were recombined for use as the starting materials.

### Isomer of lower polarity:

Infrared Absorption Spectrum (liquid film), νₘₐₓ cm⁻¹:
   1796, 1701, 1524, 1323, 1063.
Nuclear Magnetic Resonance Spectrum (CDCℓ₃, 270 MHz) δ ppm:
   0.05 (3H, singlet);
   0.08 (3H, singlet);
   0.84 (9H, singlet);
   1.26 (3H, doublet, J = 6 Hz);
   1.44 (3H, triplet, J = 7 Hz);
   3.10 (2H, quartet, J = 7 Hz);
   3.92 (1H, doublet of doublets, J = 1 & 3 Hz);
   4.28 (1H, doublet of quartets, J = 3 & 6 Hz);
   5.26 (1H, doublet, J = 14 Hz);
   5.38 (1H, doublet, J = 14 Hz);
   5.73 (1H, doublet, J = 1 Hz);
   7.62 (2H, doublet, J = 9 Hz);
   8.23 (2H, doublet, J = 9 Hz).

### Isomer of higher polarity:

Infrared Absorption Spectrum (liquid film), νₘₐₓ cm⁻¹:
   1794, 1702, 1524, 1321, 1063
Nuclear Magnetic Resonance Spectrum (CDCℓ₃, 270 MHz) δ ppm:
   0.04 (3H, singlet);
   0.07 (3H, singlet);
   0.82 (9H, singlet);
   1.26 (3H, doublet, J = 6 Hz);
   1.44 (3H, triplet, J = 7 Hz);
   3.13 (2H, doublet of quartets, J = 4 & 8 Hz);
   3.90 (1H, doublet of doublets, J = 2 & 4 Hz);
   4.27 (1H, doublet of quartets, J = 4 & 6 Hz);
   5.22 (1H, doublet, J = 14 Hz);
   5.41 (1H, doublet, J = 14 Hz);
   5.85 (1H, doublet, J = 2 Hz);
   7.59 (2H, doublet, J = 9 Hz);
   8.23 (2H, doublet, J = 9 Hz).

### PREPARATION 8

### 4-Nitrobenzyl (1R,5S,6S)-2-[(3S,5S)-5-dimethylcarbamoyl-1-(4-nitrobenzyloxycarbonyl)-3-pyrrolidinylthio]-1-methyl-6-[1(R)-hydroxyethyl]-1-carbapen-2-em-3-carboxylate

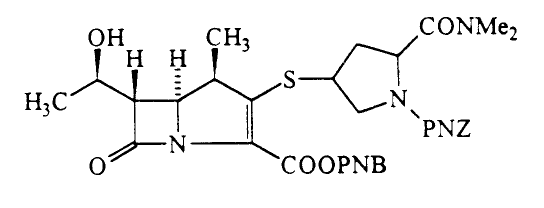

8 mg (0.039 mmol) of citric acid hydrate were added to 2 ml of a solution containing 55 mg of 4-nitrobenzyl (1R,5S,6S)-2-[(3S,5S)-5-dimethylcarbamoyl-1-(4-nitrobenzyloxycarbonyl)-3-pyrrolidinylthio]-1-methyl-6-[1(R)-trimethylsilyloxyethyl]-1-carbapen-2-em-3-carboxylate (prepared as described in Example 1) in methanol, and the resulting mixture was stirred at room temperature for 35 minutes. At the end of this time, the solvent was removed by distillation under reduced pressure, and the residue was purified by column chromatography through 5 g of silica gel, using a 1 : 9 by volume mixture of methanol and ethyl acetate as the eluent, to give 42 mg (yield 84%) of the title compound as a vitreous solid.

The physicochemical properties of this compound were in agreement with those of the compound prepared as described in Example 10.

### PREPARATION 9

### S-2-Diethylcarbamoylphenyl 2(R)-{(2S,3S)-3-[1(R)-(t-butyldimethylsilyloxy)ethyl]-4-oxo-2-azetidinyl}thiopropionate

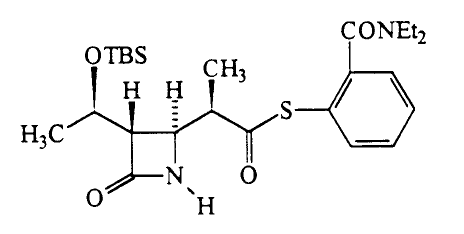

### 9(a) 1-t-Butyldimethylsilyloxy-1-(2-diethylcarbamoyl)phenylthio-1-propene

1.7 ml (12.2 mmol) of triethylamine were added to a solution of 3.20 g (12.1 mmol) of S-(2-diethylcarbamoyl)phenyl thiopropionate, which is described in Referential Example 12 of Japanese Patent Kokai Application No. Hei 4-174099, and 3.64 g (24.1 mmol) of t-butyldimethylsilyl chloride in a mixture of 12 ml of dimethylformamide and 12 ml of tetrahydrofuran, and the resulting mixture was stirred at room temperature for 3 minutes. At the end of this time, the solution was cooled to -78°C, and 12.1 ml of a 1.0 M tetrahydrofuran solution of lithium bis(trimethylsilyl)amide were added dropwise over a period of 20 minutes. The mixture was then stirred at the same temperature for 30 minutes, after which 10 ml of a saturated aqueous solution of sodium hydrogencarbonate were added. The reaction mixture was then extracted with hexane, and the extract was washed twice with water, once with a 2 N aqueous solution of sodium hydroxide, three times with water and once with a saturated aqueous solution of sodium chloride, in that order. The extract was then dried over anhydrous magnesium sulphate, and the solvent was removed by distillation under reduced pressure. The resulting residue was again dissolved in hexane, and the solution was mixed with 9.0 g of aluminium oxide for chromatography and then stirred for 15 minutes. The aluminium oxide was removed from the mixture by filtration, and the aluminium oxide was washed with hexane. The filtrate and the washings were combined and the solvent was removed by distillation under reduced pressure, to give 4.24 g of Z(O)-1-t-butyldimethylsilyloxy-1-(2-diethylcarbamoyl)phenylthio-1-propene.
Analysis by nuclear magnetic resonance spectroscopy (270 MHz) showed that the ratio of the Z(O)-isomer to the E(O)-isomer in the product was more than 20.

### 9(b) S-2-Diethylcarbamoylphenyl 2(R)-{(2S,3S)-3-[1(R)-(t-butyldimethylsilyloxy)ethyl]-4-oxo-2-azetidinyl}thiopropionate

1.49 g (10.9 mmol) of anhydrous zinc chloride were added to a solution of 4.24 g of Z(O)-1-t-butyldimethylsilyloxy-1-(2-diethylcarbamoyl)phenylthio-1-propene [prepared as described in step (a) above] and 1.57 g (5.47 mmol) of (3R,4S)-3-[1(R)-t-butyldimethylsilyloxyethyl]-4-acetoxy-2-azetidinone in 55 ml of methylene chloride, and the resulting mixture was heated under reflux for 40 minutes. At the end of this time, the reaction mixture was cooled to room temperature, and the solvent was removed by distillation under reduced pressure. The residue was dissolved in ethyl acetate, and the solution was washed three times with water and once with a saturated aqueous solution of sodium chloride. The mixture was then dried over anhydrous magnesium sulphate, after which the solvent was removed by distillation under reduced pressure, and the residue was recrystallized from about 10 ml of diisopropyl ether, to give 2.08 g (yield 77%) of the title compound as crystals, melting at 130.5 - 132°C.
Nuclear Magnetic Resonance Spectrum (CDCℓ₃, 270 MHz), δ ppm:
   0.08 (6H, singlet);
   0.87 (9H, singlet);
   1.03 (4H, triplet, J = 7 Hz);
   1.21 (3H, doublet, J = 6 Hz);
   1.25 (3H, triplet, J = 7 Hz);
   1.29 (3H, doublet, J = 7 Hz);
   2.96 - 3.15 (4H, multiplet);
   3.20 - 3.85 (2H, broad);
   3.96 (1H, dd, J = 2, 4 Hz);
   4.19 (1H, quintet, J = 6 Hz);
   5.90 - 6.10 (1H, broad singlet);
   7.30 - 7.35 (1H, multiplet);
   7.41 - 7.51 (3H, multiplet).
Mass spectrum (m/z): 492 (M⁺, C₂₅H₄₀N₂O₄SSi).

## Claims

1. A process for preparing a compound of formula (I): 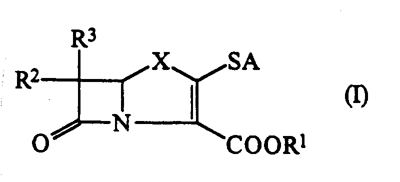 in which:
R¹ represents a hydrogen atom or a carboxy-protecting group;
R² and R³ are the same or different and each represents a hydrogen atom, an alkyl group having from 1 to 6 carbon atoms or a group of formula 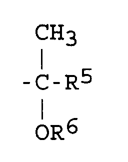
where R⁵ represents a hydrogen atom or a methyl group; and R⁶ represents a hydrogen atom or a hydroxy-protecting group;
or
R² and R³ together represent a group of formula =C(CH₃)CH₂OR⁶, in which R⁶ is as defined above;
X represents a sulfur atom or a group of formula >CHR⁷, where R⁷ represents a hydrogen atom, an alkyl group having from 1 to 6 carbon atoms or an alkoxy group having from 1 to 6 carbon atoms; and
A represents
an alkyl group which has from 1 to 6 carbon atoms and which is unsubstituted or is substituted by 1 or 2 of substituents K, defined below,
an aryl group, as defined below,
an aralkyl group in which an alkyl group having from 1 to 6 carbon atoms is substituted by at least one aryl group as defined below,
an aromatic or non-aromatic heterocylic group which may be monocyclic or may consist of two or more rings attached to each other by fusion or by a spiro attachment and which has from 3 to 10 ring atoms, at least one of said atoms being a nitrogen and/or oxygen and/or sulfur hetero-atom, said group being unsubstituted or being substituted by at least one of substituents B on carbon atoms, by at least one of substituents B¹ on nitrogen hetero-atoms, and/or by at least one oxygen atom to form a sulfinyl or sulfonyl group on sulfur hetero-atoms, all as defined below;
a fused heterocyclic group in which a heterocyclic group as defined above is fused to an aryl group as defined below, or
an alkyl group which has from 1 to 6 carbon atoms and which is substituted by at least one heterocyclic group and/or fused heterocyclic group as defined above;
said aryl groups are aromatic carbocyclic groups having from 6 to 14 ring carbon atoms and are unsubstituted or are substituted by at least one of substituents C, defined below;
said substituents K are selected from hydroxy, protected hydroxy groups, amino, protected amino groups, groups of formula -C(=NR¹⁰)NR¹¹R¹², where
R¹⁰, R¹¹ and R¹² are the same or different and each represents a hydrogen atom, an amino-protecting group or an alkyl group having from 1 to 6 carbon atoms, or R¹¹ and R¹² together represent a group of formula -(CH₂)ₙ-, where n is an integer from 2 to 6, or R¹⁰ and R¹¹ together represent a group of formula -(CH₂)ₚ-, where p is 2 or 3, and
groups of formula -NR¹³C(=NR¹⁴)R¹⁵, where
R¹³ and R¹⁴ are the same or different and each represents a hydrogen atom, an amino-protecting group or an alkyl group having from 1 to 6 carbon atoms, and
R¹⁵ represents a hydrogen atom, an alkyl group having from 1 to 6 carbon atoms, an amino or a protected amino group,
or
any two of R¹³, R¹⁴ and R¹⁵ together represent a group of formula -(CH₂)ₚ-, where p is 2 or 3;
said substituents B are selected from
alkyl groups having from 1 to 6 carbon atoms,
alkoxy groups having from 1 to 6 carbon atoms,
hydroxy,
halogen,
cyano,
nitro,
alkoxycarbonyl groups having from 2 to 7 carbon atoms,
carboxy,
oxygen atoms, to form with a ring carbon atom a carbonyl group,
cycloalkyl groups having from 3 to 7 ring carbon atoms,
alkoxyalkyl groups in which the alkoxy and alkyl parts both have from 1 to 6 carbon atoms,
alkoxycarbonylalkyl groups in which the alkoxy and alkyl parts both have from 1 to 6 carbon atoms,
cyanoalkyl groups in which the alkyl part has from 1 to 6 carbon atoms,
haloalkyl groups having from 1 to 6 carbon atoms,
alkanoyloxy groups having from 1 to 6 carbon atoms,
azido,
alkylthio groups having from 1 to 6 carbon atoms,
alkylsulfinyl groups having from 1 to 6 carbon atoms,
alkylsulfonyl groups having from 1 to 6 carbon atoms,
groups of formula -NR⁸R⁹ and groups of formula -CONR⁸R⁹, where R⁸ and R⁹ are the same or different and each represents a hydrogen atom, an amino-protecting group, an alkyl group having from 1 to 6 carbon atoms or a phenyl group, or R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
groups of formulae (B-I), (B-II), (B-III), (B-IV), (B-V), (B-VI), (B-VII), (B-VIII) and (B-IX): 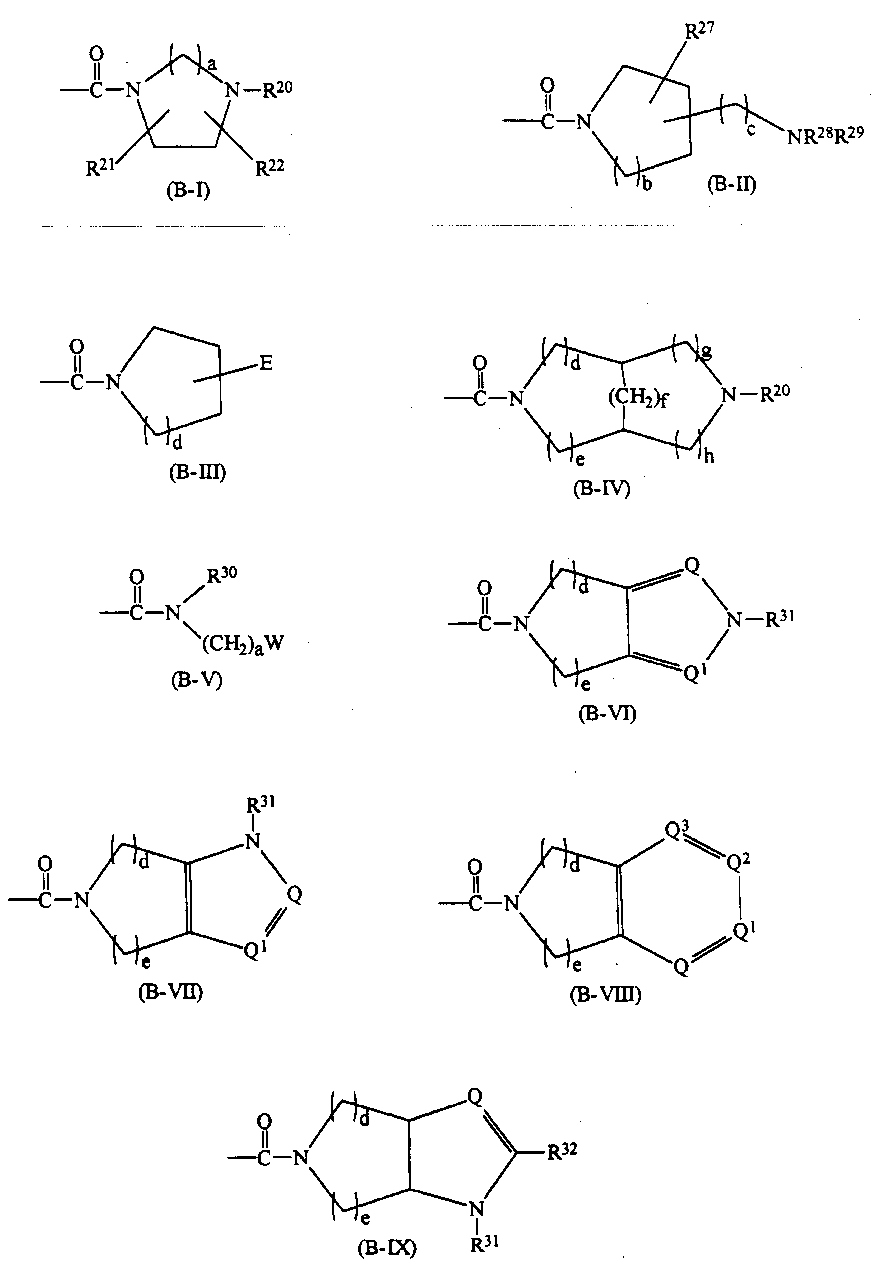 in which:
a is 1, 2 or 3,
b is 0, 1 or 2,
c is 0 or 1,
d is 0, 1 or 2,
e is 0, 1 or 2,
f is 0, 1 or 2,
g is 0, 1 or 2,
h is 0, 1 or 2,
R²⁰ represents a hydrogen atom, an amino-protecting group, a group of formula-COR²⁶,
where
R²⁶ represents an alkyl group having from 1 to 6 carbon atoms or a substituted alkyl group which has from 1 to 6 carbon atoms and which is substituted by at least one of substituents K;
a group of formula -C(=NR¹⁶)R¹⁷, where
R¹⁶ and R¹⁷ are the same or different and each represents a hydrogen atom, an alkyl group having from 1 to 6 carbon atoms, an amino, a protected amino group or an amino-protecting group;
an unsubstituted alkyl group having from 1 to 6 carbon atoms, or a substituted alkyl group which has from 1 to 6 carbon atoms and which is substituted by at least one of substituents D, defined below
R²¹ and R²² are the same or different and each represents a hydrogen atom, an alkyl group having from 1 to 6 carbon atoms, a hydroxy or a protected hydroxy groups;
R²⁷ represents a hydrogen atom or an alkyl group having from 1 to 6 carbon atoms;
R²⁸ and R²⁹ are the same or different and each represents a hydrogen atom, an alkyl group having from 1 to 6 carbon atoms, an amino-protecting group, an amino, a protected amino group and a group of formula -C(=NR¹⁶)R¹⁷, where R¹⁶ and R¹⁷ are as defined above;
R³⁰ represents a hydrogen atom, an alkyl group having from 1 to 6 carbon atoms or a substituted alkyl group which has from 1 to 6 carbon atoms and which is substituted by at least one substituent selected from hydroxy and protected hydroxy groups;
R³¹ represents a hydrogen atom, an alkyl group having from 1 to 6 carbon atoms or an amino-protecting group;
R³² represents a hydrogen atom, an alkyl group having from 1 to 6 carbon atoms or a group of formula -NR²⁸R²⁹, in which R²⁸ and R²⁹ are as defined above;
E represents an imidazolyl group or a triazolyl group;
W represents an aromatic heterocyclic group having from 5 to 8 ring atoms, of which from 1 to 4 are nitrogen atoms, said aromatic heterocyclic group being unsubstituted or being substituted by at least one alkyl group having from 1 to 6 carbon atoms;
Q, Q¹, Q² and Q³ are independently selected from the group consisting of groups of formula 〉̶CH and 〉̶N;
said substituents B¹ are selected from alkyl groups having from 1 to 6 carbon atoms, amino-protecting groups, groups of formula -C(=NR¹⁶)R¹⁷, where R¹⁶ and R¹⁷ are as defined above, amino-protecting groups and groups of formula -CONR¹⁸R¹⁹, where
R¹⁸ and R¹⁹ are the same or different and each represents an alkyl group having from 1 to 6 carbon atoms or a carboxylic acyl group;
said substituents C are selected from alkyl groups having from 1 to 6 carbon atoms, alkoxy groups having from 1 to 6 carbon atoms, halogen atoms, alkoxycarbonyl groups having from 2 to 7 carbon atoms, groups of formula -CONR⁸R⁹, where R⁸ and R⁹ are as defined above, cyano, hydroxy and nitro;
said substituents D are selected from hydroxy, protected hydroxy groups, carboxy, protected carboxy groups, cyano, alkoxy groups having from 1 to 6 carbon atoms, alkylsulfonyl groups having from 1 to 6 carbon atoms, groups of formula -NHCOR²³, -NR²⁴R²⁵, -CONR²⁴R²⁵ or -OCONR²⁴R²⁵, where
R²³, R²⁴ and R²⁵ are the same or different and each represents a hydrogen atom or an alkyl group having from 1 to 6 carbon atoms,
or a pharmaceutically acceptable salt thereof or a pharmaceutically acceptable ester thereof, which process comprises reacting a compound of formula (II): 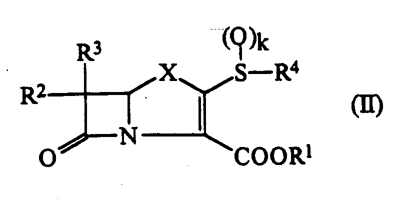
in which R¹, R², R³ and X are as defined above, k is 1 or 2, and R⁴ represents:
an alkyl group which has from 1 to 6 carbon atoms and which is unsubstituted or is substituted by at least one of substituents E, defined below,
an alkenyl group which has from 2 to 6 carbon atoms and which is unsubstituted or is substituted by at least one of substituents E, defined below,
an aryl group, as defined above,
an aralkyl group in which an alkyl group having from 1 to 6 carbon atoms is substituted by at least one aryl group as defined above,
a cycloalkyl group which has from 3 to 7 carbon atoms and which is unsubstituted or is substituted by at least one of substituents C, defined above,
an aromatic heterocylic group which has from 5 to 7 ring atoms in an aromatic ring, at least one of said
atoms being a nitrogen and/or oxygen and/or sulfur hetero-atom, said group being unsubstituted or being substituted by at least one of substituents C, defined above;
a fused heterocyclic group in which an aromatic heterocyclic group as defined above is fused to an aryl group as defined above, or
an alkyl group which has from 1 to 6 carbon atoms and which is substituted by at least one aromatic heterocylic group and/or fused heterocyclic group defined above; and
said substituents E are selected from hydroxy, protected hydroxy groups, amino and protected amino groups;
with a compound of formula (III):
ASH (III)
in which A is as defined above, and, if desired, removing any protecting groups, characterised in that the reaction of said compound of formula (II) with said compound of formula (III) is carried out in the presence of a salt of magnesium or aluminium selected from the following group:
a magnesium bromide - diethyl ether complex; a mixture of magnesium bromide - diethyl ether complex with diisopropylethylamine, 1-ethylpiperidine or 1,5-diazabicyclo[5,4,0]-5-undecene; bromomagnesium cyclohexylisopropylamide, bromomagnesium diisopropylamide, bromomagnesium diethylamide or bromomagnesium hexamethyldisilazide; bromomagnesium t-butoxide; mixtures of lithium diisopropylamide or lithium bistrimethylsilylamide with a magnesium bromide - diethyl ether complex; or diethylaluminium diisopropylamide;
the compound of formula (II) and the mercaptan of formula (III) being employed in a molar ratio of from 1 : 1 to 1 : 3 and the salt of magnesium or aluminium being present in an amount of from 1 to 20 moles per mole of the compound of formula (II).

2. A process according to Claim 1, in which said salt is a salt of aluminum.

3. A process according to Claim 1, in which said salt is a salt of magnesium.

4. A process according to any one of Claims 1 to 3, wherein:
R¹ represents:
a hydrogen atom,
an alkyl group having from 1 to 6 carbon atoms,
an aralkyl group which is an alkyl group having from 1 to 4 carbon atoms and which is substituted by at least one aryl group,
an alkenyl group having from 2 to 6 carbon atoms, and which is unsubstituted or is substituted by one or more halogen atoms,
a haloalkyl group having from 1 to 6 carbon atoms,
a tri-substituted silylethyl group, in which all three or two or one of the substituents are alkyl groups having from 1 to 5 carbon atoms, and none, one or two of the substituents are aryl groups,
an acyloxyalkyl group, in which the acyl part is an alkanoyl group having from 1 to 6 carbon atoms and the alkyl part has from 1 to 6 carbon atoms,
an alkoxycarbonyloxyalkyl group in which the alkoxy and alkyl parts each have from 1 to 6 carbon atoms,
a cycloalkoxycarbonyloxyalkyl group in which the cycloalkyl part has from 3 to 7 ring carbon atoms and is unsubstituted or substituted by at least one of substituents C, and the alkyl part has from 1 to 6 carbon atoms
a (5-alkyl or 5-aryl -2-oxo-1,3-dioxolen-4-yl)methyl group in which the alkyl part has from 1 to 4 carbon atoms; or
the 3-phthalidyl group.

5. A process according to any one of Claims 1 to 4, in which:
R² and R³ are the same or different and each represents a hydrogen atom, an alkyl group having from 1 to 4 carbon atoms or a group of formula 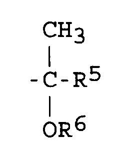
where R⁵ represents a hydrogen atom or a methyl group; and R⁶ represents:
a hydrogen atom,
a substituted silyl group, in which the silyl group has 3 substituents selected from alkyl groups having from 1 to 6 carbon atoms and phenyl groups,
an aralkyl group, in which an alkyl group having from 1 to 4 carbon atoms is substituted by at least one aryl group,
an aralkyloxycarbonyl group, in which the aralkyl part is as defined above,
an alkenyloxycarbonyl group in which the alkenyl part has from 2 to 6 carbon atoms and is unsubstituted or is substituted by at least one halogen atom,
an alkoxycarbonyl group which has from 2 to 7 carbon atoms,
a substituted silylalkoxycarbonyl group, in which the alkoxycarbonyl part and the substituted silyl part are as defined above,
an oxygen-containing heterocyclic group,
an alkoxyalkyl group in which the alkoxy and alkyl parts each have from 1 to 6 carbon atoms,
a substituted silylalkoxyalkyl group, in which the alkoxyalkyl part and the substituted silyl part are as defined above, or
an alkanoyl or haloalkanoyl group which has no more than 6 carbon atoms;
or
R² and R³ together represent a group of formula =C(CH₃)CH₂OR⁶.

6. A process according to any one of Claims 1 to 5, in which X represents a sulfur atom or a group of formula >CHR⁷, where R⁷ represents a hydrogen atom, an alkyl group having from 1 to 4 carbon atoms or an alkoxy group having from 1 to 4 carbon atoms.

7. A process according to any one of Claims 1 to 6, in which A represents
an alkyl group which has from 1 to 4 carbon atoms and which is unsubstituted or is substituted by 1 or 2 of substituents K^{a}, defined below,
an aryl group, as defined below,
an aralkyl group in which an alkyl group having from 1 to 3 carbon atoms is substituted by at least one aryl group as defined below,
an aromatic or non-aromatic heterocylic group which may be monocyclic or may consist of two or more rings attached to each other by fusion or by a spiro attachment and which has from 4 to 7 ring atoms, at least one of said atoms being a nitrogen and/or oxygen and/or sulfur hetero-atom, said group being unsubstituted or being substituted by at least one of substituents B^{a} on carbon atoms, by at least one of substituents B^{a1} on nitrogen hetero-atoms, and/or by at least one oxygen atom to form a sulfinyl or sulfonyl group on sulfur hetero-atoms, all as defined below; or
an alkyl group which has from 1 to 3 carbon atoms and which is substituted by at least one substituent selected from the heterocyclic groups defined above;
said aryl groups are aromatic carbocyclic groups having from 6 to 10 ring carbon atoms and are unsubstituted or are substituted by at least one of substituents C^{a}, defined below;
said substituents K^{a} are selected from hydroxy, protected hydroxy groups, amino groups, protected amino groups, groups of formula -C(=NR¹⁰)NR¹¹R¹², where
R¹⁰, R¹¹and R¹² are the same or different and each represents a hydrogen atom, an amino-protecting group or an alkyl group having from 1 to 4 carbon atoms, or R¹¹ and R¹² together represent a group of formula -(CH₂)ₙ-, where n is an integer from 2 to 6, or R¹⁰ and R¹¹ together represent a group of formula -(CH₂)ₚ-, where p is 2 or 3, and
groups of formula -NR¹³C(=NR¹⁴)R¹⁵, where
R¹³ and R¹⁴ are the same or different and each represents a hydrogen atom, an amino-protecting group or an alkyl group having from 1 to 6 carbon atoms, and
R¹⁵ represents a hydrogen atom, an alkyl group having from 1 to 4 carbon atoms, an amino or a protected amino group,
or
any two of R¹³, R¹⁴ and R¹⁵ together represent a group of formula -(CH₂)ₚ-, where p is 2 or 3;
said substituents B^{a} are selected from
alkyl groups having from 1 to 4 carbon atoms,
alkoxy groups having from 1 to 6 carbon atoms,
hydroxy,
halogen atoms,
cyano,
nitro,
alkoxycarbonyl groups having from 2 to 5 carbon atoms,
carboxy,
oxygen atoms, to form with a ring carbon atom a carbonyl group,
alkoxyalkyl groups in which the alkoxy and alkyl parts both have from 1 to 6 carbon atoms,
haloalkyl groups having from 1 to 6 carbon atoms,
alkanoyloxy groups having from 1 to 6 carbon atoms,
groups of formula -NR⁸R⁹ and groups of formula -CONR⁸R⁹, where R⁸ and R⁹ are the same or different and each represents a hydrogen atom, an amino-protecting group, an alkyl group having from 1 to 4 carbon atoms or a phenyl group, or R⁸ and R⁹ together represent a group of formula -(CH₂)_{q}-Oᵣ-(CH₂)ₛ-, where q and s are the
same or different and each represents 0 or an integer of from 1 to 5 and r is 0 or 1, provided that (q+s) is an integer of at least 2; and
groups of formulae (B-I), (B-II), (B-III), (B-IV), (B-V), (B-VI), (B-VII), (B-VIII) and (B-IX);
said substituents B^{a1} are selected from alkyl groups having from 1 to 4 carbon atoms, amino-protecting groups, groups of formula -C(=NR¹⁶)R¹⁷, where R¹⁶ and R¹⁷ are as defined above, amino-protecting groups and groups of formula -CONR¹⁸R¹⁹, where
R¹⁸ and R¹⁹ are the same or different and each represents an alkyl group having from 1 to 4 carbon atoms, an alkanoyl group having from 2 to 5 carbon atoms or a benzoyl groups;
said substituents C^{a} are selected from alkyl groups having from 1 to 4 carbon atoms, alkoxy groups having from 1 to 4 carbon atoms, halogen atoms, alkoxycarbonyl groups having from 2 to 5 carbon atoms, groups of formula -CONR⁸R⁹, where R⁸ and R⁹ are as defined above, cyano, hydroxy and nitro.

8. A process according to any one of Claims 1 to 3, in which:
R¹ represents:
a hydrogen atom,
an alkyl group having from 1 to 4 carbon atoms,
an alkenyl group having from 2 to 4 carbon atoms,
an aralkyl group which is an alkyl group having 1 or
2 carbon atoms and which is substituted by at least one phenyl group
a haloalkyl group having from 1 to 4 carbon atoms,
a tri-substituted silylethyl group, in which all three or two of the substituents are alkyl groups having from 1 to 4 carbon atoms, and none or one of the substituents are phenyl groups,
an acyloxyalkyl group, in which the acyl part is an alkanoyl group having from 2 to 5 carbon atoms and the alkyl part has 1 or 2 carbon atoms,
an alkoxycarbonyloxyalkyl group in which the alkoxy and alkyl parts each have from 1 to 4 carbon atoms,
a (5-methyl-2-oxo-1,3-dioxolen-4-yl)methyl group, or
a 3-phthalidyl group.

9. A process according to any one of Claims 1 to 3 and 8, in which:
R² and R³ are the same or different and each represents a hydrogen atom, an alkyl group having from 1 to 4 carbon atoms or a group of formula 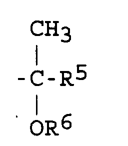
where R⁵ represents a hydrogen atom or a methyl group; and R⁶ represents:
a hydrogen atom,
a substituted silyl group, in which the silyl group has 3 substituents selected from alkyl groups having from 1 to 4 carbon atoms and phenyl groups,
an aralkyl group, in which an alkyl group having from 1 to 4 carbon atoms is substituted by at least one aryl group, as defined in Claim 7,
an alkoxycarbonyl group which has from 2 to 7 carbon atoms,
a tetrahydropyranyl group,
an alkoxyalkyl group in which the alkoxy and alkyl parts each have from 1 to 4 carbon atoms,
an alkanoyl or haloalkanoyl group which has from 2 to 5 carbon atoms;
or
R² and R³ together represent a group of formula =C(CH₃)CH₂OR⁶.

10. A process according to any one of Claims 1 to 3, 8 and 9, in which X represents a sulfur atom or a group of formula >CHR⁷, where R⁷ represents a hydrogen atom, an alkyl group having 1 or 2 carbon atoms or an alkoxy group having 1 or 2 carbon atoms.

11. A process according to any one of Claims 1 to 3, 8, 9 and 10 in which A represents
an alkyl group which has 1 or 2 carbon atoms and which is unsubstituted or is substituted by 1 or 2 of substituents K^{b}, defined below,
a phenyl group, which is unsubstituted or is substituted by 1 or 2 of substituents C^{b}, defined below,
an aralkyl group in which an alkyl group having from 1 to 3 carbon atoms is substituted by at least one phenyl group, which is unsubstituted or is substituted by 1 or 2 of substituents C^{b}, defined below,
an aromatic or non-aromatic heterocylic group which may be monocyclic or may consist of two or more rings attached to each other by fusion or by a spiro attachment and which has 5 or 6 ring atoms, from 1 to 3 of said atoms being a nitrogen and/or oxygen and/or sulfur hetero-atom, said group being unsubstituted or being substituted by at least one of substituents B^{b} on carbon atoms, by at least one of substituents B^{b1} on nitrogen hetero-atoms, and/or by at least one oxygen atom to form a sulfinyl or sulfonyl group on sulfur hetero-atoms, all as defined below;
said substituents K^{b} are selected from hydroxy, protected hydroxy groups, amino, protected amino groups and groups of formula -C(=NR¹⁰)NR¹¹R¹², where
R¹⁰, R¹¹ and R¹² are the same or different and each represents a hydrogen atom, an amino-protecting group or an alkyl group having 1 or 2 carbon atoms;
said substituents B^{b} are selected from
alkyl groups having 1 or 2 carbon atoms,
alkoxy groups having 1 or 2 carbon atoms,
hydroxy,
halogen atoms,
cyano,
nitro,
alkoxycarbonyl groups having from 2 to 5 carbon atoms,
carboxy,
haloalkyl groups having from 1 to 6 carbon atoms,
alkanoyloxy groups having from 1 to 6 carbon atoms,
groups of formula -NR⁸R⁹ and groups of formula -CONR⁸R⁹, where R⁸ and R⁹ are the same or different and each represents a hydrogen atom, an amino-protecting group, an alkyl group having 1 or 2 carbon atoms or a phenyl group, or R⁸ and R⁹ together represent a group of formula -(CH₂)_{q}-Oᵣ-(CH₂)ₛ-, where q and s are the same or different and each represents 0 or an integer of from 1 to 5 and r is 0 or 1, provided that (q+s) is an integer of at least 2; and
groups of formulae (B-I), (B-II), (B-III), (B-IV), (B-V), (B-VI), (B-VII), (B-VIII) and (B-IX);
said substituents B^{b1} are selected from alkyl groups having 1 or 2 carbon atoms, amino-protecting groups, groups of formula -C(=NR¹⁶)R¹⁷, where R¹⁶ and
R¹⁷ are independently selected from hydrogen atoms, methyl groups, amino-protecting groups and groups of formula -CONR¹⁸R¹⁹, where
R¹⁸ and R¹⁹ are the same or different and each represents an alkyl group having 1 or 2 carbon atoms, an alkanoyl group having from 2 to 5 carbon atoms or a benzoyl group;
said substituents C^{b} are selected from alkyl groups having 1 or 2 carbon atoms, alkoxy groups having 1 or 2 carbon atoms, halogen atoms, groups of formula -CONR⁸R⁹, cyano, hydroxy and nitro.

## Patentansprüche

1. Verfahren zur Herstellung einer Verbindung der Formel (I): 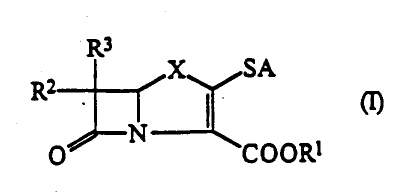 worin:
R¹ ein Wasserstoffatom oder eine Schutzgruppe für Carboxygruppen darstellt;
R² und R³ gleich oder unterschiedlich sind und jeweils ein Wasserstoffatom, eine Alkylgruppe mit 1 bis 6 Kohlenstoffatomen oder eine Gruppe der Formel 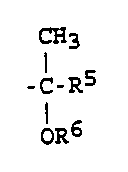 darstellen,
worin R⁵ ein Wasserstoffatom oder eine Methylgruppe darstellt und R⁶ ein Wasserstoffatom oder eine Schutzgruppe für Hydroxygruppen darstellt;
oder
R² und R³ zusammen eine Gruppe der Formel =C(CH₃)CH₂OR⁶ darstellen, worin R⁶ wie vorstehend definiert ist;
X ein Schwefelatom oder eine Gruppe der Formel >CHR⁷ darstellt, worin R⁷ ein Wasserstoffatom, eine Alkylgruppe mit 1 bis 6 Kohlenstoffatomen oder eine Alkoxygruppe mit 1 bis 6 Kohlenstoffatomen darstellt; und
A darstellt:
eine Alkylgruppe, die 1 bis 6 Kohlenstoffatome hat und unsubstituiert oder mit 1 oder 2 der nachstehend definierten Substituenten K substituiert ist,
eine nachstehend definierte Arylgruppe,
eine Aralkylgruppe, worin die Alkylgruppe mit 1 bis 6 Kohlenstoffatomen mit mindestens einer nachstehend definierten Arylgruppe substituiert ist,
eine aromatische oder nicht-aromatische heterocyclische Gruppe, die monocyclisch sein kann oder aus zwei oder mehr Ringen bestehen kann, die durch Kondensation oder durch eine Spiroverknüpfung aneinander gebunden sind, und die 3 bis 10 Ringatome hat, wobei mindestens eines der Atome ein Stickstoff- und/oder Sauerstoff- und/oder Schwefelheteroatom ist, die Gruppe unsubstituiert oder mit mindestens einem Substituenten B an Kohlenstoffatomen, mit mindestens einem der Substituenten B¹ an Stickstoffheteroatomen und/oder mit mindestens einem Sauerstoffatom unter Bildung einer Sulfinyl- oder Sulfonylgruppe an Schwefelheteroatomen substituiert ist, wobei die nachstehenden Definitionen gelten,
eine kondensierte heterocyclische Gruppe, worin eine vorstehend definierte heterocyclische Gruppe an eine nachstehend definierte Arylgruppe ankondensiert ist, oder
eine Alkylgruppe, die 1 bis 6 Kohlenstoffatome hat und mit mindestens einer vorstehend definierten heterocyclischen Gruppe und/oder kondensierten heterocyclischen Gruppe substituiert ist;
wobei die Arylgruppen aromatische carbocyclische Gruppen mit 6 bis 14 Ringkohlenstoffatomen sind und unsubstituiert oder mit mindestens einem der nachstehend definierten Substituenten C substituiert sind;
wobei die Substituenten K unter der Hydroxygruppe, geschützten Hydroxygruppen, der Aminogruppe, geschützten Aminogruppen, Gruppen der Formel -C(=NR¹⁰)NR¹¹R¹²,
worin R¹⁰, R¹¹ und R¹² gleich oder unterschiedlich sind und jeweils ein Wasserstoffatom, eine Schutzgruppe für Aminogruppen oder eine Alkylgruppe mit 1 bis 6 Kohlenstoffatomen darstellen, oder R¹¹ und R¹² zusammen eine Gruppe der Formel -(CH₂)ₙ-, worin n eine ganze Zahl von 2 bis 6 ist, oder R¹⁰ und R¹¹ zusammen eine Gruppe der Formel -(CH₂)ₚ- darstellen, worin p 2 oder 3 ist, und
Gruppen der Formel -NR¹³C(=NR¹⁴)R¹⁵ ausgewählt sind,
worin R¹³ und R¹⁴ gleich oder unterschiedlich sind und jeweils ein Wasserstoffatom, eine Schutzgruppe für Aminogruppen oder eine Alkylgruppe mit 1 bis 6 Kohlenstoffatomen darstellen und
R¹⁵ ein Wasserstoffatom, eine Alkylgruppe mit 1 bis 6 Kohlenstoffatomen, eine Aminogruppe oder eine geschützte Aminogruppe darstellt,
oder
beliebige zwei der Gruppen R¹³, R¹⁴ und R¹⁵ zusammen eine Gruppe der Formel -(CH₂)ₚ- darstellen, worin p 2 oder 3 ist;
wobei die Substituenten B unter
Alkylgruppen mit 1 bis 6 Kohlenstoffatomen,
Alkoxygruppen mit 1 bis 6 Kohlenstoffatomen,
der Hydroxygruppe,
Halogenatomen,
der Cyangruppe,
der Nitrogruppe,
Alkoxycarbonylgruppen mit 2 bis 7 Kohlenstoffatomen,
der Carboxygruppe,
Sauerstoffatomen unter Bildung einer Carbonylgruppe mit einem Ringkohlenstoffatom,
Cycloalkylgruppen mit 3 bis 7 Ringkohlenstoffatomen,
Alkoxyalkylgruppen, worin der Alkoxyteil und der Alkylteil jeweils 1 bis 6 Kohlenstoffatome haben,
Alkoxycarbonylalkylgruppen, worin der Alkoxyteil und der Alkylteil jeweils 1 bis 6 Kohlenstoffatome hat,
Cyanalkylgruppen, worin der Alkylteil 1 bis 6 Kohlenstoffatome hat,
Halogenalkylgruppen mit 1 bis 6 Kohlenstoffatomen,
Alkanoyloxygruppen mit 1 bis 6 Kohlenstoffatomen,
der Azidogruppe,
Alkylthiogruppen mit 1 bis 6 Kohlenstoffatomen,
Alkylsulfinylgruppen mit 1 bis 6 Kohlenstoffatomen,
Alkylsulfonylgruppen mit 1 bis 6 Kohlenstoffatomen,
Gruppen der Formel -NR⁸R⁹ und Gruppen der Formel -CONR⁸R⁹, worin R⁸ und R⁹ gleich oder unterschiedlich sind und jeweils ein Wasserstoffatom, eine Schutzgruppe für Aminogruppen, eine Alkylgruppe mit 1 bis 6 Kohlenstoffatomen oder eine Phenylgruppe darstellen, oder R⁸ und R⁹ zusammen eine Gruppe der Formel -(CH₂)_{q}-Oᵣ-(CH₂)ₛ darstellen, worin q und s gleich oder unterschiedlich sind und jeweils 0 oder eine ganze Zahl von 1 bis 5 darstellen und r 0 oder 1 ist, mit der Maßgabe, daß (q+s) eine ganze Zahl von mindestens 2 ist und
Gruppen der Formeln (B-I), (B-II), (B-III), (B-IV), (B-V), (B-VI), (B-VII), (B-VIII) und (B-IX) ausgewählt sind: 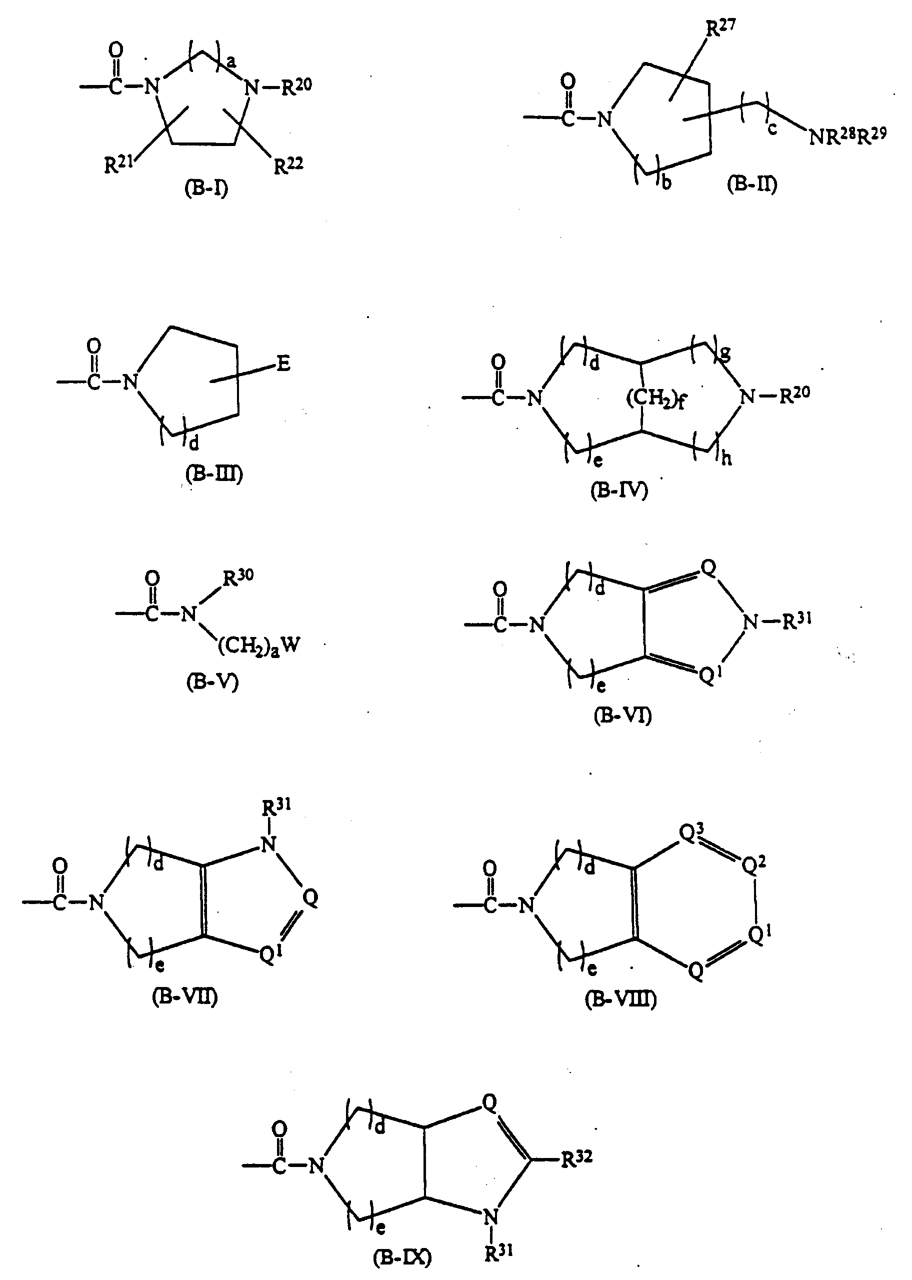 worin:
a 1, 2 oder 3 ist,
b 0, 1 oder 2 ist,
c 0 oder 1 ist,
d 0, 1 oder 2 ist,
e 0, 1 oder 2 ist,
f 0, 1 oder 2 ist,
g 0, 1 oder 2 ist,
h 0, 1 oder 2 ist,
R²⁰ ein Wasserstoffatom, eine Schutzgruppe für Aminogruppen, eine Gruppe der Formel -COR²⁶,
worin R²⁶ eine Alkylgruppe mit 1 bis 6 Kohlenstoffatomen oder eine substituierte Alkylgruppe darstellt, die 1 bis 6 Kohlenstoffatome hat und mit mindestens einem der Substituenten K substituiert ist,
eine Gruppe der Formel -C(=NR¹⁶)R¹⁷,
worin R¹⁶ und R¹⁷ gleich oder unterschiedlich sind und jeweils ein Wasserstoffatom, eine Alkylgruppe mit 1 bis 6 Kohlenstoffatomen, eine Aminogruppe, eine geschützte Aminogruppe oder eine Schutzgruppe für Aminogruppen darstellen;
eine unsubstituierte Alkylgruppe mit 1 bis 6 Kohlenstoffatomen oder eine substituierte Alkylgruppe darstellt, die 1 bis 6 Kohlenstoffatome hat und mit mindestens einem der nachstehend definierten Substituenten D substituiert ist;
R²¹ und R²² gleich oder unterschiedlich sind und jeweils ein Wasserstoffatom, eine Alkylgruppe mit 1 bis 6 Kohlenstoffatomen, die Hydroxygruppe oder eine geschützte Hydroxygruppe darstellen;
R²⁷ ein Wasserstoffatom oder eine Alkylgruppe mit 1 bis 6 Kohlenstoffatomen darstellt;
R²⁸ und R²⁹ gleich oder unterschiedlich sind und jeweils ein Wasserstoffatom, eine Alkylgruppe mit 1 bis 6 Kohlenstoffatomen, eine Schutzgruppe für Aminogruppen, eine Aminogruppe, eine geschützte Aminogruppe oder eine Gruppe der Formel -C(=NR¹⁶)R¹⁷ darstellen, worin R¹⁶ und R¹⁷ wie vorstehend definiert sind;
R³⁰ ein Wasserstoffatom, eine Alkylgruppe mit 1 bis 6 Kohlenstoffatomen oder eine substituierte Alkylgruppe darstellt, die 1 bis 6 Kohlenstoffatome hat und mit mindestens einem Substituenten substituiert ist, der unter der Hydroxygruppe und geschützten Hydroxygruppen ausgewählt ist;
R³¹ ein Wasserstoffatom, eine Alkylgruppe mit 1 bis 6 Kohlenstoffatomen oder eine Schutzgruppe für Aminogruppen darstellt;
R³² ein Wasserstoffatom, eine Alkylgruppe mit 1 bis 6 Kohlenstoffatomen oder eine Gruppe der Formel -NR²⁸R²⁹ darstellt, worin R²⁸ und R²⁹ wie vorstehend definiert sind;
E eine Imidazolylgruppe oder eine Triazolylgruppe darstellt;
W eine aromatische heterocyclische Gruppe mit 5 bis 8 Ringatomen darstellt, von denen 1 bis 4 Stickstoffatome sind, wobei die aromatische heterocyclische Gruppe unsubstituiert oder mit mindestens einer Alkylgruppe mit 1 bis 6 Kohlenstoffatomen substituiert ist;
Q, Q¹, Q² und Q³ unabhängig voneinander aus der Gruppe ausgewählt sind, die aus Gruppen der Formel 〉̶CH und 〉̶N besteht;
wobei die Substituenten B¹ unter Alkylgruppen mit 1 bis 6 Kohlenstoffatomen, Schutzgruppen für Aminogruppen, Gruppen der Formel -C(=NR¹⁶)R¹⁷, worin R¹⁶ und R¹⁷ wie vorstehend definiert sind, Schutzgruppen für Aminogruppen und Gruppen der Formel -CONR¹⁸R¹⁹ ausgewählt sind,
worin R¹⁸ und R¹⁹ gleich oder unterschiedlich sind und jeweils eine Alkylgruppe mit 1 bis 6 Kohlenstoffatomen oder eine Acylgruppe einer Carbonsäure darstellen;
wobei die Substituenten C unter Alkylgruppen mit 1 bis 6 Kohlenstoffatomen, Alkoxygruppen mit 1 bis 6 Kohlenstoffatomen, Halogenatomen, Alkoxycarbonylgruppen mit 2 bis 7 Kohlenstoffatomen, Gruppen der Formel -CONR⁸R⁹, worin R⁸ und R⁹ wie vorstehend definiert sind, der Cyangruppe, der Hydroxygruppe und der Nitrogruppe ausgewählt sind;
wobei die Substituenten D unter der Hydroxygruppe, geschützten Hydroxygruppen, der Carboxygruppe, geschützten Carboxygruppen, der Cyangruppe, Alkoxygruppen mit 1 bis 6 Kohlenstoffatomen, Alkylsulfonylgruppen mit 1 bis 6 Kohlenstoffatomen, Gruppen der Formel -NHCOR²³, -NR²⁴R²⁵, -CONR²⁴R²⁵ oder -OCONR²⁴R²⁵ ausgewählt sind,
worin R²³, R²⁴ und R²⁵ gleich oder unterschiedlich sind und jeweils ein Wasserstoffatom oder eine Alkylgruppe mit 1 bis 6 Kohlenstoffatomen darstellen;
oder eines pharmazeutisch geeigneten Salzes oder pharmazeutisch geeigneten Esters davon, wobei das Verfahren das Umsetzen einer Verbindung der Formel (II) 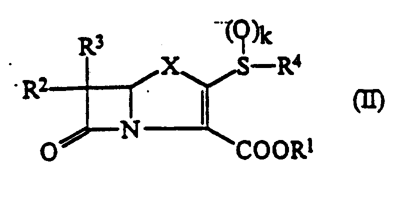
worin R¹, R², R³ und X wie vorstehend definiert sind, k 1 oder 2 ist und R⁴ darstellt:
eine Alkylgruppe mit 1 bis 6 Kohlenstoffatomen, die unsubstituiert oder mit mindestens einem der nachstehend definierten Substituenten E substituiert ist,
eine Alkenylgruppe mit 2 bis 6 Kohlenstoffatomen, die unsubstituiert oder mit mindestens einem der nachstehend definierten Substituenten E substituiert ist,
eine vorstehend definierte Arylgruppe,
eine Aralkylgruppe, worin die Alkylgruppe mit 1 bis 6 Kohlenstoffatomen mit mindestens einer vorstehend definierten Arylgruppe substituiert ist,
eine Cycloalkylgruppe mit 3 bis 7 Kohlenstoffatomen, die unsubstituiert oder mit mindestens einem der vorstehend definierten Substituenten C substituiert ist,
eine aromatische heterocyclische Gruppe mit 5 bis 7 Ringatomen in einem aromatischen Ring, wobei mindestens eines der Atome ein Stickstoff- und/oder Sauerstoff- und/oder Schwefelheteroatom ist, wobei die Gruppe unsubstituiert oder mit mindestens einem der vorstehend definierten Substituenten C substituiert ist,
eine kondensierte heterocyclische Gruppe, worin eine vorstehend definierte aromatische heterocyclische Gruppe an eine vorstehend definierte Arylgruppe kondensiert ist, oder
eine Alkylgruppe mit 1 bis 6 Kohlenstoffatomen, die mit mindestens einer vorstehend definierten aromatischen heterocyclischen Gruppe und/oder kondensierten heterocyclischen Gruppe substituiert ist;
wobei die Substituenten E unter der Hydroxygruppe, geschützten Hydroxygruppen, der Aminogruppe und geschützten Aminogruppen ausgewählt sind;
mit einer Verbindung der Formel (III):
ASH (III)
worin A wie vorstehend definiert ist;
und, falls gewünscht, das Entfernen beliebiger Schutzgruppen umfaßt, dadurch gekennzeichnet, daß die Reaktion der Verbindung der Formel (II) mit der Verbindung der Formel (III) in Anwesenheit eines Magnesium- oder Aluminiumsalzes durchgeführt wird, das aus der folgenden Gruppe ausgewählt ist:
ein Magnesiumbromid-Diethylether-Komplex; ein Gemisch von Magnesiumbromid-Diethylether-Komplex mit Diisopropylethylamin, 1-Ethylpiperidin oder 1,5-Diazabicyclo[5.4.0]-5-undecen; Brommagnesiumcyclohexylisopropylamid, Brommagnesiumdiisopropylamid, Brommagnesiumdiethylamid oder Brommagnesiumhexamethyldisilazid; Brommagnesium-t-butoxid; Gemische von Lithiumdiisopropylamid oder Lithiumbistrimethylsilylamid mit einem Magnesiumbromid-Diethylether-Komplex oder Diethylaluminiumdiisopropylamid;
wobei die Verbindung der Formel (II) und das Mercaptan der Formel (III) in einem Molverhältnis von 1:1 bis 1:3 eingesetzt wird und das Magnesium- oder Aluminiumsalz in einer Menge von 1 bis 20 Mol pro Mol der Verbindung der Formel (II) vorhanden ist.

2. Verfahren nach Anspruch 1, wobei das Salz ein Aluminiumsalz ist.

3. Verfahren nach Anspruch 1, wobei das Salz ein Magnesiumsalz ist.

4. Verfahren nach einem der Ansprüche 1 bis 3, wobei
R¹ darstellt:
ein Wasserstoffatom,
eine Alkylgruppe mit 1 bis 6 Kohlenstoffatomen,
eine Aralkylgruppe, die eine Alkylgruppe mit 1 bis 4 Kohlenstoffatomen ist, die mit mindestens einer Arylgruppe substituiert ist,
eine Alkenylgruppe mit 2 bis 6 Kohlenstoffatomen, die unsubstituiert oder mit einem oder mehreren Halogenatomen substituiert ist,
eine Halogenalkylgruppe mit 1 bis 6 Kohlenstoffatomen,
eine trisubstituierte Silylethylgruppe, worin alle drei oder zwei oder einer der Substituenten eine Alkylgruppe bzw. Alkylgruppen mit 1 bis 5 Kohlenstoffatomen ist bzw. sind und keiner, einer oder zwei der Substituenten eine Arylgruppe bzw. Arylgruppen ist bzw. sind,
eine Acyloxyalkylgruppe, worin der Acylteil eine Alkanoylgruppe mit 1 bis 6 Kohlenstoffatomen ist und der Alkylteil 1 bis 6 Kohlenstoffatome hat,
eine Alkoxycarbonyloxyalkylgruppe, worin der Alkoxyteil und der Alkylteil jeweils 1 bis 6 Kohlenstoffatome haben,
eine Cycloalkoxycarbonyloxyalkylgruppe, worin der Cycloalkylteil 3 bis 7 Ringkohlenstoffatome hat und unsubstituiert oder mit mindestens einem der Substituenten C substituiert ist und der Alkylteil 1 bis 6 Kohlenstoffatome hat,
eine (5-Alkyl- oder 5-Aryl-2-oxo-1,3-dioxolen-4-yl)methylgruppe, worin der Alkylteil 1 bis 4 Kohlenstoffatome hat, oder
die 3-Phthalidylgruppe.

5. Verfahren nach einem der Ansprüche 1 bis 4, wobei
R² und R³ gleich oder unterschiedlich sind und jeweils ein Wasserstoffatom, eine Alkylgruppe mit 1 bis 4 Kohlenstoffatomen oder eine Gruppe der Formel 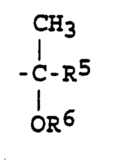 darstellen,
worin R⁵ ein Wasserstoffatom oder eine Methylgruppe darstellt und R⁶ darstellt:
ein Wasserstoffatom,
eine substituierte Silylgruppe, worin die Silylgruppe 3 Substituenten hat, die unter Alkylgruppen mit 1 bis 6 Kohlenstoffatomen und Phenylgruppen ausgewählt sind,
eine Aralkylgruppe, worin die Alkylgruppe mit 1 bis 4 Kohlenstoffatomen mit mindestens einer Arylgruppe substituiert ist,
eine Aralkyloxycarbonylgruppe, worin der Aralkylteil wie vorstehend definiert ist,
eine Alkenyloxycarbonylgruppe, worin der Alkenylteil 2 bis 6 Kohlenstoffatome hat und unsubstituiert oder mit mindestens einem Halogenatom substituiert ist,
eine Alkoxycarbonylgruppe mit 2 bis 7 Kohlenstoffatomen,
eine substituierte Silylalkoxycarbonylgruppe, worin der Alkoxycarbonylteil und der substituierte Silylteil wie vorstehend definiert sind,
eine Sauerstoff enthaltende heterocyclische Gruppe,
eine Alkoxyalkylgruppe, worin der Alkoxyteil und der Alkylteil jeweils 1 bis 6 Kohlenstoffatome haben,
eine substituierte Silylalkoxyalkylgruppe, worin der Alkoxyalkylteil und der substituierte Silylteil wie vorstehend definiert sind, oder
eine Alkanoyl- oder Halogenalkanoylgruppe, die nicht mehr als 6 Kohlenstoffatome hat;
oder
R² und R³ zusammen eine Gruppe der Formel =C(CH₃)CH₂OR⁶ darstellen.

6. Verfahren nach einem der Ansprüche 1 bis 5, wobei X ein Schwefelatom oder eine Gruppe der Formel >CHR⁷ darstellt, worin R⁷ ein Wasserstoffatom, eine Alkylgruppe mit 1 bis 4 Kohlenstoffatomen oder eine Alkoxygruppe mit 1 bis 4 Kohlenstoffatomen darstellt.

7. Verfahren nach einem der Ansprüche 1 bis 6 , wobei A darstellt:
eine Alkylgruppe mit 1 bis 4 Kohlenstoffatomen, die unsubstituiert oder mit 1 oder 2 der nachstehend definierten Substituenten K^{a} substituiert ist,
eine nachstehend definierte Arylgruppe,
eine Aralkylgruppe, worin die Alkylgruppe mit 1 bis 3 Kohlenstoffatomen mit mindestens einer nachstehend definierten Arylgruppe substituiert ist,
eine aromatische oder nicht-aromatische heterocyclische Gruppe, die monocyclisch sein oder aus zwei oder mehr Ringen bestehen kann, die durch Kondensation oder durch eine Spiroverknüpfung aneinander gebunden sind, und die 4 bis 7 Ringatome hat, wobei mindestens eines der Atome ein Stickstoff- und/oder Sauerstoff- und/oder Schwefelheteroatom ist, wobei die Gruppe unsubstituiert oder mit mindestens einem der Substituenten B^{a} an Kohlenstoffatomen, mit mindestens einem der Substituenten B^{a1} an Stickstoffheteroatomen und/oder mit mindestens einem Sauerstoffatom unter Bildung einer Sulfinyl- oder Sulfonylgruppe an Schwefelheteroatomen substituiert ist, wobei die nachstehenden Definitionen gelten, oder
eine Alkylgruppe, die 1 bis 3 Kohlenstoffatome hat und mit mindestens einem der unter den vorstehend definierten heterocyclischen Gruppen ausgewählten Substituenten substituiert ist;
wobei die Arylgruppen aromatische carbocyclische Gruppen mit 6 bis 10 Ringkohlenstoffatomen sind und unsubstituiert oder mit mindestens einem der nachstehend definierten Substituenten C^{a} substituiert sind,
wobei die Substituenten K^{a} unter der Hydroxygruppe, geschützten Hydroxygruppen, Aminogruppen, geschützten Aminogruppen, Gruppen der Formel -C(=NR¹⁰)NR¹¹R¹²,
worin R¹⁰, R¹¹ und R¹² gleich oder unterschiedlich sind und jeweils ein Wasserstoffatom, eine Schutzgruppe für Aminogruppen oder eine Alkylgruppe mit 1 bis 4 Kohlenstoffatomen darstellen, oder R¹¹ und R¹² zusammen eine Gruppe der Formel -(CH₂)ₙ- darstellen, worin n eine ganze Zahl von 2 bis 6 ist, oder R¹⁰ und R¹¹ zusammen eine Gruppe der Formel -(CH₂)ₚ- darstellen, worin p 2 oder 3 ist, und
Gruppen der Formel -NR¹³C(=NR¹⁴)R¹⁵ ausgewählt sind,
worin R¹³ und R¹⁴ gleich oder unterschiedlich sind und jeweils ein Wasserstoffatom, eine Schutzgruppe für Aminogruppen oder eine Alkylgruppe mit 1 bis 6 Kohlenstoffatomen darstellen, und
R¹⁵ ein Wasserstoffatom, eine Alkylgruppe mit 1 bis 4 Kohlenstoffatomen, eine Aminogruppe oder eine geschützte Aminogruppe darstellt,
oder
beliebige zwei der Gruppen R¹³, R¹⁴ und R¹⁵ zusammen eine Gruppe der Formel -(CH₂)ₚ- darstellen, worin p 2 oder 3 ist;
wobei die Substituenten B^{a} ausgewählt sind unter
Alkylgruppen mit 1 bis 4 Kohlenstoffatomen,
Alkoxygruppen mit 1 bis 6 Kohlenstoffatomen,
der Hydroxygruppe,
Halogenatomen,
der Cyangruppe,
der Nitrogruppe,
Alkoxycarbonylgruppen mit 2 bis 5 Kohlenstoffatomen,
der Carboxygruppe,
Sauerstoffatomen unter Bildung einer Carbonylgruppe mit einem Ringkohlenstoffatom,
Alkoxyalkylgruppen, worin der Alkoxyteil und der Alkylteil jeweils 1 bis 6 Kohlenstoffatome haben,
Halogenalkylgruppen mit 1 bis 6 Kohlenstoffatomen,
Alkanoyloxygruppen mit 1 bis 6 Kohlenstoffatomen,
Gruppen der Formel -NR⁸R⁹ und Gruppen der Formel -CONR⁸R⁹, worin R⁸ und R⁹ gleich oder unterschiedlich sind und jeweils ein Wasserstoffatom, eine Schutzgruppe für Aminogruppen, eine Alkylgruppe mit 1 bis 4 Kohlenstoffatomen oder eine Phenylgruppe darstellen, oder R⁸ und R⁹ zusammen eine Gruppe der Formel -(CH₂)_{q}-Oᵣ-(CH₂)ₛ- darstellen, worin q und s gleich oder unterschiedlich sind und jeweils 0 oder eine ganze Zahl von 1 bis 5 darstellen und r 0 oder 1 ist, mit der Maßgabe, daß (q+s) eine ganze Zahl von mindestens 2 ist; und
Gruppen der Formeln (B-I), (B-II), (B-III), (B-IV), (B-V), (B-VI), (B-VII), (B-VIII) und (B-IX);
wobei die Substituenten B^{a1} unter Alkylgruppen mit 1 bis 4 Kohlenstoffatomen, Schutzgruppen für Aminogruppen, Gruppen der Formel -C(=NR¹⁶)R¹⁷, worin R¹⁶ und R¹⁷ wie vorstehend definiert sind, und Gruppen der Formel -CONR¹⁸R¹⁹ ausgewählt sind,
worin R¹⁸ und R¹⁹ gleich oder unterschiedlich sind und jeweils eine Alkylgruppe mit 1 bis 4 Kohlenstoffatomen, eine Alkanoylgruppe mit 2 bis 5 Kohlenstoffatomen oder eine Benzoylgruppe darstellen;
wobei die Substituenten C^{a} unter Alkylgruppen mit 1 bis 4 Kohlenstoffatomen, Alkoxygruppen mit 1 bis 4 Kohlenstoffatomen, Halogenatomen, Alkoxycarbonylgruppen mit 2 bis 5 Kohlenstoffatomen, Gruppen der Formel -CONR⁸R⁹, worin R⁸ und R⁹ wie vorstehend definiert sind, der Cyangruppe, der Hydroxygruppe und der Nitrogruppe ausgewählt sind.

8. Verfahren nach einem der Ansprüche 1 bis 3, wobei
R¹ darstellt:
ein Wasserstoffatom,
eine Alkylgruppe mit 1 bis 4 Kohlenstoffatomen,
eine Alkenylgruppe mit 2 bis 4 Kohlenstoffatomen,
eine Aralkylgruppe, die eine Alkylgruppe mit 1 oder 2 Kohlenstoffatomen ist, die mit mindestens einer Phenylgruppe substituiert ist,
eine Halogenalkylgruppe mit 1 bis 4 Kohlenstoffatomen,
eine trisubstituierte Silylethylgruppe, worin alle drei oder zwei der Substituenten Alkylgruppen mit 1 bis 4 Kohlenstoffatomen sind und keiner oder einer der Substituenten eine Phenylgruppe ist,
eine Acyloxyalkylgruppe, worin der Acylteil eine Alkanoylgruppe mit 2 bis 5 Kohlenstoffatomen ist und der Alkylteil 1 oder 2 Kohlenstoffatome hat,
eine Alkoxycarbonyloxyalkylgruppe, worin der Alkoxyteil und der Alkylteil jeweils 1 bis 4 Kohlenstoffatome haben,
eine (5-Methyl-2-oxo-1,3-dioxolen-4-yl)methylgruppe, oder
eine 3-Phthalidylgruppe.

9. Verfahren nach einem der Ansprüche 1 bis 3 und 8, wobei
R² und R³ gleich oder unterschiedlich sind und jeweils ein Wasserstoffatom, eine Alkylgruppe mit 1 bis 4 Kohlenstoffatomen oder eine Gruppe der Formel 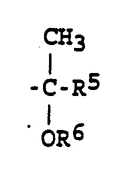 darstellen,
worin R⁵ ein Wasserstoffatom oder eine Methylgruppe darstellt und R⁶ darstellt:
ein Wasserstoffatom,
eine substituierte Silylgruppe, worin die Silylgruppe 3 Substituenten hat, die unter Alkylgruppen mit 1 bis 4 Kohlenstoffatomen und Phenylgruppen ausgewählt sind,
eine Aralkylgruppe, worin die Alkylgruppe mit 1 bis 4 Kohlenstoffatomen mit mindestens einer in Anspruch 7 definierten Arylgruppe substituiert ist,
eine Alkoxycarbonylgruppe mit 2 bis 7 Kohlenstoffatomen,
eine Tetrahydropyranylgruppe,
eine Alkoxyalkylgruppe, worin der Alkoxyteil und der Alkylteil jeweils 1 bis 4 Kohlenstoffatome haben,
eine Alkanoyl- oder Halogenalkanoylgruppe mit 2 bis 5 Kohlenstoffatomen;
oder
R² und R³ zusammen eine Gruppe der Formel =C(CH₃)CH₂OR⁶ darstellen.

10. Verfahren nach einem der Ansprüche 1 bis 3, 8 und 9, wobei X ein Schwefelatom oder eine Gruppe der Formel >CHR⁷ darstellt, worin R⁷ ein Wasserstoffatom, eine Alkylgruppe mit 1 oder 2 Kohlenstoffatomen oder eine Alkoxygruppe mit 1 oder 2 Kohlenstoffatomen darstellt.

11. Verfahren nach einem der Ansprüche 1 bis 3, 8, 9 und 10, wobei A darstellt:
eine Alkylgruppe mit 1 oder 2 Kohlenstoffatomen, die unsubstituiert oder mit 1 oder 2 der nachstehend definierten Substituenten K^{b} substituiert ist,
eine Phenylgruppe, die unsubstituiert oder mit 1 oder 2 der nachstehend definierten Substituenten C^{b} substituiert ist,
eine Aralkylgruppe, worin die Alkylgruppe mit 1 bis 3 Kohlenstoffatomen mit mindestens einer Phenylgruppe substituiert ist, die unsubstituiert oder mit 1 oder 2 der nachstehend definierten Substituenten C^{b} substituiert ist,
eine aromatische oder nicht-aromatische heterocyclische Gruppe, die monocyclisch sein oder aus zwei oder mehr Ringen bestehen kann, die durch Kondensation oder durch eine Spiroverknüpfung aneinander gebunden sind, und die 5 oder 6 Ringatome hat, wobei 1 bis 3 der Atome Stickstoff- und/oder Sauerstoff- und/oder Schwefelheteroatom sind, wobei die Gruppe unsubstituiert oder mit mindestens einem der Substituenten B^{b} an Kohlenstoffatomen, mit mindestens einem der Substituenten B^{b¹} an Stickstoffheteroatomen und/oder mit mindestens einem Sauerstoffatom unter Bildung einer Sulfinyl- oder Sulfonylgruppe an Schwefelheteroatomen substituiert ist, wobei die nachstehenden Definitionen gelten;
wobei die Substituenten K^{b} unter der Hydroxygruppe, geschützten Hydroxygruppen, der Aminogruppe, geschützten Aminogruppen und Gruppen der Formel -C(=NR¹⁰)NR¹¹R¹² ausgewählt sind,
worin R¹⁰, R¹¹ und R¹² gleich oder unterschiedlich sind und jeweils ein Wasserstoffatom, eine Schutzgruppe für
Aminogruppen oder eine Alkylgruppe mit 1 oder 2 Kohlenstoffatomen darstellen;
wobei die Substituenten B^{b} ausgewählt sind unter
Alkylgruppen mit 1 oder 2 Kohlenstoffatomen,
Alkoxygruppen mit 1 oder 2 Kohlenstoffatomen,
der Hydroxygruppe,
Halogenatomen,
der Cyangruppe,
der Nitrogruppe,
Alkoxycarbonylgruppen mit 2 bis 5 Kohlenstoffatomen,
der Carboxygruppe,
Halogenalkylgruppen mit 1 bis 6 Kohlenstoffatomen,
Alkanoyloxygruppen mit 1 bis 6 Kohlenstoffatomen,
Gruppen der Formel -NR⁸R⁹ und Gruppen der Formel -CONR⁸R⁹, worin R⁸ und R⁹ gleich oder unterschiedlich sind und jeweils ein Wasserstoffatom, eine Schutzgruppe für Aminogruppen, eine Alkylgruppe mit 1 oder 2 Kohlenstoffatomen oder eine Phenylgruppe darstellen, oder R⁸ und R⁹ zusammen eine Gruppe der Formel -(CH₂)_{q}-Oᵣ-(CH₂)ₛ- darstellen, worin q und s gleich oder unterschiedlich sind und jeweils 0 oder eine ganze Zahl von 1 bis 5 darstellen und r 0 oder 1 ist, mit der Maßgabe, daß (q+s) eine ganze Zahl von mindestens 2 ist, und
Gruppen der Formeln (B-I), (B-II), (B-III), (B-IV), (B-V), (B-VI), (B-VII), (B-VIII) und (B-IX);
wobei die Substituenten B^{b1} unter Alkylgruppen mit 1 oder 2 Kohlenstoffatomen, Schutzgruppen für Aminogruppen und Gruppen der Formel -C(=NR¹⁶)R¹⁷ ausgewählt sind, worin R¹⁶ und R¹⁷ unabhängig voneinander unter Wasserstoffatomen, Methylgruppen, Schutzgruppen für Aminogruppen und Gruppen der Formel -CONR¹⁸R¹⁹ ausgewählt sind,
worin R¹⁸ und R¹⁹ gleich oder unterschiedlich sind und jeweils eine Alkylgruppe mit 1 oder 2 Kohlenstoffatomen, eine Alkanoylgruppe mit 2 bis 5 Kohlenstoffatomen oder eine Benzoylgruppe darstellen;
wobei die Substituenten C^{b} unter Alkylgruppen mit 1 oder 2 Kohlenstoffatomen, Alkoxygruppen mit 1 oder 2 Kohlenstoffatomen, Halogenatomen, Gruppen der Formel -CONR⁸R⁹, der Cyangruppe, der Hydroxygruppe und der Nitrogruppe ausgewählt sind.

## Revendications

1. Procédé de préparation d'un composé de formule (I) : 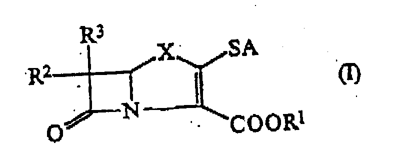 dans laquelle :
R¹ représente un atome d'hydrogène ou un groupe protecteur de carboxyle ;
R² et R³ sont identiques ou différents et représentent chacun un atome d'hydrogène, un groupe alkyle ayant 1 à 6 atomes de carbone ou un groupe de formule 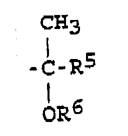
où R⁵ représente un atome d'hydrogène ou un groupe méthyle ; et R⁶ représente un atome d'hydrogène ou un groupe protecteur d'hydroxyle ;
ou bien
R² et R³ représentent ensemble un groupe de formule =C(CH₃)CH₂OR⁶ où R⁶ est tel que défini ci-dessus ;
X représente un atome de soufre ou un groupe de formule 〉CHR où R⁷ représente un atome d'hydrogène, un groupe alkyle ayant 1 à 6 atomes de carbone ou un groupe alcoxy ayant 1 à 6 atomes de carbone ; et
A représente
un groupe alkyle ayant 1 à 6 atomes de carbone et qui n'est pas substitué ou est substitué par 1 ou 2 des substituants K définis ci-dessous,
un groupe aryle tel que défini ci-dessous,
un groupe aralkyle dans lequel un groupe alkyle ayant 1 à 6 atomes de carbone est substitué par au moins un groupe aryle tel que défini ci-dessous,
un groupe hétérocyclique aromatique ou non aromatique qui peut être monocyclique ou peut consister en deux ou plusieurs cycles liés entre eux par condensation ou par une liaison spiro et qui compte 3 à 10 atomes endocycliques au moins l'un desdits atomes étant un hétéroatome d'azote et/ou d'oxygène et/ou de soufre, ledit groupe n'étant pas substitué ou étant substitué par au moins l'un des substituants B sur des atomes de carbone, par au moins l'un des substituants B¹ sur des hétéroatomes d'azote et/ou par au moins un atome d'oxygène pour former un groupe sulfinyle ou sulfonyle sur des hétéroatomes de soufre, tous étant tels que définis ci-dessous ;
un groupe hétérocyclique condensé dans lequel un groupe hétérocyclique tel que défini ci-dessus est condensé avec un groupe aryle tel que défini ci-dessous, ou
un groupe alkyle ayant 1 à 6 atomes de carbone et qui est substitué par au moins un groupe hétérocyclique et/ou un groupe hétérocyclique condensé tel que défini ci-dessus ;
lesdits groupes aryle sont des groupes carbocycliques aromatiques ayant 6 à 14 atomes de carbone cycliques et ne sont pas substitués ou sont substitués par au moins l'un des substituants C définis ci-dessous ;
lesdits substituants K sont choisis parmi les groupes hydroxyle, et hydroxyle protégés, les groupes amino et amino protégés, les groupes de formule -C(=NR¹⁰)NR¹¹R¹² où
R¹⁰, R¹¹ et R¹² sont identiques ou différents et représentent chacun un atome d'hydrogène, un groupe protecteur d'amine ou un groupe alkyle ayant 1 à 6 atomes de carbone, ou bien R¹¹ et R¹² représentent ensemble un groupe de formule -(CH₂)ₙ- où n est un nombre entier de 2 à 6, ou bien R¹⁰ et R¹¹ représentent ensemble un groupe de formule -(CH₂)ₚ-, où p est 2 ou 3, et
les groupes de formule -NR¹³C(=NR¹⁴)R¹⁵ où
R¹³ et R¹⁴ sont identiques ou différents et représentent chacun un atome d'hydrogène, un groupe protecteur d'amine ou un groupe alkyle ayant 1 à 6 atomes de carbone, et
R¹⁵ représente un atome d'hydrogène, un groupe alkyle ayant 1 à 6 atomes de carbone, un groupe amino ou amino protégé,
ou bien
deux quelconques de R¹³, R¹⁴ et R¹⁵ représentent ensemble un groupe de formule -(CH₂)ₚ- où p est 2 ou 3 ;
lesdits substituants B sont choisis parmi
les groupes alkyle ayant 1 à 6 atomes de carbone,
les groupes alcoxy ayant 1 à 6 atomes de carbone,
le groupe hydroxyle,
les atomes d'halogènes,
le groupe cyano,
le groupe nitro,
les groupes alcoxycarbonyle ayant 2 à 7 atomes de carbone,
le groupe carboxyle,
les atomes d'oxygène, pour former un groupe carbonyle avec un atome de carbone endocyclique,
les groupes cycloalkyle ayant 3 à 7 atomes de carbone cycliques,
les groupes alcoxyalkyle dans lesquels les portions alcoxy et alkyle ont toutes deux 1 à 6 atomes de carbone,
les groupes alcoxycarbonylalkyle dont les portions alcoxy et alkyle ont toutes deux 1 à 6 atomes de carbone,
les groupes cyanoalkyle dont la portion alkyle compte 1 à 6 atomes de carbone,
les groupes halogénoalkyle ayant 1 à 6 atomes de carbone,
les groupes alcanoyloxy ayant 1 à 6 atomes de carbone,
le groupe azido,
les groupes alkylthio ayant 1 à 6 atomes de carbone,
les groupes alkylsulfinyle ayant 1 à 6 atomes de carbone,
les groupes alkylsulfonyle ayant 1 à 6 atomes de carbone,
les groupes de formule -NR⁸R⁹ et les groupes de formule -CONR⁸R⁹ où R⁸ et R⁹ sont identiques ou différents et représentent chacun un atome d'hydrogène, un groupe protecteur d'amine, un groupe alkyle ayant 1 à 6 atomes de carbone ou un groupe phényle, ou bien R⁸ et R⁹ représentent ensemble un groupe de formule -(CH₂)_{q}-Oᵣ-(CH₂)ₛ-, où q et s sont identiques ou différents et représentent chacun 0 ou un nombre entier de 1 à 5, et r est 0 ou 1, à condition que (q + s) soit un nombre entier égal ou supérieur à 2 ; et
les groupes de formules (B-I), (B-II), (B-III), (B-IV), (B-V), (B-VI), (B-VII), (B-VIII) et (B-IX) 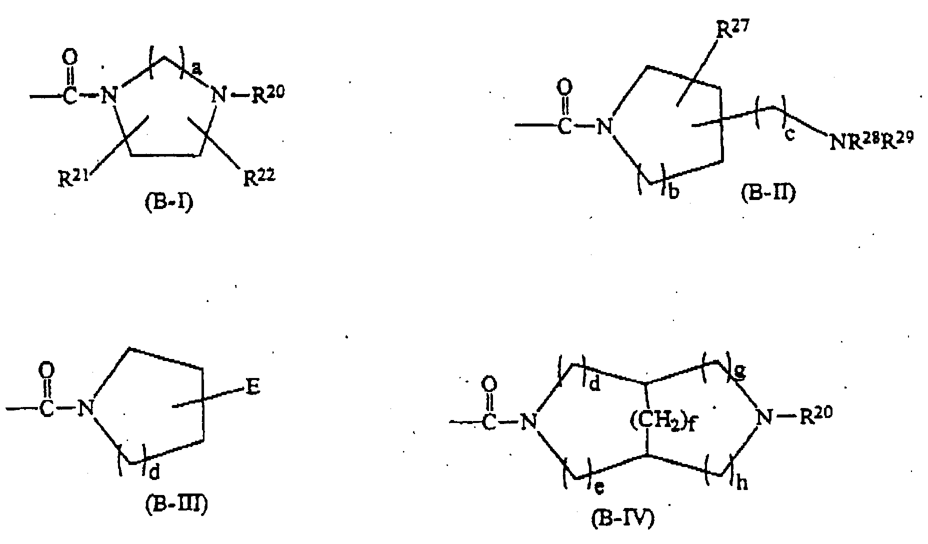 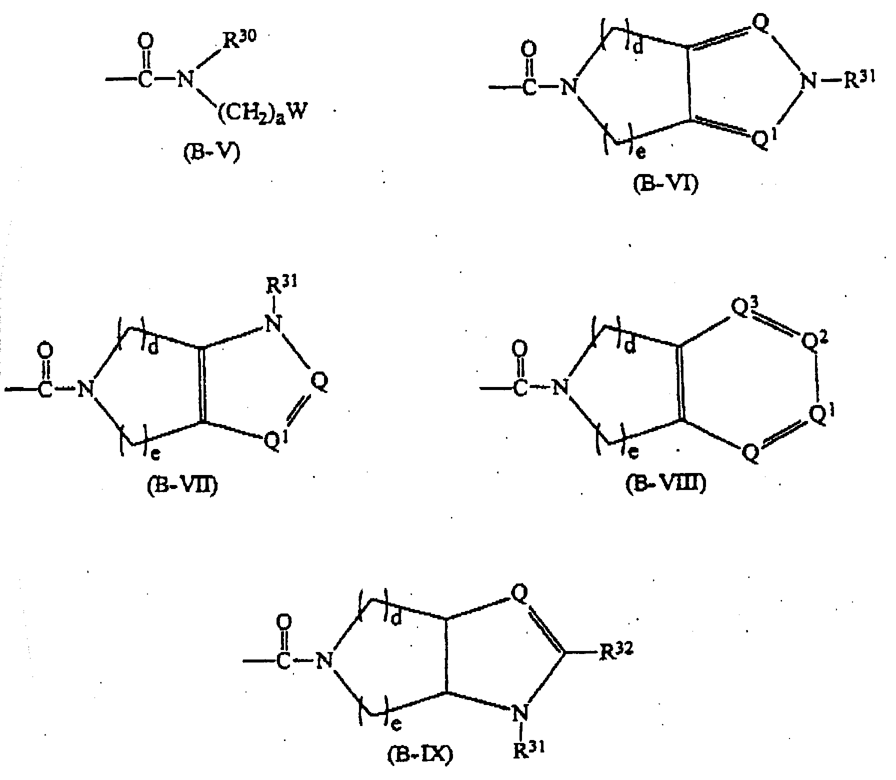 dans lesquelles :
a est 1, 2 ou 3,
b est 0, 1 ou 2,
c est 0 ou 1,
d est 0, 1 ou 2,
e est 0, 1 ou 2,
f est 0, 1 ou 2,
g est 0, 1 ou 2,
h est 0, 1 ou 2,
R²⁰ représente un atome d'hydrogène, un groupe protecteur d'amine, un groupe de formule -COR²⁶, où
R²⁶ représente un groupe alkyle ayant 1 à 6 atomes de carbone ou un groupe alkyle substitué ayant 1 à 6 atomes de carbone et qui et substitué par au moins un des substituants K ;
un groupe de formule -C(=NR¹⁶)R¹⁷, où
R¹⁶ et R¹⁷ sont identiques ou différents et représentent chacun un atome d'hydrogène, un groupe alkyle ayant 1 à 6 atomes de carbone, un groupe amino ou amino protégé ou un groupe protecteur d'amine ;
un groupe alkyle non substitué ayant 1 à 6 atomes de carbone, ou un groupe alkyle substitué qui compte 1 à 6 atomes de carbone et qui est substitué par au moins l'un des substituants D définis ci-dessous ;
R²¹ et R²² sont identiques ou différents et représentent chacun un atome d'hydrogène, un groupe alkyle ayant 1 à 6 atomes de carbone, un groupe hydroxyle ou hydroxyle protégé ;
R²⁷ représente un atome d'hydrogène ou un groupe alkyle ayant 1 à 6 atomes de carbone ;
R²⁸ et R²⁹ sont identiques ou différents et représentent chacun un atome d'hydrogène, un groupe alkyle ayant 1 à 6 atomes de carbone, un groupe protecteur d'amine, un groupe amino ou amino protégé et un groupe de formule -C(=NR¹⁶)R¹⁷ où R¹⁶ et R¹⁷ sont tels que définis ci-dessus ;
R³⁰ représente un atome d'hydrogène, un groupe alkyle ayant 1 à 6 atomes de carbone ou un groupe alkyle substitué ayant 1 à 6 atomes de carbone et qui est substitué par au moins un substituant choisi parmi les groupes hydroxyle et hydroxyle protégés ;
R³¹ représente un atome d'hydrogène, un groupe alkyle ayant 1 à 6 atomes de carbone ou un groupe protecteur d'amine ;
R³² représente un atome d'hydrogène, un groupe alkyle ayant 1 à 6 atomes de carbone ou un groupe de formule -NR²⁸R²⁹ où R²⁸ et R²⁹ sont tels que définis ci-dessus ;
E représente un groupe imidazolyle ou un groupe triazolyle ;
W représente un groupe hétérocyclique aromatique ayant 5 à 8 atomes endocycliques dont 1 à 4 sont des atomes d'azote, ledit groupe hétérocyclique aromatique n'étant pas substitué ou étant substitué par au moins un groupe alkyle ayant 1 à 6 atomes de carbone ;
Q, Q¹, Q² et Q³ sont choisis indépendamment dans la classe formée par les groupes de formule ≡CH et ≡N ;
lesdits substituants B¹ sont choisis parmi les groupes alkyle ayant 1 à 6 atomes de carbone, les groupes protecteurs d'amine, les groupes de formule -C(=NR¹⁶)R¹⁷ où R¹⁶ et R¹⁷ sont tels que définis ci-dessus, les groupes protecteurs d'amine et les groupes de formule -CONR¹⁸R¹⁹, où
R¹⁸ et R¹⁹ sont identiques ou différents et représentent chacun un groupe alkyle ayant 1 à 6 atomes de carbone ou un groupe acyle carboxylique ;
lesdits substituants C sont choisis parmi les groupes alkyle ayant 1 à 6 atomes de carbone, les groupes alcoxy ayant 1 à 6 atomes de carbone, les atomes d'halogènes, les groupes alcoxycarbonyle ayant 2 à 7 atomes de carbone, les groupes de formule -CONR⁸R⁹ où R⁸ et R⁹ sont tels que définis ci-dessus, les groupes cyano, hydroxyle et nitro ;
lesdits substituants D sont choisis parmi les groupes hydroxyle et hydroxyle protégés, les groupes carboxyle et carboxyle protégés, le groupe cyano, les groupes alcoxy ayant 1 à 6 atomes de carbone, les groupes alkylsulfonyle ayant 1 à 6 atomes de carbone, les groupes de formule -NHCOR²³, -NR²⁴R²⁵, -CONR²⁴R²⁵ ou -OCONR²⁴R²⁵, où
R²³, R²⁴ et R²⁵ sont identiques ou différents et représentent chacun un atome d'hydrogène ou un groupe alkyle ayant 1 à 6 atomes de carbone,
ou d'un sel pharmaceutiquement acceptable de ce composé ou d'un ester pharmaceutiquement acceptable de ce composé, lequel procédé comprend la réaction d'un composé de formule (II) : 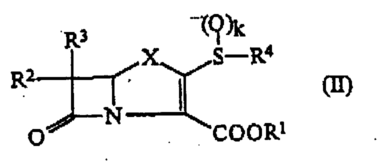 dans laquelle
R¹, R², R³ et X sont tels que définis ci-dessus,
k est 1 ou 2, et
R⁴ représente :
un groupe alkyle ayant 1 à 6 atomes de carbone et qui n'est pas substitué ou est substitué par au moins l'un des substituants E définis ci-dessous,
un groupe alcényle ayant 2 à 6 atomes de carbone et qui n'est pas substitué ou est substitué par au moins l'un des substituants E définis ci-dessous,
un groupe aryle, tel que défini ci-dessus,
un groupe aralkyle dans lequel un groupe alkyle ayant 1 à 6 atomes de carbone est substitué par au moins un groupe aryle tel que défini ci-dessus,
un groupe cycloalkyle ayant 3 à 7 atomes de carbone et qui n'est pas substitué ou est substitué par au moins l'un des substituants C définis ci-dessus,
un groupe hétérocyclique aromatique ayant 5 à 7 atomes endocycliques dans un noyau aromatique, au moins l'un desdits atomes étant un hétéroatome d'azote et/ou d'oxygène et/ou de soufre, ledit groupe n'étant pas substitué ou étant substitué par au moins l'un des substituants C définis ci-dessus ;
un groupe hétérocyclique condensé dans lequel un groupe hétérocyclique aromatique tel que défini ci-dessus est condensé avec un groupe aryle tel que défini ci-dessus, ou un groupe alkyle ayant 1 à 6 atomes de carbone et qui est substitué par au moins un groupe hétérocyclique aromatique et/ou un groupe hétérocyclique condensé définis ci-dessus ; et
lesdits substituants E sont choisis parmi les groupes hydroxyle et hydroxyle protégés, les groupes amino et amino protégés ;
avec un composé de formule (III) :
ASH (III)
dans laquelle A est tel que défini ci-dessus et, si cela est souhaité, l'élimination de tous groupes protecteurs, caractérisé en ce que la réaction dudit composé de formule (II) avec ledit composé de formule (III) est conduite en présence d'un sel de magnésium ou d'aluminium choisi dans le groupe suivant :
un complexe bromure de magnésium-éther de diéthyle ; un mélange de complexe bromure de magnésium-éther de diéthyle avec la diisopropyléthylamine, la 1-éthylpipéridine ou le 1,5-diazabicyclo[5,4,0]-5-undécène ; le cyclohexylisopropylamidure de bromomagnésium, le diisopropylamidure de bromomagnésium, le diéthylamidure de bromomagnésium ou l'hexaméthyldisilazidure de bromomagnésium ; le *t*-butylate de bromomagnésium ; les mélanges de diisopropylamidure de lithium ou bistriméthylsilylamidure de lithium avec un complexe bromure de magnésium-éther de diéthyle ; ou le diisopropylamidure de diéthylaluminium ;
le composé de formule (II) et le mercaptan de formule (III) étant utilisés dans un rapport molaire de 1:1 à 1:3 et le sel de magnésium ou d'aluminium étant présent en une quantité de 1 à 20 moles par mole du composé de formule (II).

2. Procédé selon la revendication 1, dans lequel ledit sel est un sel d'aluminium.

3. Procédé selon la revendication 1, dans lequel ledit sel est un sel de magnésium.

4. Procédé selon l'une quelconque des revendications 1 à 3, dans lequel
R¹ représente :
un atome d'hydrogène,
un groupe alkyle ayant 1 à 6 atomes de carbone,
un groupe aralkyle qui est un groupe alkyle ayant 1 à 4 atomes de carbone et qui est substitué par au moins un groupe aryle,
un groupe alcényle ayant 2 à 6 atomes de carbone et qui n'est pas substitué ou est substitué par un ou plusieurs atomes d'halogène,
un groupe halogénoalkyle ayant 1 à 6 atomes de carbone,
un groupe silyléthyle trisubstitué dans lequel les trois, ou deux ou un des substituants sont des groupes alkyle ayant 1 à 5 atomes de carbone, et zéro, un ou deux des substituants sont des groupes aryle,
un groupe acyloxyalkyle dans lequel la portion acyle est un groupe alcanoyle ayant 1 à 6 atomes de carbone et la portion alkyle compte 1 à 6 atomes de carbone,
un groupe alcoxycarbonyloxyalkyle dans lequel les portions alcoxy et alkyle ont chacune 1 à 6 atomes de carbone,
un groupe cycloalcoxycarbonyloxyalkyle dans lequel la portion cycloalkyle compte 3 à 7 atomes de carbone cycliques et n'est pas substituée ou est substituée par au moins l'un des substituants C, et la portion alkyle compte 1 à 6 atomes de carbone,
un groupe (5-alkyl- ou 5-aryl-2-oxo-1,3-dioxolène-4-yl)méthyle dans lequel la portion alkyle compte 1 à 4 atomes de carbone ; ou
le groupe 3-phtalidyle.

5. Procédé selon l'une quelconque des revendications 1 à 4, dans lequel :
R² et R³ sont identiques ou différents et représentent chacun un atome d'hydrogène, un groupe alkyle ayant 1 à 4 atomes de carbone ou un groupe de formule 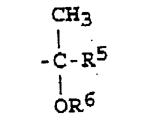
où R⁵ représente un atome d'hydrogène ou un groupe méthyle ; et
R⁶ représente :
un atome d'hydrogène,
un groupe silyle substitué dans lequel le groupe silyle porte 3 substituants choisis parmi les groupes alkyle ayant 1 à 6 atomes de carbone et le groupe phényle,
un groupe aralkyle dans lequel un groupe alkyle ayant 1 à 4 atomes de carbone est substitué par au moins un groupe aryle,
un groupe aralkyloxycarbonyle dans lequel la portion aralkyle est telle que définie ci-dessus,
un groupe alcényloxycarbonyle dans lequel la portion alcényle compte 2 à 6 atomes de carbone et n'est pas substituée ou est substituée par au moins un atome d'halogène,
un groupe alcoxycarbonyle ayant 2 à 7 atomes de carbone,
un groupe silylalcoxycarbonyle substitué dans lequel la portion alcoxycarbonyle et la portion silyle substitué sont telles que définies ci-dessus,
un groupe hétérocyclique contenant de l'oxygène,
un groupe alcoxyalkyle dans lequel les portions alcoxy et alkyle ont chacune 1 à 6 atomes de carbone,
un groupe silylalcoxyalkyle substitué dans lequel la portion alcoxyalkyle et la portion silyle substitué sont telles que définies ci-dessus, ou
un groupe alcanoyle ou halogénoalcanoyle n'ayant pas plus de 6 atomes de carbone ;
ou bien
R² et R³ représentent ensemble un groupe de formule =C(CH₃)CH₂OR⁶.

6. Procédé selon l'une quelconque des revendications 1 à 5, dans lequel X représente un atome de soufre ou un groupe de formule 〉CHR⁷ où R⁷ représente un atome d'hydrogène, un groupe alkyle ayant 1 à 4 atomes de carbone ou un groupe alcoxy ayant 1 à 4 atomes de carbone.

7. Procédé selon l'une quelconque des revendications 1 à 6, dans lequel
A représente
un groupe alkyle ayant 1 à 4 atomes de carbone et qui n'est pas substitué ou est substitué par 1 ou 2 des substituants K^{a} définis ci-dessous,
un groupe aryle tel que défini ci-dessous,
un groupe aralkyle dans lequel un groupe alkyle ayant 1 à 3 atomes de carbone est substitué par au moins un groupe aryle tel que défini ci-dessous,
un groupe hétérocyclique aromatique ou non aromatique qui peut être monocyclique ou peut consister en deux ou plusieurs cycles liés entre eux par condensation ou par une liaison spiro et qui compte 4 à 7 atomes endocycliques, au moins l'un desdits atomes étant un hétéroatome d'azote et/ou d'oxygène et/ou de soufre, ledit groupe n'étant pas substitué ou étant substitué par au moins l'un des substituants B^{a} sur des atomes de carbone, par au moins l'un des substituants B^{a1} sur des hétéroatomes d'azote et/ou par au moins un atome d'oxygène pour former un groupe sulfinyle ou sulfonyle sur des hétéroatomes de soufre, tous étant tels que définis ci-dessous ; ou
un groupe alkyle ayant 1 à 3 atomes de carbone et qui est substitué par au moins un substituant choisi parmi les groupes hétérocycliques définis ci-dessus ;
lesdits groupes aryle sont des groupes carbocycliques aromatiques ayant 6 à 10 atomes de carbone endocycliques et ne sont pas substitués ou sont substitués par au moins l'un des substituants C^{a} définis ci-dessous ;
lesdits substituants K^{a} sont choisis parmi les groupes hydroxyle et hydroxyle protégés, les groupes amino et amino protégés, les groupes de formule -C(=NR¹⁰)NR¹¹R¹², où
R¹⁰, R¹¹ et R¹² sont identiques ou différents et représentent chacun un atome d'hydrogène, un groupe protecteur d'amine ou un groupe alkyle ayant 1 à 4 atomes de carbone, ou bien R¹¹ et R¹² représentent ensemble un groupe de formule -(CH₂)ₙ- où n est un nombre entier de 2 à 6, ou bien R¹⁰ et R¹¹ représentent ensemble un groupe de formule -(CH₂)ₚ-, où p est 2 ou 3, et
les groupes de formule -NR¹³C(=NR¹⁴)R¹⁵ où
R¹³ et R¹⁴ sont identiques ou différents et représentent chacun un atome d'hydrogène, un groupe protecteur d'amine ou un groupe alkyle ayant 1 à 6 atomes de carbone, et
R¹⁵ représente un atome d'hydrogène, un groupe alkyle ayant 1 à 4 atomes de carbone, un groupe amino ou amino protégé,
ou bien
deux quelconques de R¹³, R¹⁴ et R¹⁵ représentent ensemble un groupe de formule -(CH₂)ₚ- ou p est 2 ou 3 ;
lesdits substituants B^{a} sont choisis parmi
les groupes alkyle ayant 1 à 4 atomes de carbone,
les groupes alcoxy ayant 1 à 6 atomes de carbone,
le groupe hydroxyle,
les atomes d'halogènes,
le groupe cyano,
le groupe nitro,
les groupes alcoxycarbonyle ayant 2 à 5 atomes de carbone,
le groupe carboxyle,
les atomes d'oxygène, pour former un groupe carbonyle avec un atome de carbone cyclique,
les groupes alcoxyalkyle dans lesquels les portions alcoxy et alkyle ont toutes deux 1 à 6 atomes de carbone,
les groupes halogénoalkyle ayant 1 à 6 atomes de carbone,
les groupes alcanoyloxy ayant 1 à 6 atomes de carbone,
les groupes de formule -NR⁸R⁹ et les groupes de formule -CONR⁸R⁹ où R⁸ et R⁹ sont identiques ou différents et représentent chacun un atome d'hydrogène, un groupe protecteur d'amine, un groupe alkyle ayant 1 à 4 atomes de carbone ou un groupe phényle, ou bien R⁸ et R⁹ représentent ensemble un groupe de formule -(CH₂)_{q}-Oᵣ-(CH₂)ₛ-, où q et s sont identiques ou différents et représentent chacun 0 ou un nombre entier de 1 à 5, et r est 0 ou 1, à condition que (q + s) soit un nombre entier égal ou supérieur à 2 ; et les groupes de formules (B-I), (B-II), (B-III), (B-IV), (B-V), (B-VI), (B-VII), (B-VIII) et (B-IX) ;
lesdits substituants B^{a1} sont choisis parmi les groupes alkyle ayant 1 à 4 atomes de carbone, les groupes protecteurs d'amine, les groupes de formule -C(=NR¹⁶)R¹⁷ où R¹⁶ et R¹⁷ sont tels que définis ci-dessus, les groupes protecteurs d'amine et les groupes de formule -CONR¹⁸R¹⁹ où
R¹⁸ et R¹⁹ sont identiques ou différents et représentent chacun un groupe alkyle ayant 1 à 4 atomes de carbone, un groupe alcanoyle ayant 2 à 5 atomes de carbone ou un groupe benzoyle ;
lesdits substituants C^{a} sont choisis parmi les groupes alkyle ayant 1 à 4 atomes de carbone, les groupes alcoxy ayant 1 à 4 atomes de carbone, les atomes d'halogènes, les groupes alcoxycarbonyle ayant 2 à 5 atomes de carbone, les groupes de formule -CONR⁸R⁹ où R⁸ et R⁹ sont tels que définis ci-dessus, les groupes cyano, hydroxyle et nitro.

8. Procédé selon l'une quelconque des revendications 1 à 3, dans lequel :
R¹ représente :
un atome d'hydrogène,
un groupe alkyle ayant 1 à 4 atomes de carbone,
un groupe alcényle ayant 2 à 4 atomes de carbone,
un groupe aralkyle qui est un groupe alkyle ayant 1 ou 2 atomes de carbone et qui est substitué par au moins un groupe phényle,
un groupe halogénoalkyle ayant 1 à 4 atomes de carbone,
un groupe silyléthyle trisubstitué dans lequel les trois ou deux des substituants sont des groupes alkyle ayant 1 à 4 atomes de carbone, et zéro ou un des substituants est un groupe phényle,
un groupe acyloxyalkyle dans lequel la portion acyle est un groupe alcanoyle ayant 2 à 5 atomes de carbone et la portion alkyle compte 1 ou 2 atomes de carbone,
un groupe alcoxycarbonyloxyalkyle dans lequel les portions alcoxy et alkyle ont chacune 1 à 4 atomes de carbone, un groupe (5-méthyl-2-oxo-1,3-dioxolène-4-yl)méthyle ; ou le groupe 3-phtalidyle.

9. Procédé selon l'une quelconque des revendications
1 à 3 et 8, dans lequel : R² et R³ sont identiques ou différents et représentent chacun un atome d'hydrogène, un groupe alkyle ayant 1 à 4 atomes de carbone ou un groupe de formule 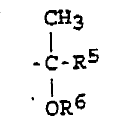
où R⁵ représente un atome d'hydrogène ou un groupe méthyle ; et
R⁶ représente :
un atome d'hydrogène,
un groupe silyle substitué dans lequel le groupe silyle porte 3 substituants choisis parmi les groupes alkyle ayant 1 à 4 atomes de carbone et le groupe phényle,
un groupe aralkyle dans lequel un groupe alkyle ayant 1 à 4 atomes de carbone est substitué par au moins un groupe aryle, comme défini dans la revendication 7,
un groupe alcoxycarbonyle ayant 2 à 7 atomes de carbone,
un groupe tétrahydropyrannyle,
un groupe alcoxyalkyle dans lequel les portions alcoxy et alkyle ont chacune 1 à 4 atomes de carbone,
un groupe alcanoyle ou halogénoalcanoyle ayant 2 à 5 atomes de carbone ;
ou bien
R² et R³ représentent ensemble un groupe de formule =C(CH₃)CH₂OR⁶.

10. Procédé selon l'une quelconque des revendications 1 à 3, 8 et 9, dans lequel X représente un atome de soufre ou un groupe de formule 〉CHR⁷ où R⁷ représente un atome d'hydrogène, un groupe alkyle ayant 1 ou 2 atomes de carbone ou un groupe alcoxy ayant 1 ou 2 atomes de carbone.

11. Procédé selon l'une quelconque des revendications 1 à 3, 8, 9 et 10, dans lequel
A représente
un groupe alkyle ayant 1 ou 2 atomes de carbone et qui n'est pas substitué ou est substitué par 1 ou 2 des substituants K^{b} définis ci-dessous,
un groupe phényle qui n'est pas substitué ou est substitué par 1 ou 2 des substituants C^{b} définis ci-dessous,
un groupe aralkyle dans lequel un groupe alkyle ayant 1 à 3 atomes de carbone est substitué par au moins un groupe phényle qui n'est pas substitué ou est substitué par 1 ou 2 des substituants C^{b} définis ci-dessous,
un groupe hétérocyclique aromatique ou non aromatique qui peut être monocyclique ou peut consister en deux ou plusieurs cycles liés entre eux par condensation ou par une liaison spiro et qui compte 5 ou 6 atomes endocycliques, 1 à 3 desdits atomes étant des hétéroatomes d'azote et/ou d'oxygène et/ou de soufre, ledit groupe n'étant pas substitué ou étant substitué par au moins l'un des substituants B^{b} sur des atomes de carbone, par au moins l'un des substituants B^{b1} sur des hétéroatomes d'azote et/ou par au moins un atome d'oxygène pour former un groupe sulfinyle ou sulfonyle sur des hétéroatomes de soufre, tous étant tels que définis ci-dessous ;
lesdits substituants K^{b} sont choisis parmi les groupes hydroxyle et hydroxyle protégés, les groupes amino et amino protégés, et les groupes de formule -C(=NR¹⁰)NR¹¹R¹², où
R¹⁰, R¹¹ et R¹² sont identiques ou différents et représentent chacun un atome d'hydrogène, un groupe protecteur d'amine ou un groupe alkyle ayant 1 ou 2 atomes de carbone ;
lesdits substituants B^{b} sont choisis parmi
les groupes alkyle ayant 1 ou 2 atomes de carbone,
les groupes alcoxy ayant 1 ou 2 atomes de carbone,
le groupe hydroxyle,
les atomes d'halogènes,
le groupe cyano,
le groupe nitro,
les groupes alcoxycarbonyle ayant 2 à 5 atomes de carbone,
le groupe carboxyle,
les groupes halogénoalkyle ayant 1 à 6 atomes de carbone,
les groupes alcanoyloxy ayant 1 à 6 atomes de carbone,
les groupes de formule -NR⁸R⁹ et les groupes de formule -CONR⁸R⁹ où R⁸ et R⁹ sont identiques ou différents et représentent chacun un atome d'hydrogène, un groupe protecteur d'amine, un groupe alkyle ayant 1 ou 2 atomes de carbone ou un groupe phényle, ou bien R⁸ et R⁹ représentent ensemble un groupe de formule -(CH₂)_{q}-Oᵣ-(CH₂)ₛ-, où q et s sont identiques ou différents et représentent chacun 0 ou un nombre entier de 1 à 5, et r est 0 ou 1, à condition que (q + s) soit un nombre entier égal ou supérieur à 2 ; et
les groupes de formules (B-I), (B-II), (B-III), (B-IV), (B-V), (B-VI), (B-VII), (B-VIII) et (B-IX) ;
lesdits substituants B^{b1} sont choisis parmi les groupes alkyle ayant 1 ou 2 atomes de carbone, les groupes protecteurs d'amine, les groupes de formule -C(=NR¹⁶)R¹⁷ où R¹⁶ et R¹⁷ sont choisis indépendamment parmi les atomes d'hydrogène, les groupes méthyle, les groupes protecteurs d'amine et les groupes de formule -CONR¹⁸R¹⁹ où
R¹⁸ et R¹⁹ sont identiques ou différents et représentent chacun un groupe alkyle ayant 1 ou 2 atomes de carbone, un groupe alcanoyle ayant 2 à 5 atomes de carbone ou un groupe benzoyle ;
lesdits substituants C^{b} sont choisis parmi les groupes alkyle ayant 1 ou 2 atomes de carbone, les groupes alcoxy ayant 1 ou 2 atomes de carbone, les atomes d'halogènes, les groupes de formule -CONR⁸R⁹, les groupes cyano, hydroxyle et nitro.
